# EUROPEAN PATENT APPLICATION

(11) **EP 4 653 020 A1**
(43) Date of publication of application: **26.11.2025**
(21) Application number: 24744401.1
(22) Date of filing: 19.01.2024
(51) Int. Cl.: A61K 47/68, A61K 39/44, A61P 35/00

(54) **TYROSINASE-MEDIATED PROTEIN SITE-SPECIFIC CONJUGATION AND USE THEREOF**

(30) Priority: 19.01.2023 CN 202310090079
(71) Applicant: Shanghai Shengdi Pharmaceutical Co., Ltd, Shanghai 201210 (CN); Jiangsu Hengrui Pharmaceuticals Co., Ltd., Lianyungang, Jiangsu 222047 (CN)
(72) Inventor: ZHANG, Wei, Shanghai 201210 (CN); QIAN, Mengxin, Shanghai 201210 (CN); YUAN, Xiaojing, Shanghai 201210 (CN); BAO, Luyao, Shanghai 201210 (CN); REN, Wenming, Shanghai 201210 (CN); LIAO, Cheng, Shanghai 201210 (CN)
(74) Representative: Dragotti & Associati S.P.A.
(86) International application number: PCT/CN2024/073258
(87) International publication number: WO 2024/153232

(57) **Abstract**

The present disclosure relates to tyrosinase-mediated protein site-specific conjugation and a use thereof. Specifically, the present disclosure provides tyrosinase-mediated protein site-specific conjugation and a use thereof in the preparation of a drug (for example, an ADC).

## Description

The present disclosure claims priority to the Chinese Patent Application (Application No. CN202310090079.0) filed on January 19, 2023.

### TECHNICAL FIELD

The present disclosure relates to the field of biopharmaceutics and particularly to tyrosinase-mediated site-specific protein conjugation and use thereof in the manufacture of a medicament (e.g., an ADC).

### BACKGROUND

The design and development of antibody-drug conjugates (ADCs) involve antibody selectivity, the cytotoxicity of small-molecule toxins, and the covalent linkage between antibodies and small-molecule toxins. The conjugation of antibodies and small-molecule toxins is one of the cores of the clinical success of ADC drugs. Studies on the conjugation technologies that have been approved for use in ADCs have shown that Adcetris^{®}, etc. are prepared by reducing interchain disulfide bonds to release cysteine residues and then linking to a linker, and Kadcyla^{®}, etc. are prepared by random lysine conjugation (Joubert N, et al. Pharmaceuticals (Basel). 2020 Sep 14;13(9):245). Both methods involve non-specific modification of lysine or cysteine, and the obtained ADCs are actually highly heterogeneous mixtures, which are difficult to characterize and purify, heterogeneous in pharmacokinetic properties, poor in stability, and prone to aggregation (Junutula JR, et al. Nat Biotechnol. 2008 Aug;26(8):925-32).

Studies on site-specifically modifying antibodies to generate homogeneous ADCs include the Thiomab technology of Genentech, which inserts additional cysteines into antibody protein sequences to obtain ADCs (Pillow TH, et al. J Med Chem. 2014 Oct 9;57(19):7890-9), and the technology of Schultz et al. of the Scripps Research Institute, which introduces unique active non-natural amino acids (NNAAs) into antibodies and applies them to the selective modification of toxins (Hallam TJ, et al. Mol Pharm. 2015 Jun 1;12(6):1848-62). However, there is serum instability in maleimide-based cysteine conjugation, which has been shown to cause ADCs to lose effectiveness, or off-target toxicity. The introduction of non-natural amino acids requires expression in genetically modified cells or cell-free systems. However, the expression level is low, and there is a concern that non-natural amino acids lead to the immunogenicity of ADCs *in vivo.* Enzymatic reaction-mediated site-specific conjugation technology is an emerging technology for ADC production. Synaffix modified the characteristic glycan chain at position N297 of an antibody, achieved the introduction of an azido group using endoglycosidase and glycosyltransferase, and further used click chemistry to conjugate a toxin to the antibody (Geel RV, et al. Bioconjug Chem. 2015 Nov 18;26(11):2233-42). However, the glycan chain at position N297 itself has an important effect on the solubility and stability of the antibody. Triphase, NBE Therapeutics, and Legochem introduced to the C-terminus of the heavy or light chain of an antibody a specific amino acid sequence that can be recognized by formylglycine generating enzyme (FGE), SortaseA, and prenyl transferase, thereby completing the site-specific modification of toxin molecules. However, such C-terminal modification methods have certain limitations in terms of conjugation position and drug antibody ratio (DAR). Usually, they can only achieve site-specific conjugation with a DAR value of 2, and the sequence that needs to be additionally introduced is relatively long (more than 5 amino acids), which may pose a potential immunogenicity risk (Walsh SJ, et al. Chem Soc Rev. 2021 Jan 21;50(2):1305-1353). The use of enzymes to specifically modify particular amino acids in antibodies to achieve site-specific conjugation is one of the most cutting-edge technologies in the field of site-specific conjugation. Full-length IgG antibodies of human isotypes contain a conserved glutamine residue (Q295) at position 295 of their heavy chains. Therefore, transglutaminase (mTG) can be used to site-specifically modify the glutamine in the antibodies. However, Q295 is in close proximity to a N-glycosylation site (N297), and the N-glycosylation is generally believed to render Q295 inaccessible to mTG. Therefore, before conjugation reactions, the Fc regions of the antibodies need to be deglycosylated or mutated to remove the N-glycosylation site (see WO2013/092998). Meanwhile, the overall reaction efficiency of mTG-mediated conjugation reactions is also relatively low. Jorick J. Bruins et al. used mushroom-derived tyrosinase to perform site-specific modification on Y296 and Y300 near N297 (Bruins JJ, et al. Bioconjug Chem. 2021 Oct 20;32(10):2167-2172). However, both transglutaminase and mushroom-derived tyrosinase are only capable of modifying deglycosylated antibodies.

To date, there has been no report on introducing additional mutations into antibody constant regions to achieve modification of particular amino acids in the antibody constant regions by tyrosinase, nor have related applications of tyrosinase-mediated site-specific conjugation in glycosyl-containing intact antibodies been disclosed. The present disclosure provides a technology for tyrosinase-mediated site-specific modification of tyrosine in an antibody constant region or a fragment thereof, which has the characteristics of fast reaction rates, high site-specific modification specificity, ADC products being highly homogeneous, and no need for deglycosylation of the antibody or to mutate the antibody to remove N-glycosylation sites. Meanwhile, introducing multiple reactive tyrosines can achieve multi-site site-specific conjugation, which resolves the problem that the current enzymatic reaction-mediated site-specific conjugation of antibodies can only achieve a DAR value of 2, provides a choice of a higher DAR value, and can be used for developing more efficient and safer ADC drugs.

### SUMMARY

The present disclosure provides a protein (e.g., an antibody or an antigen-binding fragment thereof) comprising an antibody constant region or a constant region thereof and a conjugate prepared therefrom, a preparation method therefor, and use thereof in the manufacture of a medicament.

### Antibody or Antigen-Binding Fragment Thereof

The present disclosure provides an antibody or an antigen-binding fragment thereof, comprising an engineered tyrosine residue, wherein the engineered tyrosine residue is a tyrosine (Tyr) residue obtained by mutating (e.g., substituting or replacing) a natural amino acid residue.

In some embodiments, the natural amino acid residue or the engineered tyrosine residue obtained by mutation thereof may be one or more (e.g., 2, 3, 4, 5, 6, 7, 8, 9, 10, 12, 14, or 16) in number and is located in a heavy chain constant region and/or a light chain constant region. For example, the residue is located in a C_{H}1 region, a hinge region, an Fc region, and/or a CL region. In some embodiments, the residue is located in the C_{H}1 region, the hinge region, a C_{H}2 region, a C_{H}3 region, and/or a Cκ region and a Cλ region. In the present disclosure, the heavy chain constant region comprises the C_{H}1, the hinge region, the C_{H}2, and the C_{H}3.

In some embodiments, the antibody or the antigen-binding fragment thereof is of an IgG type, such as an IgG1, IgG2, IgG3, or IgG4 type, such as the human or mouse IgG1, IgG2, IgG3, or IgG4 type. In the present disclosure, human IgG1, IgG2, IgG3, and IgG4 are used interchangeably with IGHG1, IGHG2, IGHG3, and IGHG4.

In some embodiments, the natural amino acid residue is located at any one or any combination of positions 135, 137, 192, 223, 298, and 329 of a heavy chain HC and position 202 of a light chain LC.

In some embodiments, the natural amino acid residue is located at any one or any combination of positions 135T, 137G, 192S, 223T, 298S, and 329P of the heavy chain HC and position 202S of the light chain LC.

In some embodiments, the engineered tyrosine residue is located at any one or any combination of positions 135, 137, 192, 223, 298, and 329 of the heavy chain HC and position 202 of the light chain LC.

In some embodiments, the engineered tyrosine residue is selected from the group consisting of any one or any combination of T135Y, G137Y, S192Y, T223Y, S298Y, and P329Y of the heavy chain HC and S202Y of the light chain LC.

In some embodiments, the heavy chain HC is IGHG1. In some embodiments, the light chain LC is Cκ. Unless otherwise specified, the amino acid position numbers in the present disclosure are defined based on the EU numbering scheme.

In some embodiments, the natural amino acid residues at positions 135, 137, and 192 or the tyrosine residues into which they are mutated are located in the C_{H}1 region, the natural amino acid residue at position 223 or the tyrosine residue into which it is mutated is located in the hinge region, the natural amino acid residues at positions 298 and 329 or the tyrosine residues into which they are mutated are located in the C_{H}2 region, and the natural amino acid residue at position 202 or the tyrosine residue into which it is mutated is located in the Cκ region.

In some embodiments, the amino acid residue is located at any one or any combination of positions 135, 137, 192, 223, 298, and 329 of IGHG1 (or located at positions in IGHG2, IGHG3, and IGHG4 corresponding to positions 135, 137, 192, 223, 298, and 329 of IGHG1) and position 202 of Kappa LC (or a position in lammda LC corresponding to position 202 of Kappa LC).

In some embodiments, the engineered tyrosine residue contained in the antibody or the antigen-binding fragment thereof is selected from the group consisting of the following combinations:
T135Y/T223Y of the HC;
T223Y/P329Y of the HC;
T135Y/P329Y of the HC;
T135Y/T223Y/P329Y of the HC;
T135Y/T223Y/P329Y/S192Y of the HC;
T135Y/T223Y of the HC and S202Y of the LC;
T135Y/T223Y of the HC and S202Y of the LC;
T223Y/P329Y of the HC and S202Y of the LC;
T135Y/P329Y of the HC and S202Y of the LC;
G137Y of the HC and S202Y of the LC;
T135Y/T223Y/P329Y of the HC and S202Y of the LC;
T135Y/T223Y/P329Y/S192Y of the HC and S202Y of the LC;
P329Y of the HC and S202Y of the LC; and
S192Y of the HC and S202Y of the LC;
"/" represents "and".

In some embodiments, the antibody is a tumor antigen-targeting antibody, e.g., an anti-HER2 antibody, an anti-LIV-1 antibody, or an anti-TROP2 antibody.

In some embodiments, the anti-HER2 antibody or the antigen-binding fragment thereof comprises the heavy chain CDRs (HCDRs) and the light chain CDRs (LCDRs), or the heavy chain variable region (VH) and the light chain variable region (VL), of trastuzumab. The CDRs are as follows (defined according to the Kabat numbering scheme):
HCDR1: DTYIH (SEQ ID NO: 51)
HCDR2: RIYPTNGYTRYADSVKG (SEQ ID NO: 52)
HCDR3: WGGDGFYAMDY (SEQ ID NO: 53)
LCDR1: RASQDVNTAVA (SEQ ID NO: 54)
LCDR2: SASFLYS (SEQ ID NO: 55)
LCDR3: QQHYTTPPT (SEQ ID NO: 56)
> Trastuzumab VH (SEQ ID NO: 57)
> Trastuzumab VL (SEQ ID NO: 58)


.

In some embodiments, the present disclosure provides an anti-HER2 antibody or an antigen-binding fragment thereof, comprising a heavy chain and a light chain, wherein:
the heavy chain comprises the amino acid sequence set forth in any one of SEQ ID NOs: 3-10, and the light chain comprises the amino acid sequence set forth in SEQ ID NO: 2; the heavy chain comprises the amino acid sequence set forth in SEQ ID NO: 1, and the light chain comprises the amino acid sequence set forth in SEQ ID NO: 11;
the heavy chain comprises the amino acid sequence set forth in any one of SEQ ID NOs: 24-27, 7, and 9, and the light chain comprises the amino acid sequence set forth in SEQ ID NO: 2 or 11; for example, the heavy chain comprises the amino acid sequence set forth in any one of SEQ ID NOs: 24, 26, 7, and 9, and the light chain comprises the amino acid sequence set forth in SEQ ID NO: 11; or the heavy chain comprises the amino acid sequence set forth in any one of SEQ ID NOs: 25 and 27, and the light chain comprises the amino acid sequence set forth in SEQ ID NO: 2.

In some embodiments, the anti-LIV-1 antibody or the antigen-binding fragment thereof comprises the following heavy chain CDRs (HCDRs) and light chain CDRs (LCDRs), or the following heavy chain variable region (VH) and light chain variable region (VL). The CDRs are as follows (defined according to the Kabat numbering scheme):
HCDR1: TYAMS (SEQ ID NO: 59)
HCDR2: TISDSDYYADNVKG (SEQ ID NO: 60)
HCDR3: DDWDGDFDY (SEQ ID NO: 61)
LCDR1: KSTQSLLYSDGETYLN (SEQ ID NO: 62)
LCDR2: LVSKLDS (SEQ ID NO: 63)
LCDR3: WQGSHFPQT (SEQ ID NO: 64)
   > 0102 VH (SEQ ID NO: 65) 
EVQLVESGGGLVKPGGSLRLSCAASGFTFSTYAMSWVRQAPEKRLEWVATISDS
DYYADNVKGRFTISRDNAKNSLYLQMNSLRAEDTAVYYCVRDDWDGDFDYW
GQGTLVTVSS
   > 0102 VL (SEQ ID NO: 66)

DVVMTQSPLSLPVTPGEPASISCKSTQSLLYSDGETYLNWLLQKPGQSPQRLIYL
VSKLDSGVPDRFSGSGSGTDFTLKISRVEAEDVGVYYCWQGSHFPQTFGGGTK
VEIK.

In some embodiments, the present disclosure provides an anti-LIV-1 antibody or an antigen-binding fragment thereof, comprising a heavy chain and a light chain, wherein the heavy chain comprises the sequence set forth in any one of SEQ ID NOs: 14-19, and the light chain comprises the sequence set forth in SEQ ID NO: 13; or the heavy chain comprises the sequence set forth in SEQ ID NO: 12, and the light chain comprises the sequence set forth in SEQ ID NO: 20.

In some embodiments, the present disclosure provides an anti-LIV-1 antibody or an antigen-binding fragment thereof, comprising a heavy chain and a light chain, wherein the heavy chain comprises the sequence set forth in SEQ ID NO: 42, and the light chain comprises the sequence set forth in SEQ ID NO: 43. In some embodiments, the anti-TROP2 antibody or the antigen-binding fragment thereof comprises the following heavy chain CDRs (HCDRs) and light chain CDRs (LCDRs), or the following heavy chain variable region (VH) and light chain variable region (VL).

The CDRs are as follows (defined according to the Kabat numbering scheme):
HCDR1: DYAMH (SEQ ID NO: 68)
HCDR2: AITWNSGHIDYADSVEG (SEQ ID NO: 69)
HCDR3: VSYLSTASSLDY (SEQ ID NO: 70)
LCDR1: RASQGIRNYLA (SEQ ID NO: 71)
LCDR2: AASTLQS (SEQ ID NO: 72)
LCDR3: QRYNRAPYT (SEQ ID NO: 73)
   > TROP2 VH (SEQ ID NO: 74) EVQLVESGGGLVQPGRSLRLSCAASGFTFDDYAMHWVRQAPGKGLEWVSAIT 
WNSGHIDYADSVEGRFTISRDNAKNSLYLQMNSLRAEDTAVYYCAKVSYLSTAS
S
   > TROP2 VL (SEQ ID NO: 75)

DIQMTQSPSSLSASVGDRVTITCRASQGIRNYLAWYQQKPGKAPKLLIYAASTLQ
SGVPSRFSGSGSGTDFTLTISSLQPEDVATYYCQRYNRAPYTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLYSPVTKSFNRGEC.

In some embodiments, the antigen-binding fragment includes, but is not limited to, any one of the following: Fab, Fv, sFv, Fab', F(ab')2, linear antibodies, single-chain antibodies, scFv, sdAb, sdFv, nanobodies, peptibodies, domain antibodies, and multispecific antibodies (bispecific antibodies, diabodies, triabodies and tetrabodies, tandem di-scFv, and tandem tri-scFv).

### Protein

The present disclosure provides a protein comprising an antibody constant region or a fragment thereof, wherein the antibody constant region or the fragment thereof comprises one or more engineered tyrosine residues. The engineered tyrosine can be conjugated to a payload (e.g., a therapeutic agent or a diagnostic agent). The phenol moieties of the engineered tyrosine residues are oxidized by tyrosinase to o-quinone moieties. The payload (e.g., a therapeutic agent or a diagnostic agent) has an alkene or alkyne moiety, and the o-quinone moieties undergo [4+2] cycloaddition reactions with the alkene or alkyne moiety to form a conjugate.

The present disclosure provides a protein comprising an antibody constant region or a fragment thereof, wherein the antibody constant region or the fragment thereof comprises one or more engineered tyrosine residues. The phenol moieties of the engineered tyrosine residues are oxidizable by monomeric tyrosinase to o-quinone moieties but are not oxidizable by multimeric tyrosinase to o-quinone moieties. In some embodiments, the monomeric tyrosinase is derived from *Bacillus megaterium* or *Verrucomicrobium spinosum,* and the multimeric tyrosinase is a tetrameric enzyme derived from a mushroom. In some embodiments, the "derived from" may mean that the amino acid sequence encoding the enzyme is derived from the bacterium or that the enzyme is secreted by the bacterium or obtained by preparation (the enzyme may be commercially available). In some specific embodiments, the mushroom-derived tetrameric tyrosinase is Sigma Aldrich T3824.

The present disclosure provides a protein comprising an antibody constant region or a fragment thereof, wherein the antibody constant region or the fragment thereof comprises one or more engineered tyrosine residues. In some embodiments, the engineered tyrosine residues are not located near a N-glycosylation modification site, e.g., not located within 10 amino acids of the glycosylation modification site; or the protein does not require removal/reduction of glycosylation modification before reacting with tyrosinase. In some specific embodiments, the N-glycosylation modification is located at 297N of a heavy chain, as defined according to the EU numbering scheme. In some embodiments, the engineered tyrosine residues are not located between the amino acid residues at positions 292 and 302, positions 293 and 303, positions 291 and 301, positions 293 and 302, or positions 292 and 301 of the heavy chain HC. In some embodiments, the protein is a N-glycosylation-modified protein, specifically a 297N-glycosylation-modified protein.

The present disclosure provides a protein comprising an antibody constant region or a fragment thereof, wherein the antibody constant region or the fragment thereof comprises one or more engineered tyrosine residues, wherein:
I) the engineered tyrosine residues are located at any one or any combination of positions 135, 137, 192, 223, 298, and 329 of a heavy chain HC and position 202 of a light chain LC; or
II) the engineered tyrosine residues are located at any one or any combination of positions 135, 137, 192, 223, and 329 of a heavy chain HC and position 202 of a light chain LC.

In some embodiments, when the engineered tyrosine residues are located at position 298 of the heavy chain HC, the protein (or the antibody constant region or the fragment thereof therein) requires removal/reduction of glycosylation modification, and/or a natural tyrosine near position 298 is mutated into a non-natural amino acid. In some embodiments, the natural tyrosine near position 298 is selected from the group consisting of 296Y and/or 300Y. In some embodiments, the 296Y and/or 300Y are/is mutated into phenylalanine (Phe).

In some embodiments, the glycosylation modification is located at position 297N of the heavy chain. In some embodiments, the removal/reduction of glycosylation modification is achieved by a method selected from the group consisting of: (A) contacting the protein with an amidase; (B) contacting the protein with an endoglycosidase; and (C) mutating the protein such that 297N is substituted with a non-glycosylated amino acid. Exemplary modes include: (A) contacting the protein (or the antibody constant region or the fragment thereof therein) with an amidase (e.g., PNGase F) such that the protein (or the antibody constant region or the fragment thereof therein) is deglycosylated to obtain a protein (or an antibody constant region or a fragment thereof therein) from which a glycan is removed; or (B) contacting the protein (or the antibody constant region or the fragment thereof therein) with an endoglycosidase such that the protein (or the antibody constant region or the fragment thereof therein) is modified to form a protein (or an antibody constant region or a fragment thereof therein) having a glycan of the structure - GlcNAc(Fuc)_{b}, wherein b is 0 or 1; or (C) providing a mutated protein (or an antibody constant region or a fragment thereof therein) in which a glycosylated asparagine is substituted with a non-glycosylated amino acid.

The present disclosure provides a protein comprising an antibody constant region or a fragment thereof, wherein the antibody constant region or the fragment thereof comprises one or more engineered tyrosine residues, and the engineered tyrosine residues are located at any one or any combination of positions 135, 137, 192, 223, and 329 of a heavy chain HC and position 202 of a light chain LC.

In some embodiments, the engineered tyrosine residues in the protein are located at any one or any combination of positions 135, 137, 192, 223, and 329 of the heavy chain HC and position 202 of the light chain LC.

Illustratively, the combination is selected from the group consisting of:
202Y of the LC and 135Y of the HC;
202Y of the LC and 137Y of the HC;
202Y of the LC and 192Y of the HC;
202Y of the LC and 223Y of the HC;
202Y of the LC and 329Y of the HC;
135Y/137Y of the HC;
135Y/192Y of the HC;
135Y/223Y of the HC;
135Y/329Y of the HC;
137Y/192Y of the HC;
137Y/223Y of the HC;
137Y/329Y of the HC;
192Y/223Y of the HC;
192Y/329Y of the HC;
223Y/329Y of the HC;
135Y/137Y of the HC and 202Y of the LC;
135Y/192Y of the HC and 202Y of the LC;
135Y/223Y of the HC and 202Y of the LC;
135Y/329Y of the HC and 202Y of the LC;
137Y/192Y of the HC and 202Y of the LC;
137Y/223Y of the HC and 202Y of the LC;
137Y/329Y of the HC and 202Y of the LC;
192Y/223Y of the HC and 202Y of the LC;
192Y/329Y of the HC and 202Y of the LC;
223Y/329Y of the HC and 202Y of the LC;
135Y/137Y/192Y of the HC;
135Y/137Y/223Y of the HC;
135Y/137Y/329Y of the HC;
135Y/137Y/192Y of the HC and 202Y of the LC;
135Y/137Y/223Y of the HC and 202Y of the LC;
135Y/137Y/329Y of the HC and 202Y of the LC;
135Y/192Y/223Y of the HC;
135Y/192Y/329Y of the HC;
135Y/192Y/223Y of the HC and 202Y of the LC;
135Y/192Y/329Y of the HC and 202Y of the LC;
137Y/192Y/223Y of the HC;
137Y/192Y/329Y of the HC;
137Y/192Y/223Y of the HC and 202Y of the LC;
137Y/192Y/329Y of the HC and 202Y of the LC;
137Y/223Y/329Y of the HC;
137Y/223Y/329Y of the HC and 202Y of the LC;
192Y/223Y/329Y of the HC;
192Y/223Y/329Y of the HC and 202Y of the LC;
135Y/223Y/329Y of the HC; and
135Y/223Y/329Y of the HC and 202Y of the LC.

In some embodiments, the antibody constant region or the fragment thereof in the aforementioned protein is derived from an IgG, IgA, IgM, IgD, or IgE constant region or a fragment thereof. In some specific embodiments, the IgG is preferably IgG1, IgG2, IgG3, or IgG4, for example, IgG1, and for example, IGHG1, IGHG2, IGHG3, or IGHG4. In some embodiments, the antibody constant region or the fragment thereof in the aforementioned protein is a heavy chain constant region or a fragment thereof and/or a light chain constant region or a fragment thereof. In some embodiments, the heavy chain constant region is selected from the group consisting of any one or any combination of a C_{H}1 region, a hinge region, and a C_{H}2 region. In some embodiments, the light chain constant region is a Cκ region or a Cλ region. In some embodiments, the heavy chain constant region further comprises a C_{H}3 region.

In some embodiments, the antibody constant region or the fragment thereof in the aforementioned protein is glycosylation-modified or non-glycosylation-modified. In some embodiments, the glycosylation modification is a 297N glycosylation modification. In some embodiments, the aforementioned protein is selected from the group consisting of an antibody or an antigen-binding fragment thereof, an Fc region or a fragment thereof, and a fusion protein. In some embodiments, the antibody or the antigen-binding fragment thereof is a monospecific, bispecific, or multispecific antibody or an antigen-binding fragment thereof. In some embodiments, the antibody or the antigen-binding fragment thereof is an anti-tumor antibody or an antigen-binding fragment thereof; illustratively, the antibody or the antigen-binding fragment thereof is an anti-HER2 antibody or an antigen-binding fragment thereof, an anti-LIV-1 antibody or an antigen-binding fragment thereof, or an anti-TROP2 antibody or an antigen-binding fragment thereof.

In some embodiments, the anti-HER2 antibody or the antigen-binding fragment thereof comprises a VH and a VL, wherein the VH comprises a HCDR1, a HCDR2, and a HCDR3 of the amino acid sequences set forth in SEQ ID NOs: 51-53, and the VL comprises a LCDR1, a LCDR, and a LCDR3 of the amino acid sequences set forth in SEQ ID NOs: 54-56. In some embodiments, the VH comprises an amino acid sequence having at least 90% identity to SEQ ID NO: 57, and the VL comprises an amino acid sequence having at least 90% identity to SEQ ID NO: 58.

In some embodiments, the anti-LIV-1 antibody or the antigen-binding fragment thereof comprises a VH and a VL, wherein the VH comprises a HCDR1, a HCDR2, and a HCDR3 of the amino acid sequences set forth in SEQ ID NOs: 59-61, and the VL comprises a LCDR1, a LCDR, and a LCDR3 of the amino acid sequences set forth in SEQ ID NOs: 62-64. In some embodiments, the VH comprises an amino acid sequence having at least 90% identity to SEQ ID NO: 65, and the VL comprises an amino acid sequence having at least 90% identity to SEQ ID NO: 66.

In some embodiments, the anti-TROP2 antibody or the antigen-binding fragment thereof comprises a VH and a VL, wherein the VH comprises a HCDR1, a HCDR2, and a HCDR3 of the amino acid sequences set forth in SEQ ID NOs: 68-70, and the VL comprises a LCDR1, a LCDR, and a LCDR3 of the amino acid sequences set forth in SEQ ID NOs: 71-73. In some embodiments, the VH comprises an amino acid sequence having at least 90% identity to SEQ ID NO: 74, and the VL comprises an amino acid sequence having at least 90% identity to SEQ ID NO: 75.

In some embodiments, the aforementioned antibody is selected from the group consisting of a murine antibody, a chimeric antibody, a humanized antibody, and a fully human antibody.

In some embodiments, the aforementioned protein is a monomer or a multimer, e.g., a dimer, a trimer, or a tetramer. For example, the tetramer is an IgG antibody comprising two heavy chains and two light chains. In some embodiments, the tetramer comprises two identical heavy chains and two identical light chains. In some other embodiments, the tetramer comprises two different heavy chains and two different light chains.

In some embodiments, the aforementioned protein comprises a polypeptide fragment at a N-terminus and/or a C-terminus, and the fragment comprises one or more tyrosine residues. In some embodiments, the fragment is linked to the N-terminus and/or the C-terminus of the protein, either directly or by a linker. In some embodiments, the linker is a GS or polyG linker. In some embodiments, the fragment is YGGGG or YGGGGS and is linked to the N-terminus of the protein by a linker. In some embodiments, the fragment is GGGGY or SGGGGY and is linked to the N-terminus of the protein by a linker. In some embodiments, the protein may comprise the aforementioned N-terminal and C-terminal fragments simultaneously or individually.

In some embodiments, the "engineered tyrosine residue" of the present disclosure, in the context of an amino acid sequence, refers to the presence of a non-wild-type amino acid residue. For example, when a wild-type sequence and an engineered form of the sequence are aligned, the "engineered tyrosine residue" is not present in the corresponding wild-type polypeptide sequence, but is introduced into the polypeptide (by mutating (e.g., substituting or replacing) the existing amino acid residue. In some embodiments, the polypeptide is any of the proteins, antibody constant regions or fragments thereof, antibodies or antigen-binding fragments thereof of the present disclosure.

In some embodiments, the engineered tyrosine residues in the protein are one or more (e.g., 2, 3, 4, 5, 6, 7, 8, 9, 10, 12, 14, or 16) in number.

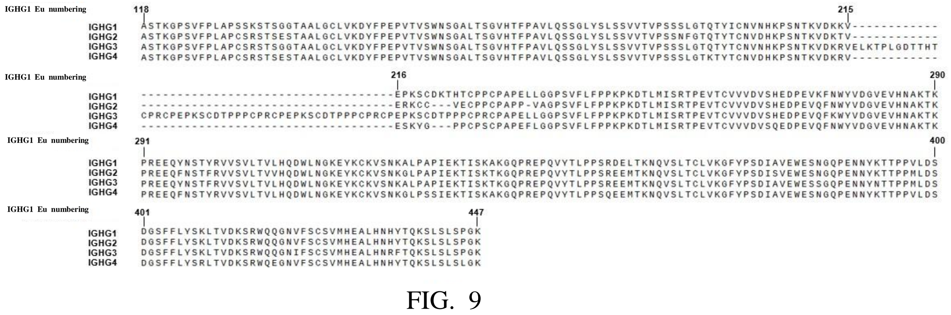

In some embodiments, the engineered tyrosine residue is located at any one or any combination of positions 135, 137, 192, 223, 298, and 329 of IGHG1 (or located at positions in IGHG2, IGHG3, and IGHG4 corresponding to positions 135, 137, 192, 223, 298, and 329 of IGHG1) and position 202 of Cκ (or a position in Cλ corresponding to position 202 of Cκ). In other embodiments, the engineered tyrosine residue is located at any one or any combination of positions 135, 137, 192, 223, and 329 of IGHG1 (or located at positions in IGHG2, IGHG3, and IGHG4 corresponding to positions 135, 137, 192, 223, and 329 of IGHG1) and position 202 of Cκ (or a position in Cλ corresponding to position 202 of Cκ). In some embodiments, the positions of IGHG1 are numbered according to the EU numbering scheme, and IGHG2, IGHG3, and IGHG4 correspond to the corresponding positions of IGHG1, for example, see FIG. 9.

In some embodiments, provided is an antibody constant region or a fragment thereof, comprising any one of the following:
1) C_{H}1 or a fragment thereof, wherein the C_{H}1 or the fragment thereof comprises (or has):
   1-1) an amino acid substitution at position 135, e.g., 135Y or T135Y;
   1-2) an amino acid substitution at position 137, e.g., 137Y or G137Y;
   1-3) an amino acid substitution at position 192, e.g., 192Y or S192Y;
   1-4) amino acid substitutions at positions 135 and 137, e.g., 135Y/137Y or T135Y/G137Y;
   1-5) amino acid substitutions at positions 135 and 192, e.g., 135Y/192Y or T135Y/S192Y;
   1-6) amino acid substitutions at positions 137 and 192, e.g., 137Y/192Y or G137Y/S192Y;
   1-7) amino acid substitutions at positions 135, 137, and 192, e.g., 135Y/137Y/192Y or T135Y/G137Y/S192Y;
2) a hinge region or a fragment thereof, wherein the hinge region or the fragment thereof comprises (or has) an amino acid substitution at position 223, e.g., 223Y or T223Y;
3) C_{H}2 or a fragment thereof, wherein the C_{H}2 or the fragment thereof comprises (or has):
   3-1) an amino acid substitution at position 298, e.g., 298Y or S298Y;
   3-2) an amino acid substitution at position 329, e.g., 329Y or P329Y;
   3-3) amino acid substitutions at positions 298 and 329, e.g., 298Y/329Y or S298Y/P329Y, wherein when an amino acid substitution at position 298 (e.g., 298Y or S298Y) is present, the C_{H}2 or the fragment thereof comprises amino acid substitutions of 296X and/or 300X, the substitutions being any amino acid other than tyrosine (e.g., 296F and/or 300F, e.g., 296F/300F);
4) a CL or a fragment thereof, wherein the CL and the fragment thereof comprises (or has) an amino acid substitution at position 202, e.g., 202Y or S202Y;
5) any combination of the above 1)-4), for example:
   5-1) C_{H}1 or a fragment thereof and a hinge region or a fragment thereof, wherein the C_{H}1 or the fragment thereof is 1-1), 1-2), 1-3), 1-4), 1-5), 1-6), or 1-7) described above, and the hinge region or the fragment thereof is 2) described above;
   5-2) a hinge region or a fragment thereof and C_{H}2 or a fragment thereof, wherein the hinge region or the fragment thereof is 2) described above, and the C_{H}2 or the fragment thereof is 3-1), 3-2), or 3-3) described above;
   5-3) C_{H}1 or a fragment thereof and C_{H}2 or a fragment thereof, wherein the C_{H}1 or the fragment thereof is 1-1), 1-2), 1-3), 1-4), 1-5), 1-6), or 1-7) described above, and the C_{H}2 or the fragment thereof is 3-1), 3-2), or 3-3) described above;
   5-4) C_{H}1 or a fragment thereof, a hinge region or a fragment thereof, and C_{H}2 or a fragment thereof, wherein the C_{H}1 or the fragment thereof is 1-1), 1-2), 1-3), 1-4), 1-5), 1-6), or 1-7) described above, the hinge region or the fragment thereof is 2) described above, and the C_{H}2 or the fragment thereof is 3-1), 3-2), or 3-3) described above;
   5-5) C_{H}1 or a fragment thereof and CL or a fragment thereof, wherein the C_{H}1 or the fragment thereof is 1-1), 1-2), 1-3), 1-4), 1-5), 1-6), or 1-7) described above, and the CL or the fragment thereof is 4) described above;
   5-6) CL or a fragment thereof and C_{H}2 or a fragment thereof, wherein the CL or the fragment thereof is 4) described above, and the C_{H}2 or the fragment thereof is 3-1), 3-2), or 3-3) described above;
   5-7) a hinge region or a fragment thereof and CL or a fragment thereof, wherein the hinge region or the fragment thereof is 2) described above, and the CL or the fragment thereof is 4) described above;
   5-8) C_{H}1 or a fragment thereof, C_{H}2 or a fragment thereof, and CL or a fragment thereof, wherein the C_{H}1 or the fragment thereof is 1-1), 1-2), 1-3), 1-4), 1-5), 1-6), or 1-7) described above, the C_{H}2 or the fragment thereof is 3-1), 3-2), or 3-3) described above, and the CL or the fragment thereof is 4) described above;
   5-9) C_{H}1 or a fragment thereof, a hinge region or a fragment thereof, and CL or a fragment thereof, wherein the C_{H}1 or the fragment thereof is 1-1), 1-2), 1-3), 1-4), 1-5), 1-6), or 1-7) described above, the hinge region or the fragment thereof is 2) described above, and the CL or the fragment thereof is 4) described above;
   5-10) a hinge region or a fragment thereof, C_{H}2 or a fragment thereof, and CL or a fragment thereof, wherein the hinge region or the fragment thereof is 2) described above, the C_{H}2 or the fragment thereof is 3-1), 3-2), or 3-3) described above, and the CL or the fragment thereof is 4) described above;
   5-11) C_{H}1 or a fragment thereof, a hinge region or a fragment thereof, C_{H}2 or a fragment thereof, and CL or a fragment thereof, wherein the C_{H}1 or the fragment thereof is 1-1), 1-2), 1-3), 1-4), 1-5), 1-6), or 1-7) described above, the hinge region or the fragment thereof is 2) described above, the C_{H}2 or the fragment thereof is 3-1), 3-2), or 3-3) described above, and the CL or the fragment thereof is 4) described above.

In some embodiments, CL is Cκ or Cλ.

In some embodiments, the fragment is at least 10 amino acids near an amino acid position where a tyrosine substitution occurs, for example, in a heavy chain, 10 amino acids near position 135 are positions 130 to 140, 10 amino acids near position 137 are positions 132 to 142, 10 amino acids near position 192 are positions 187 to 197, 10 amino acids near position 223 are positions 218 to 228, 10 amino acids near position 298 are positions 293 to 235, and 10 amino acids near position 329 are positions 324 to 334; 10 amino acids near position 202 in a light chain are positions 197 to 207.

In some embodiments, the C_{H}1 or the fragment thereof has at least 90% sequence identity to SEQ ID NO: 46 on the basis of the tyrosine substitutions described in 1-1) to 1-7) above. In some embodiments, the hinge region or the fragment thereof has at least 90% sequence identity to SEQ ID NO: 47 on the basis of the tyrosine substitution described in 2) above. In some embodiments, the C_{H}2 or the fragment thereof has at least 90% sequence identity to SEQ ID NO: 48 on the basis of the tyrosine substitutions described in 3-1) to 3-3) above. In some embodiments, the CL or the fragment thereof has at least 90% sequence identity to SEQ ID NO: 50 on the basis of the tyrosine substitution described in 4) above.

In some embodiments, the amino acid sequence of the C_{H}1 region is set forth in any one of SEQ ID NOs: 84 and 88-90. In some embodiments, the amino acid sequence of the hinge region is set forth in SEQ ID NO: 85. In some embodiments, the amino acid sequence of the C_{H}2 region is set forth in SEQ ID NO: 86. In some embodiments, the amino acid sequence of the Cκ region is set forth in SEQ ID NO: 87.

In some embodiments, the antibody constant region or the fragment thereof further comprises C_{H}3, the C_{H}3 being, for example, the sequence set forth in SEQ ID NO: 49 or a sequence having at least 90% identity thereto.

In the present disclosure, "at least 90% (sequence) identity" encompasses at least 90%, at least 91%, at least 92%, at least 93%, at least 94%, at least 95%, at least 96%, at least 97%, at least 98%, at least 99%, or 100% (sequence) identity.

In some embodiments, the aforementioned antibody constant region or the fragment thereof is a monomer, e.g., a polypeptide chain.

In some embodiments, the aforementioned antibody constant region or the fragment thereof is a multimer, e.g., a dimer, a trimer, or a tetramer.

In some embodiments, the antibody constant region or the fragment thereof further comprises an antigen-binding domain, e.g., a heavy chain variable region (VH) and a light chain variable region (VL), an immunoglobulin single variable domain (or a single-domain antibody, VHH), or an scfv, which specifically bind to an antigen or an epitope thereof. In some embodiments, the antigen-binding domain, for example, specifically binds to a tumor antigen such as HER2, LIV-1, or TROP2.

In some embodiments, provided is an antibody or an antigen-binding fragment thereof, which comprises the aforementioned antibody constant region or the fragment thereof of the present disclosure. The antibody or the antigen-binding fragment thereof is, for example, a camelid antibody, a chimeric antibody, a humanized antibody, a fully human antibody, or an antigen-binding fragment thereof. In some embodiments, the antibody or the antigen-binding fragment thereof is a recombinant antibody or a fragment thereof. In some embodiments, the antibody or the antigen-binding fragment thereof is a linear antibody, a single-chain antibody, a nanobody, a peptibody, a domain antibody, a diabody, a triabody, a tetrabody, a tandem di-scFv, or a tandem tri-scFv. In some embodiments, the antibody is a monospecific antibody, a bispecific antibody, or a multispecific antibody (e.g., a trispecific antibody). In some embodiments, the antibody or the antigen-binding fragment thereof targets a tumor antigen such as HER2, LIV-1, or TROP2.

In some embodiments, provided is a protein, which is a fusion protein, e.g., a fusion protein of an antibody constant region or a fragment thereof with an additional polypeptide therapeutic or diagnostic agent.

In some embodiments, provided is an antibody constant region or a fragment thereof, which comprises a polypeptide fragment at a N-terminus and/or a C-terminus, and the fragment comprises one or more tyrosine residues. In some embodiments, the fragment is linked to the N-terminus and/or the C-terminus of the antibody constant region or the fragment thereof, either directly or by a linker. In some embodiments, the linker is a GS or polyG linker. In some embodiments, the fragment is YGGGG or YGGGGS and is linked to the N-terminus of the antibody constant region or the fragment thereof by a linker. In some embodiments, the fragment is GGGGY or SGGGGY and is linked to the N-terminus of the antibody constant region or the fragment thereof by a linker. In some embodiments, the antibody constant region or the fragment thereof may comprise the aforementioned N-terminal and C-terminal fragments simultaneously or individually.

In some embodiments, the aforementioned protein does not require removal/reduction of a 297N glycosylation modification.

In some embodiments, when the amino acid at position 298 is not an engineered tyrosine residue, the aforementioned protein does not require removal/reduction of a 297N glycosylation modification.

In some embodiments, in the aforementioned protein, in addition to the site provided by the present disclosure that can be substituted with the engineered tyrosine, other natural tyrosines may comprise mutations, and the amino acid mutations may be conservative replacements, substitutions, or modifications, and/or deletions or additions that do not affect function. In some specific embodiments, the amino acid mutation may occur in a CDR and/or a framework region (FR) and/or a constant region.

In some embodiments, the aforementioned protein comprises the aforementioned antibody or the antigen-binding fragment thereof provided by the present disclosure.

### Conjugate

The present disclosure provides use of the aforementioned antibody or antigen-binding fragment thereof, or protein for the preparation of a conjugate. In some embodiments, the conjugate is a protein-drug conjugate (encompassing an antibody-drug conjugate, an antibody-peptide conjugate, and a polypeptide-drug conjugate (e.g., an antibody constant region or fragment thereof-drug conjugate)).

In some embodiments, the antibody or the antigen-binding fragment thereof, or the protein of the present disclosure does not require an amino acid mutation of 297N (e.g., N297G) to remove glycosylation nor removal/reduction of a 297N glycosylation modification, prior to the preparation of the antibody-drug conjugate.

The present disclosure provides a conjugate comprising a structure represented by formula I or a pharmaceutically acceptable salt thereof:

Pc-[L-(D)ₓ]_{y} I

wherein Pc is any of the aforementioned antibodies or antigen-binding fragments thereof provided by the present disclosure, or any of the aforementioned proteins provided by the present disclosure;
L is a linker covalently linking Pc to D, and L is linked to an amino acid in Pc, wherein the amino acid is located in Pc;
D is a payload;
x is 1 to 20;
y is 1 to 20.

In some embodiments, the amino acid is located in a heavy chain constant region and/or a light chain constant region of Pc. For example, the amino acid is located in a C_{H}1 region, a hinge region, an Fc region, and/or a CL region. For example, the amino acid is located in the C_{H}1 region, the hinge region, the C_{H}2 region, a C_{H}3 region, and/or a Cκ region and a Cλ region.

In some embodiments, the amino acid is located at any one or any combination of positions 135, 137, 192, 223, and 329 of a heavy chain HC and position 202 of a light chain LC of the Pc antibody or the antigen-binding fragment thereof. The above position numbers are defined based on the EU numbering scheme.

In some embodiments, the heavy chain HC is IGHG1.

In some embodiments, the light chain LC is Cκ.

In some embodiments, the heavy chain HC is IGHG2, IGHG3, or IGHG4.

In some embodiments, the light chain LC is Ck.

In other embodiments, the amino acid is located at any one or any combination of positions 135, 137, 192, 223, and 329 of IGHG1 (or located at positions in IGHG2, IGHG3, and IGHG4 corresponding to positions 135, 137, 192, 223, and 329 of IGHG1) and position 202 of Cκ (or a position in Cλ corresponding to position 202 of Kappa LC).

Illustratively, in some embodiments, the amino acid is located in Pc at
position 135 and position 223 of the HC;
position 223 and position 329 of the HC;
position 135 and position 329 of the HC;
position 135, position 223, and position 329 of the HC;
position 135, position 223, position 329, and position 192 of the HC;
position 135 and position 223 of the HC and position 202 of the LC;
position 135 and position 223 of the HC and position 202 of the LC;
position 223 and position 329 of the HC and position 202 of the LC;
position 135 and position 329 of the HC and position 202 of the LC;
position 135, position 223, and position 329 of the HC and position 202 of the LC;
position 135, position 223, position 329, and position 192 of the HC and position 202 of the LC;
position 329 of the HC and position 202 of the LC; or
position 192 of the HC and position 202 of the LC.

In some embodiments, the amino acid is located in a non-glycosylation-modified region of Pc, e.g., not located at position 297 of a heavy chain, or not located within the range of 10 amino acids near position 297 of a heavy chain.

In some embodiments, the Pc comprises a binding domain that specifically binds to a tumor antigen, or is an anti-tumor antibody or an antigen-binding fragment thereof, e.g., an anti-HER2 antibody or an antigen-binding fragment thereof, an anti-LIV-1 antibody or an antigen-binding fragment thereof, or an anti-TROP2 antibody or an antigen-binding fragment thereof.

In some embodiments, the anti-HER2 antibody or the antigen-binding fragment thereof comprises the heavy chain CDRs (HCDRs) and the light chain CDRs (LCDRs), or the heavy chain variable region (VH) and the light chain variable region (VL), of trastuzumab. For example, the anti-HER2 antibody or the antigen-binding fragment thereof comprises a VH and a VL, wherein the VH comprises a HCDR1, a HCDR2, and a HCDR3 of the amino acid sequences set forth in SEQ ID NOs: 51-53, and the VL comprises a LCDR1, a LCDR, and a LCDR3 of the amino acid sequences set forth in SEQ ID NOs: 54-56; or the VH comprises an amino acid sequence having at least 90% identity to SEQ ID NO: 57, and the VL comprises an amino acid sequence having at least 90% identity to SEQ ID NO: 58.

In some embodiments, the anti-HER2 antibody or the antigen-binding fragment thereof comprises a heavy chain and a light chain, wherein:
the heavy chain comprises the amino acid sequence set forth in any one of SEQ ID NOs: 3-10, and the light chain comprises the amino acid sequence set forth in SEQ ID NO: 2; the heavy chain comprises the amino acid sequence set forth in SEQ ID NO: 1, and the light chain comprises the amino acid sequence set forth in SEQ ID NO: 11;
the heavy chain comprises the amino acid sequence set forth in any one of SEQ ID NOs: 24-27, 7, and 9, and the light chain comprises the amino acid sequence set forth in SEQ ID NO: 2 or 11; for example, the heavy chain comprises the amino acid sequence set forth in any one of SEQ ID NOs: 24, 26, 7, and 9, and the light chain comprises the amino acid sequence set forth in SEQ ID NO: 11; or the heavy chain comprises the amino acid sequence set forth in any one of SEQ ID NOs: 25 and 27, and the light chain comprises the amino acid sequence set forth in SEQ ID NO: 2.

In some embodiments, the anti-LIV-1 antibody or the antigen-binding fragment thereof comprises a VH and a VL, wherein the VH comprises a HCDR1, a HCDR2, and a HCDR3 of the amino acid sequences set forth in SEQ ID NOs: 59-61, and the VL comprises a LCDR1, a LCDR, and a LCDR3 of the amino acid sequences set forth in SEQ ID NOs: 62-64; or the VH comprises an amino acid sequence having at least 90% identity to SEQ ID NO: 65, and the VL comprises an amino acid sequence having at least 90% identity to SEQ ID NO: 66.

In some embodiments, the anti-LIV-1 antibody or the antigen-binding fragment thereof comprises a heavy chain and a light chain, wherein the heavy chain comprises the amino acid sequence set forth in any one of SEQ ID NOs: 14-19, and the light chain comprises the amino acid sequence set forth in SEQ ID NO: 13; or the heavy chain comprises the amino acid sequence set forth in SEQ ID NO: 12, and the light chain comprises the amino acid sequence set forth in SEQ ID NO: 20.

In some embodiments, the anti-TROP2 antibody or the antigen-binding fragment thereof comprises a VH and a VL, wherein the VH comprises a HCDR1, a HCDR2, and a HCDR3 of the amino acid sequences set forth in SEQ ID NOs: 68-70, and the VL comprises a LCDR1, a LCDR, and a LCDR3 of the amino acid sequences set forth in SEQ ID NOs: 71-73; or the VH comprises an amino acid sequence having at least 90% identity to SEQ ID NO: 74, and the VL comprises an amino acid sequence having at least 90% identity to SEQ ID NO: 75.

In some embodiments, to reduce non-specific conjugation or reduce the influence of natural tyrosine in the tyrosinase-catalyzed reaction process, the natural tyrosine site of Pc may be mutated, and the amino acid mutation may be a conservative replacement, substitution, or modification, and/or a deletion or addition that does not affect function. In some specific embodiments, the amino acid mutation may occur in a CDR and/or a framework region (FR) and/or a constant region. In some specific embodiments, 296Y and/or 300Y of the antibody heavy chain constant region may be mutated, e.g., to 296F and/or 300F.

In some embodiments, L and Pc in the conjugate form a structure represented by Za or Zb: 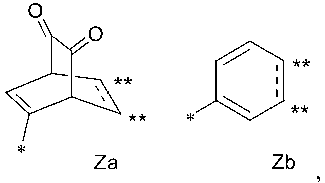 wherein * represents an end linked to Pc, ** represents an end linked to a linker, andrepresents a single bond or a double bond.

In some embodiments, the structure represented by Za in the conjugate is a structure represented by formula Za1 or Za2, 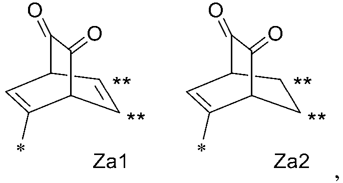 wherein * represents an end linked to Pc, and ** represents an end linked to a linker.

In some embodiments, the structure represented by Zb in the conjugate is a structure represented by formula Zb1 or Zb2, 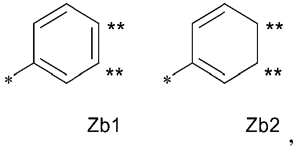 wherein * represents an end linked to Pc, and ** represents an end linked to a linker.

In certain embodiments, the amino acid linked to L is tyrosine and is oxidized by tyrosinase to o-quinone. o-Quinone undergoes a [4+2] cycloaddition reaction with a linker to link Pc to L-(D)x. The [4+2] cycloaddition reaction is well known in the art (see, e.g., WO 2014/065661, which is incorporated for illustrative purposes). In some embodiments, the [4+2] cycloaddition reaction occurs under metal-free conditions.

In some other embodiments, CO in the linker 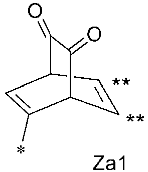 will be removed under certain conditions to form

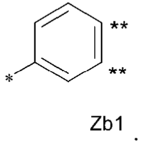

In some embodiments, the linker is stable extracellularly, such that the conjugate comprising the binding domain that specifically binds to a tumor antigen remains intact when present in an extracellular environment, but is capable of being cleaved when internalized in a cell such as a cancer cell. In some embodiments, D is cleaved from the antibody moiety when the conjugate enters a cell expressing a particular tumor antigen antigen.

In some embodiments, the cleavable moiety in the linker is a cleavable peptide moiety. In some embodiments, the conjugate comprising the cleavable peptide moiety shows a lower aggregation level, an improved antibody to drug ratio, increased targeted killing of cancer cells, reduced off-target killing of non-cancer cells, and/or a higher drug loading (p) relative to the conjugate comprising other cleavable moieties.

In some embodiments, the addition of a cleavable moiety increases cytotoxicity and/or potency relative to a non-cleavable linker. In some embodiments, the increased potency and/or cytotoxicity is one in cancers that express moderate levels of a particular tumor antigen.

In some embodiments, the linker of the conjugate comprises a cleavable peptide moiety. In some embodiments, the cleavable peptide moiety is capable of being cleaved by an enzyme, and the linker is a linker capable of being cleaved by an enzyme. In some embodiments, the enzyme is a cathepsin, and the linker is a linker capable of being cleaved by a cathepsin. In certain embodiments, the enzyme-cleavable linker (e.g., the cathepsin-cleavable linker) exhibits one or more of the improved properties described above, as compared to other cleavage mechanisms.

In some embodiments, the linker comprises a cleavable peptide moiety, and the cleavable peptide moiety preferably comprises a peptide residue consisting of 2 to 7 amino acids selected from the group consisting of phenylalanine, glycine, valine, lysine, citrulline, serine, glutamic acid, and aspartic acid, and more preferably valine-citrulline (Val-Cit), alanine-alanine-asparagine (Ala-Ala-Asn), glycine-glycine-lysine (Gly-Gly-lys), valine-lysine (Val-lys), valine-alanine (Val-Ala), valine-phenylalanine (Val-Phe), and glycine-glycine-phenylalanine-glycine (Gly-Gly-Phe-Gly).

In some embodiments, the cleavable peptide moiety comprises valine-citrulline (Val-Cit). In some embodiments, the ADC comprising Val-Cit shows increased stability, reduced off-target cell killing, increased targeted cell killing, a lower aggregation level, and/or a higher drug loading relative to the ADC comprising other amino acid units or other cleavable moieties.

In another aspect, a linker provided by some embodiments comprises a cleavable sulfonamide moiety, and the linker is capable of being cleaved under reduced conditions. In some embodiments, the linker comprises a cleavable disulfide moiety, and the linker is capable of being cleaved under reduced conditions.

In another aspect, the linker in the conjugate of the present disclosure comprises at least one spacer unit that links D to a cleavable moiety.

In some embodiments, the spacer unit comprises p-aminobenzyloxycarbonyl (PAB),

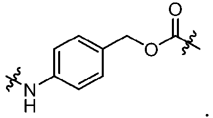

In some embodiments, the spacer unit comprises p-aminobenzoyl

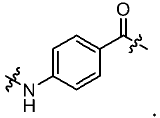

In some other embodiments, the spacer unit comprises the following moieties selected from the group consisting of:

-(CR¹R²)ₘ₁-O(CR¹R²)ₘ₂-CR³R⁴-C(O)-,

-(CR¹R²)ₘ₁NH-(CR¹R²)ₘ₂-CR³R⁴-C(O)-,

-(CR¹R²)ₘ₁O-CR³R⁴(CR¹R²)ₘ₂-,

-(CR¹R²)ₘ₁OCR³R⁴-C(O)-,

-(CR¹R²)ₘ₁-O-(CR¹R²)ₘ₂C(O)-, and -(CR¹R²)ₘ₁-S-(CR¹R²)ₘ₂-CR³R⁴-C(O)-,

wherein R¹ and R² are identical or different and are each independently selected from the group consisting of hydrogen, halogen, and alkyl; R³ is selected from the group consisting of hydrogen, C₃₋₆ cycloalkylalkyl, and C₃₋₆ cycloalkyl; R⁴ is selected from the group consisting of hydrogen, haloalkyl, and C₃₋₆ cycloalkyl, preferably hydrogen; or R³ and R⁴, together with the carbon atom to which they are attached, form C₃₋₆ cycloalkyl; m1 and m2 are each independently selected from the group consisting of 0, 1, 2, and 3.

In some embodiments, R³ is selected from the group consisting of hydrogen, and R⁴ is selected from the group consisting of C₃₋₆ cycloalkyl, e.g., cyclopropyl or cyclobutyl. In some embodiments, R³ is selected from the group consisting of C₃₋₆ cycloalkyl, e.g., cyclopropyl or cyclobutyl, and R⁴ is selected from the group consisting of hydrogen.

In some embodiments, R³ and R⁴, together with the carbon atom to which they are attached, form C₃₋₆ cycloalkyl, e.g., cyclopropyl or cyclobutyl.

In some embodiments, the spacer unit comprises the following moieties selected from the group consisting of: -(CH₂)₃-C(O)-, -CH₂-O-CH₂-C(O)-, -(CH₂)₂-O-CH₂-C(O)-,

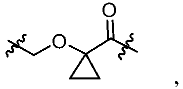

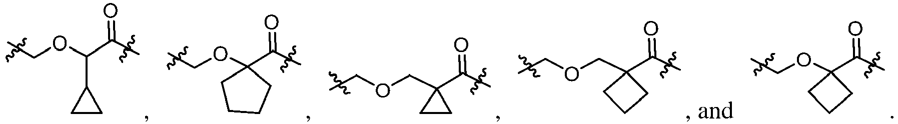

In some embodiments, the linker may comprise at least one polyethylene glycol (PEG) moiety. The PEG moiety may comprise, for example, -(PEG)ₚ₁-, 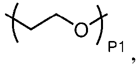 wherein p1 is an integer from 1 to 20, e.g., 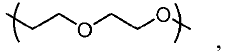 (PEG)₂; 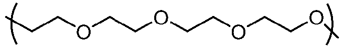 , (PEG)₄; or 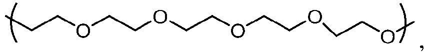 (PEG)₅.

In some other embodiments, the ADC comprising a shorter stretcher unit (e.g., (PEG)₂) shows a lower aggregation level and/or a higher drug loading relative to the ADC comprising a longer stretcher unit (e.g., (PEG)₈) despite the shorter linker length.

In another aspect, the linker L in the conjugate of the present disclosure comprises a stretcher unit (Str) covalently linked to Pc. In some embodiments, the stretcher unit Str in the linker serves as a structural unit linked to o-quinone, or the stretcher unit Str in the linker undergoes a [4+2] cycloaddition reaction with o-quinone to link Pc to L-(D)ₓ. The [4+2] cycloaddition reaction is well known in the art (see, e.g., WO 2014/065661, which is incorporated for illustrative purposes). In some embodiments, the [4+2] cycloaddition reaction occurs under metal-free conditions.

In some embodiments, the stretcher unit Str in the linker comprises cycloalkenyl, cycloalkynyl, heterocycloalkenyl, or heterocycloalkynyl. The cycloalkenyl, cycloalkynyl, cycloalkenyl, or heterocycloalkynyl herein may be optionally substituted.

In some embodiments, the stretcher unit Str in the linker comprises cycloalkynyl or heterocycloalkynyl.

In some embodiments, the stretcher unit Str in the conjugate comprises cycloheptynyl, cyclooctynyl, cyclononyl, cyclodecynyl, heterocycloheptynyl, heterocyclooctynyl, heterocyclononyl, or heterocyclodecynyl.

In some embodiments, the stretcher unit Str in the conjugate comprises a group selected from the group consisting of the following:

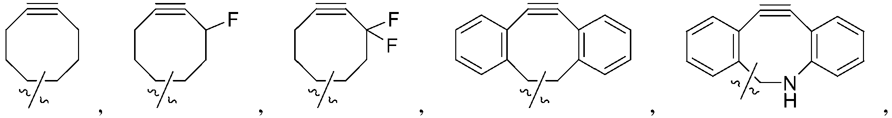

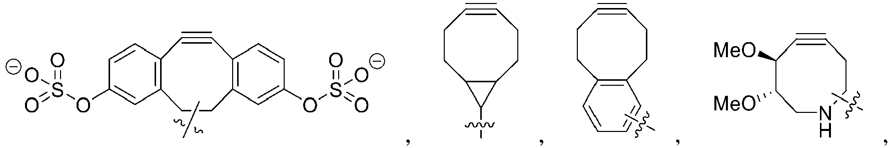

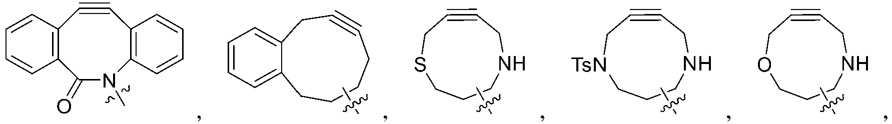

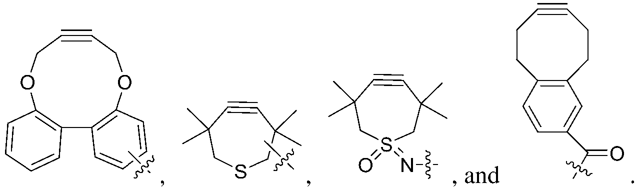

In some embodiments, the stretcher unit Str in the conjugate comprises a group selected from the group consisting of the following: 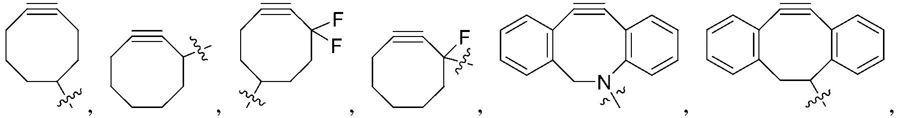 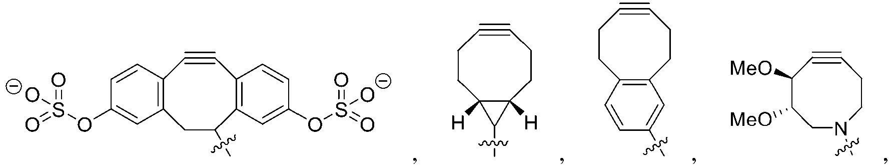 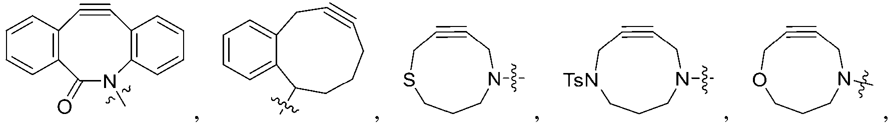 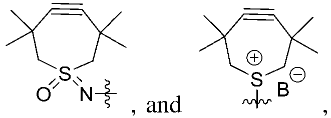 wherein S⁽⁺⁾ is a cationic sulfur atom balanced by an anion B⁽⁻⁾.

In another aspect, in some embodiments, the stretcher unit Str in the linker and Pc form the following structure: 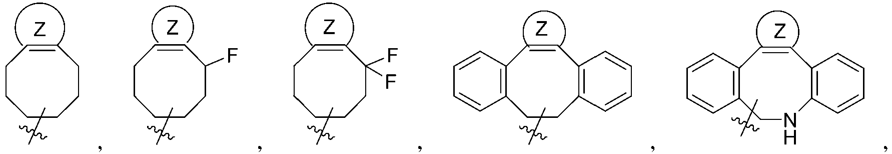 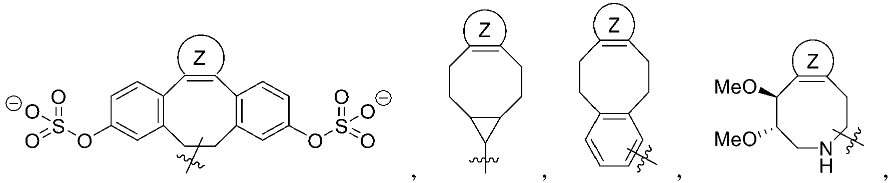 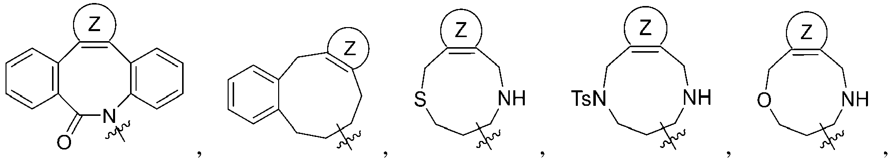 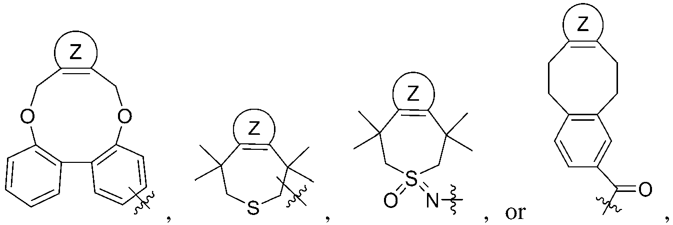 wherein ring Z is

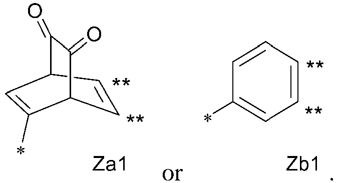

In some other embodiments, the stretcher unit Str in the linker and Pc form the following structure: 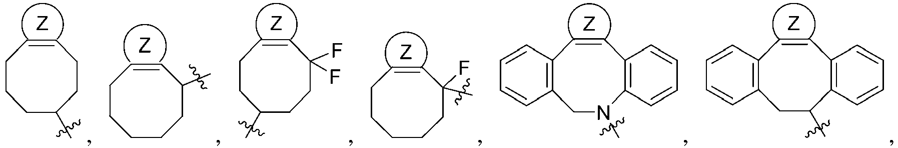 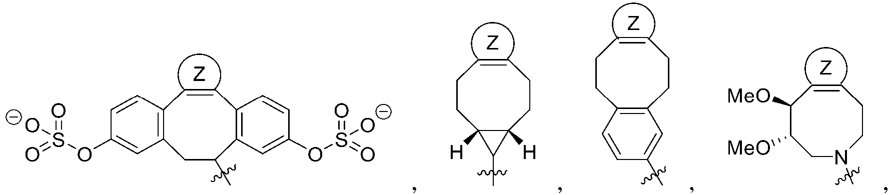 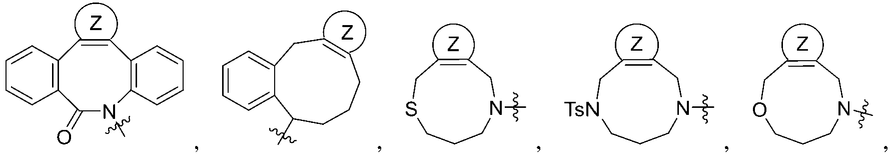 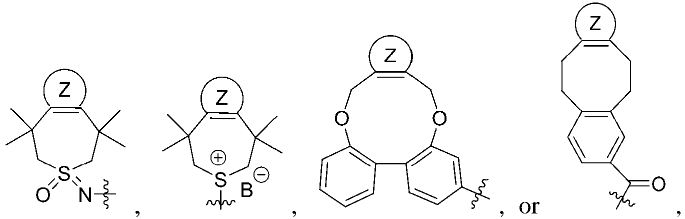 wherein ring Z is 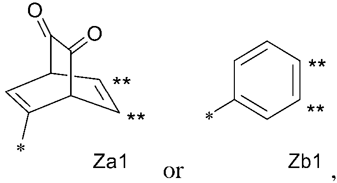 and S⁽⁺⁾ is a cationic sulfur atom balanced by an anion B⁽⁻⁾.

In certain embodiments, the stretcher unit Str in the linker and Pc form 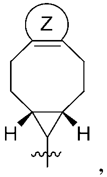 wherein ring Z is

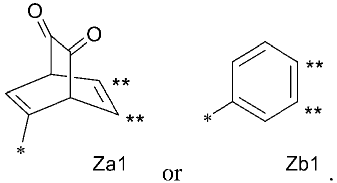

In certain embodiments, the stretcher unit Str in the linker and Pc form 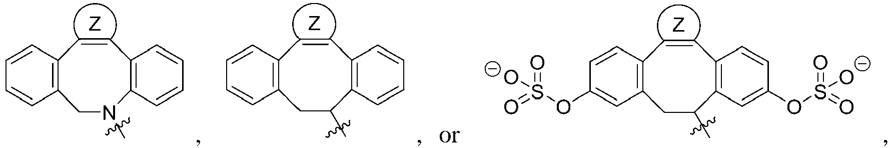 wherein ring Z is

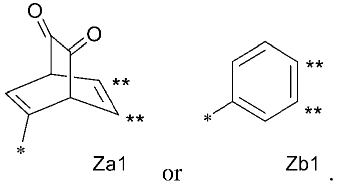

In certain embodiments, the stretcher unit Str in the linker and Pc form 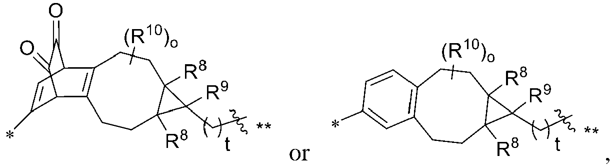
wherein each R⁸ is independently selected from the group consisting of hydrogen, halogen, C₁₋₆ alkyl, C₁₋₆ alkoxy, C₆ aryl, and C₅₋₁₀ heteroaryl, wherein the alkyl, aryl, or heteroaryl is optionally substituted with one or more halogen, C₁₋₃ alkyl, or C₁₋₃ alkoxy groups;
R⁹ is selected from the group consisting of hydrogen, halogen, C₁₋₆ alkyl, C₁₋₆ alkoxy, C₆ aryl, and C₅₋₁₀ heteroaryl, wherein the alkyl, aryl, or heteroaryl is optionally substituted with one or more halogen, C₁₋₃ alkyl, or C₁₋₃ alkoxy groups;
each R¹⁰ is independently selected from the group consisting of halogen, C₁₋₆ alkyl, C₁₋₆ alkoxy, C₃₋₆ cycloalkyl, C₃₋₆ heterocycloalkyl, C₆ aryl, and C₅₋₁₀ heteroaryl, wherein the alkyl, alkoxy, cycloalkyl, heterocycloalkyl, aryl, or heteroaryl is optionally substituted with one or more halogen, C₁₋₃ alkyl, or C₁₋₃ alkoxy groups;
t is an integer from 0 to 10;
o is an integer from 0 to 10;
* represents an end linked to Pc, and ** represents an end linked to a linker.

In certain embodiments, the stretcher unit Str in the linker and Pc form 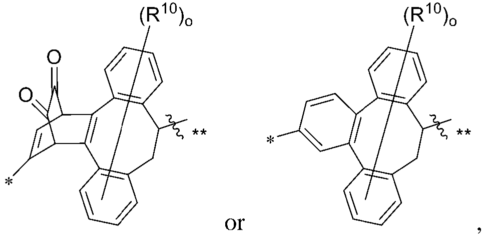
wherein each R¹⁰ is independently selected from the group consisting of halogen, C₁₋₆ alkyl, C₁₋₆ alkoxy, C₃₋₆ cycloalkyl, C₃₋₆ heterocycloalkyl, C₆ aryl, and C₅₋₁₀ heteroaryl, wherein the alkyl, alkoxy, cycloalkyl, heterocycloalkyl, aryl, or heteroaryl is optionally substituted with one or more halogen, C₁₋₃ alkyl, or C₁₋₃ alkoxy groups;
o is an integer from 0 to 10;
* represents an end linked to Pc, and ** represents an end linked to a linker.

In certain embodiments, the stretcher unit Str in the linker and Pc form 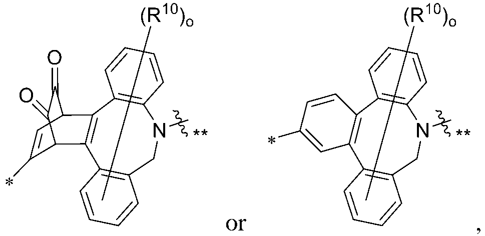
wherein each R¹⁰ is independently selected from the group consisting of halogen, C₁₋₆ alkyl, C₁₋₆ alkoxy, C₃₋₆ cycloalkyl, C₃₋₆ heterocycloalkyl, C₆ aryl, and C₅₋₁₀ heteroaryl, wherein the alkyl, alkoxy, cycloalkyl, heterocycloalkyl, aryl, or heteroaryl is optionally substituted with one or more halogen, C₁₋₃ alkyl, or C₁₋₃ alkoxy groups;
o is an integer from 0 to 10;
* represents an end linked to Pc, and ** represents an end linked to a linker.

In another aspect, the stretcher unit Str of the linker in the conjugate provided by some embodiments comprises cycloalkenyl or heterocycloalkenyl.

In certain embodiments, the stretcher unit Str in the linker comprises a group selected from the group consisting of the following: 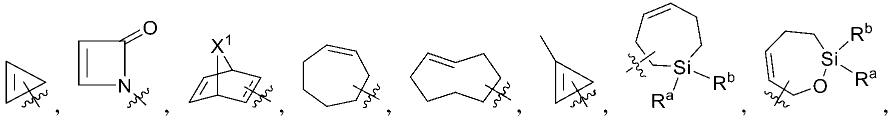 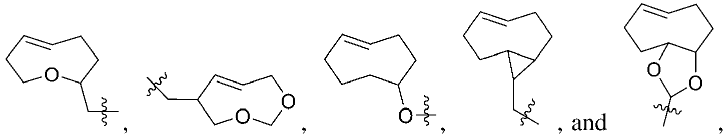 wherein R" and R^{b} are each independently selected from the group consisting of hydrogen, C₁₋₆ alkyl, and aryl; X¹ is selected from the group consisting of C₁₋₃ alkylene, NR^{c}, and O; R^{c} is selected from the group consisting of hydrogen and C₁₋₆ alkyl.

In some other embodiments, the stretcher unit Str in the linker and Pc form the following structure: 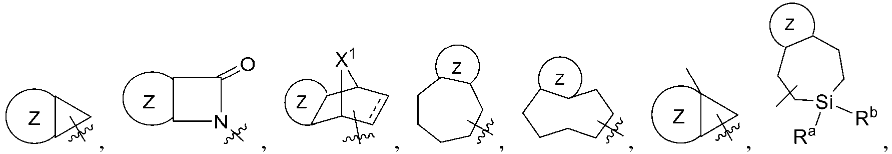 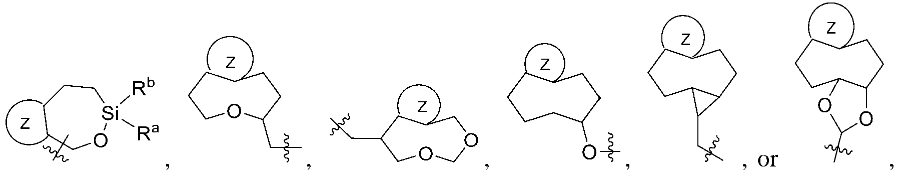 wherein R^{a}, R^{b}, and X¹ are as previously defined; ring Z is 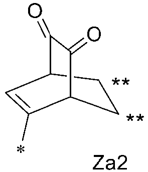 or

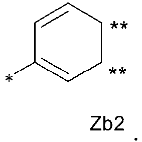

In certain embodiments, the stretcher unit Str in the linker and Pc form 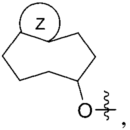 wherein ring Z is

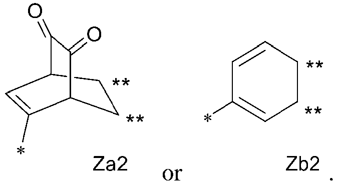

In certain embodiments, the stretcher unit Str in the linker and Pc form 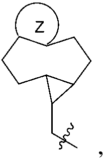 wherein ring Z is

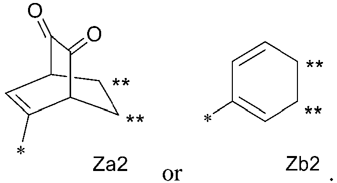

In certain embodiments, the stretcher unit Str in the linker and Pc form 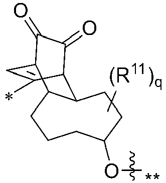 or 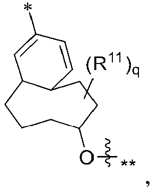
wherein each R¹¹ is independently selected from the group consisting of halogen, C₁₋₆ alkyl, C₁₋₆ alkoxy, C₃₋₆ cycloalkyl, C₃₋₆ heterocycloalkyl, C₆ aryl, and C₅₋₁₀ heteroaryl, wherein the alkyl, alkoxy, cycloalkyl, heterocycloalkyl, aryl, or heteroaryl is optionally substituted with one or more halogen, C₁₋₃ alkyl, or C₁₋₃ alkoxy groups;
q is an integer from 0 to 10;
* represents an end linked to Pc, and ** represents an end linked to a linker.

In some other embodiments, the stretcher unit Str in the linker and Pc form the following structure: 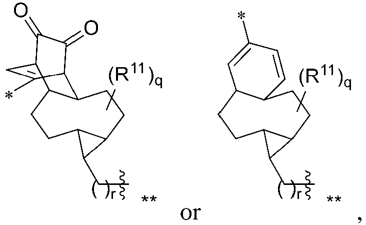
wherein each R¹¹ is independently selected from the group consisting of hydrogen, halogen, C₁₋₆ alkyl, C₁₋₆ alkoxy, C₆ aryl, and C₅₋₁₀ heteroaryl, wherein the alkyl, aryl, or heteroaryl is optionally substituted with one or more halogen, C₁₋₆ alkyl, C₁₋₆ alkoxy, C₃₋₆ cycloalkyl, C₃₋₆ heterocycloalkyl, C₆ aryl, or C₅₋₁₀ heteroaryl groups, and the alkyl, alkoxy, cycloalkyl, heterocycloalkyl, aryl, or heteroaryl is optionally substituted with one or more halogen, C₁₋₃ alkyl, or C₁₋₃ alkoxy groups;
q is an integer from 0 to 10;
r is an integer from 0 to 10;
* represents an end linked to Pc, and ** represents an end linked to a linker.

The linker in the conjugate provided by some other embodiments comprises a chemical moiety represented by the following formula: 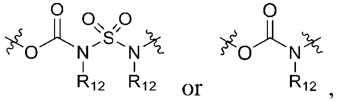 wherein each R¹² is independently selected from the group consisting of hydrogen, C₁₋₃ alkyl, and C₃₋₆ cycloalkyl.

In some embodiments, the linker comprises a chemical moiety represented by the following formula:

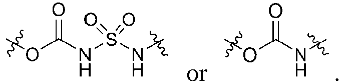

The linker in the antibody conjugate provided by some embodiments comprises a chemical moiety represented by the following formula: 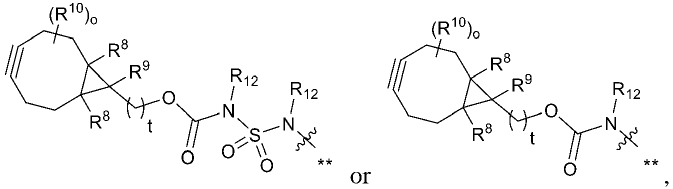
wherein each R⁸ is independently selected from the group consisting of hydrogen, halogen, C₁₋₆ alkyl, C₁₋₆ alkoxy, C₆ aryl, and C₅₋₁₀ heteroaryl, wherein the alkyl, aryl, or heteroaryl is optionally substituted with one or more halogen, C₁₋₃ alkyl, or C₁₋₃ alkoxy groups;
R⁹ is selected from the group consisting of hydrogen, halogen, C₁₋₆ alkyl, C₁₋₆ alkoxy, C₆ aryl, and C₅₋₁₀ heteroaryl, wherein the alkyl, aryl, or heteroaryl is optionally substituted with one or more halogen, C₁₋₃ alkyl, or C₁₋₃ alkoxy groups;
each R¹⁰ is independently selected from the group consisting of halogen, C₁₋₆ alkyl, C₁₋₆ alkoxy, C₃₋₆ cycloalkyl, C₃₋₆ heterocycloalkyl, C₆ aryl, and C₅₋₁₀ heteroaryl, wherein the alkyl, alkoxy, cycloalkyl, heterocycloalkyl, aryl, or heteroaryl is optionally substituted with one or more halogen, C₁₋₃ alkyl, or C₁₋₃ alkoxy groups;
t is an integer from 0 to 10;
o is an integer from 0 to 10;
** represents an end linked to a linker.

The linker in the conjugate provided by some embodiments comprises a chemical moiety represented by the following formula: 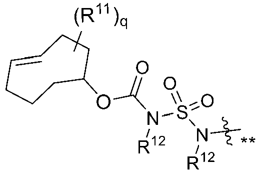 or 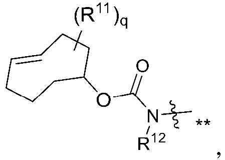
wherein each R¹¹ is independently selected from the group consisting of halogen, C₁₋₆ alkyl, C₁₋₆ alkoxy, C₃₋₆ cycloalkyl, C₃₋₆ heterocycloalkyl, C₆ aryl, and C₅₋₁₀ heteroaryl, wherein the alkyl, alkoxy, cycloalkyl, heterocycloalkyl, aryl, or heteroaryl is optionally substituted with one or more halogen, C₁₋₃ alkyl, or C₁₋₃ alkoxy groups;
each R¹² is independently selected from the group consisting of hydrogen, C₁₋₃ alkyl, and C₃₋₆ cycloalkyl;
q is an integer from 0 to 10;
** represents an end linked to a linker.

The linker in the conjugate provided by some embodiments comprises a chemical moiety represented by the following formula: 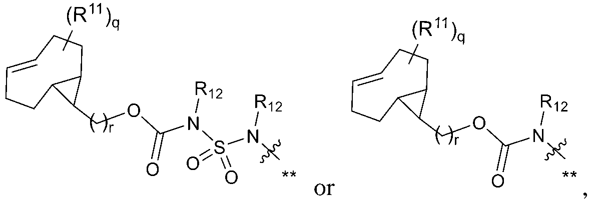
wherein each R¹¹ is independently selected from the group consisting of halogen, C₁₋₆ alkyl, C₁₋₆ alkoxy, C₃₋₆ cycloalkyl, C₃₋₆ heterocycloalkyl, C₆ aryl, and C₅₋₁₀ heteroaryl, wherein the alkyl, alkoxy, cycloalkyl, heterocycloalkyl, aryl, or heteroaryl is optionally substituted with one or more halogen, C₁₋₃ alkyl, or C₁₋₃ alkoxy groups;
each R¹² is independently selected from the group consisting of hydrogen, C₁₋₃ alkyl, and C₃₋₆ cycloalkyl;
q is an integer from 0 to 10;
r is an integer from 0 to 10;
** represents an end linked to a linker.

The linker in the conjugate provided by some embodiments comprises a chemical moiety represented by the following formula: 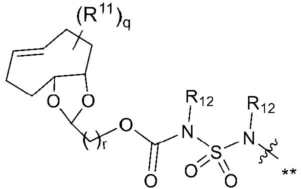 or 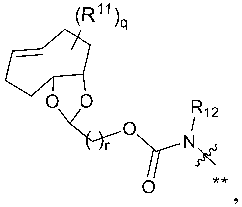
wherein each R¹¹ is independently selected from the group consisting of halogen, C₁₋₆ alkyl, C₁₋₆ alkoxy, C₃₋₆ cycloalkyl, C₃₋₆ heterocycloalkyl, C₆ aryl, and C₅₋₁₀ heteroaryl, wherein the alkyl, alkoxy, cycloalkyl, heterocycloalkyl, aryl, or heteroaryl is optionally substituted with one or more halogen, C₁₋₃ alkyl, or C₁₋₃ alkoxy groups;
each R¹² is independently selected from the group consisting of hydrogen, C₁₋₃ alkyl, and C₃₋₆ cycloalkyl;
q is an integer from 0 to 10;
r is an integer from 0 to 10;
** represents an end linked to a linker.

Further, L-(D)ₓ in the antibody conjugate (ADC) provided by some embodiments is a chemical moiety represented by the following formula:

-Str-(Pep)-Sp-(D)ₓ

wherein Str is a stretcher unit covalently linking to Pc;
Sp is a spacer unit;
Pep is a cleavable peptide moiety.

L-(D)ₓ in the conjugate provided by some embodiments comprises the following chemical moiety: 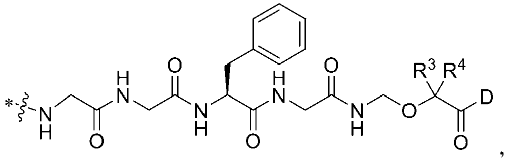 or 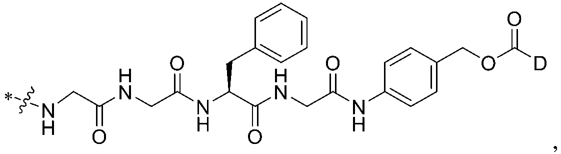 wherein R³ is selected from the group consisting of hydrogen, C₃₋₆ cycloalkylalkyl, and C₃₋₆ cycloalkyl, preferably hydrogen; R⁴ is selected from the group consisting of hydrogen, haloalkyl, and C₃₋₆ cycloalkyl, preferably hydrogen; or R³ and R⁴, together with the carbon atom to which they are attached, form C₃₋₆ cycloalkyl; * represents an end linked to Pc.

L-(D)ₓ in the conjugate provided by some embodiments is a chemical moiety represented by the following formula: 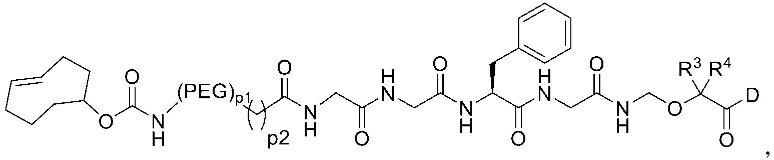 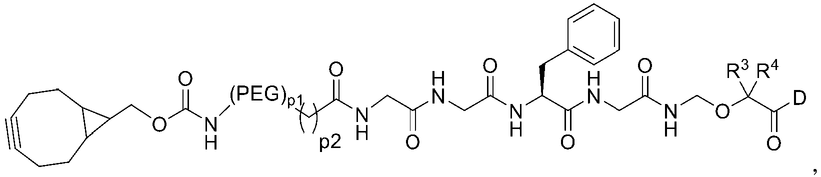 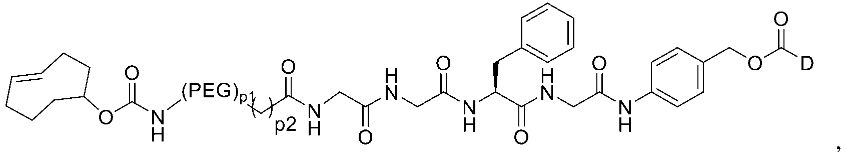 or 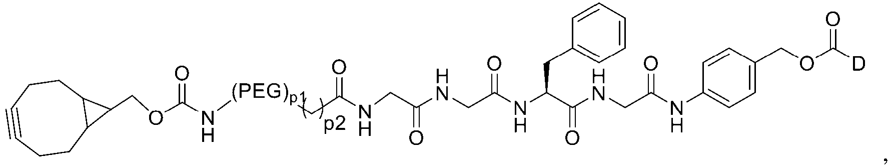 wherein R³ is selected from the group consisting of hydrogen, C₃₋₆ cycloalkylalkyl, and C₃₋₆ cycloalkyl, preferably hydrogen; R⁴ is selected from the group consisting of hydrogen, haloalkyl, and C₃₋₆ cycloalkyl, preferably hydrogen; or R³ and R⁴, together with the carbon atom to which they are attached, form C₃₋₆ cycloalkyl; p1 is selected from the group consisting of 2, 4, 6, and 8, and p2 is selected from the group consisting of 0, 1, and 2.

L-(D)ₓ in the conjugate provided by some embodiments is a chemical moiety represented by the following formula: 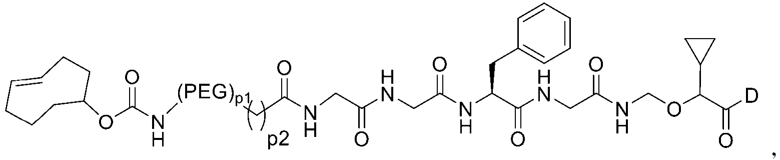 including 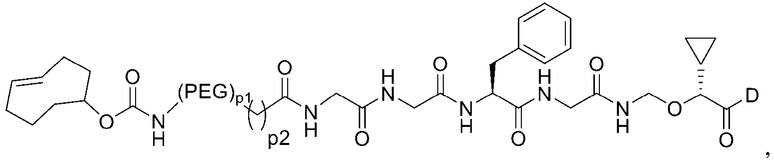 wherein p1 is selected from the group consisting of 2, 4, 6, and 8, and p2 is selected from the group consisting of 0, 1, and 2.

L-(D)ₓ in the conjugate provided by some embodiments is a chemical moiety represented by the following formula: 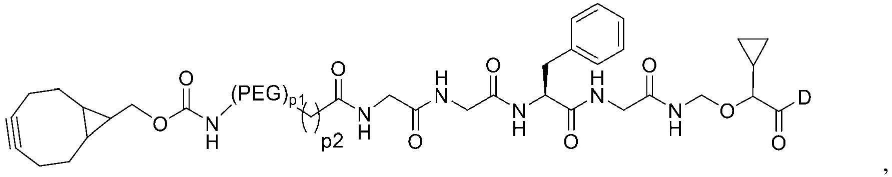 including 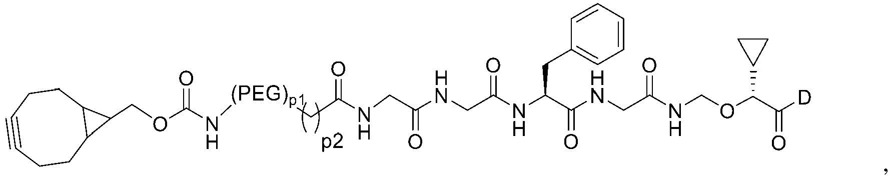 wherein p1 is selected from the group consisting of 2, 4, 6, and 8, and p2 is selected from the group consisting of 0, 1, and 2.

Further, the conjugate provided by some embodiments is represented by the following formula: 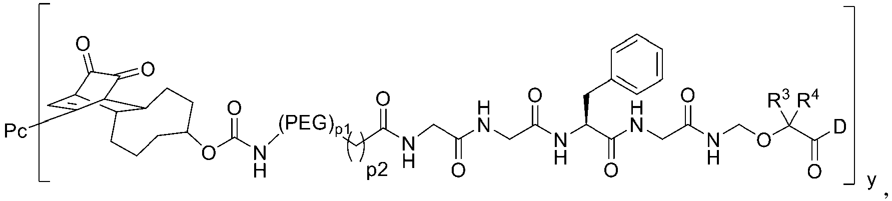 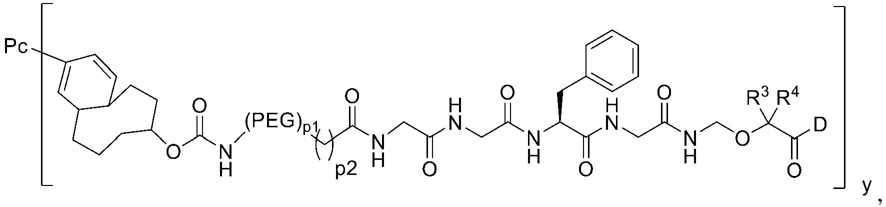 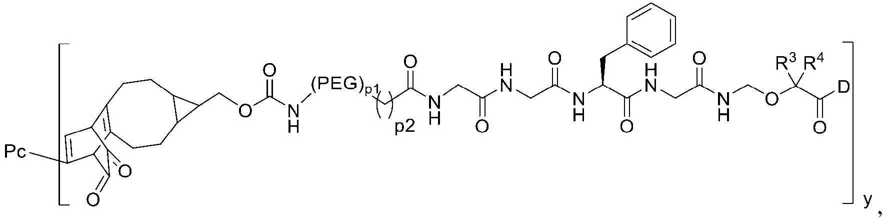 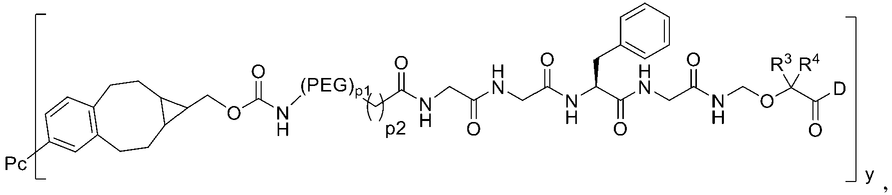 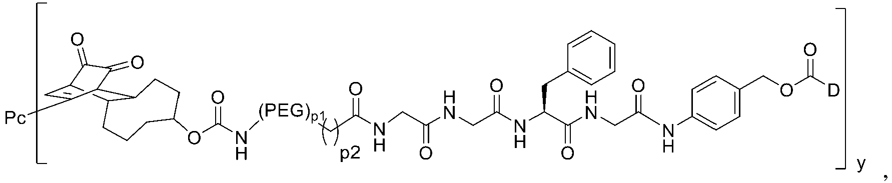 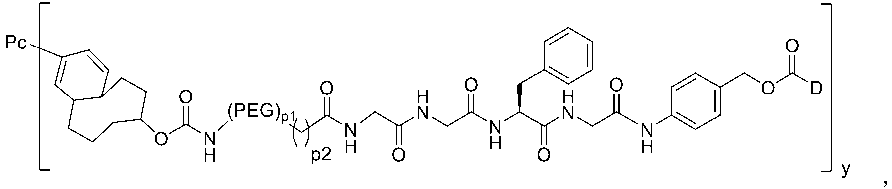 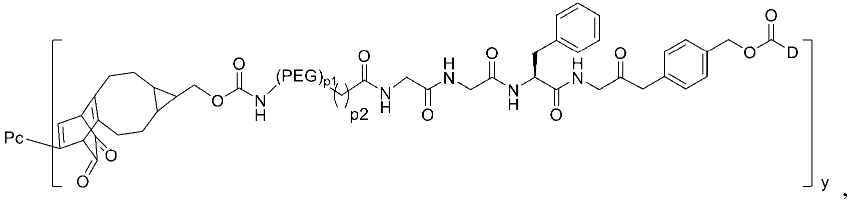 or 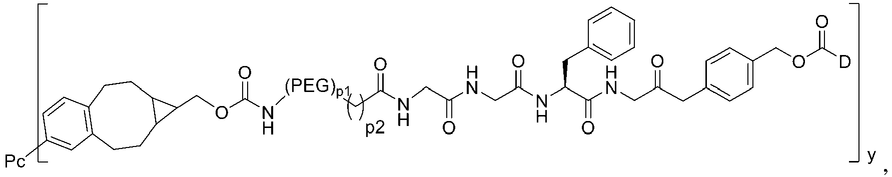 wherein R³ is selected from the group consisting of hydrogen, C₃₋₆ cycloalkylalkyl, and C₃₋₆ cycloalkyl, preferably hydrogen; R⁴ is selected from the group consisting of hydrogen, haloalkyl, and C₃₋₆ cycloalkyl, preferably hydrogen; or R³ and R⁴, together with the carbon atom to which they are attached, form C₃₋₆ cycloalkyl; y is selected from the group consisting of 1 to 20 and may be an integer or a decimal; p1 is selected from the group consisting of 2, 4, 6, and 8, and p2 is selected from the group consisting of 0, 1, and 2; Pc is an antibody or an antigen-binding fragment thereof or an antibody constant region or a fragment thereof.

In another aspect, L-(D)ₓ in the conjugate is a chemical moiety represented by the following formula:

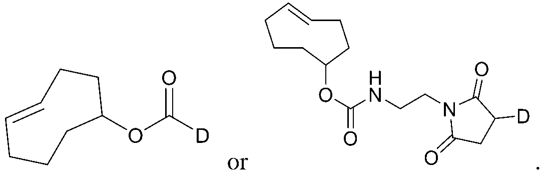

Further, the conjugate provided by some embodiments is represented by the following formula: 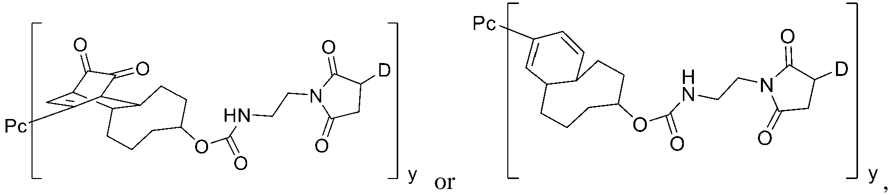 wherein y is selected from the group consisting of 1 to 20 and may be an integer or a decimal; Pc is an antibody or an antigen-binding fragment thereof.

In another aspect, the payload D described in the present disclosure is selected from the group consisting of a therapeutic agent and a diagnostic agent.

In some embodiments, the therapeutic agent is a radioactive agent, a cytotoxic agent, a nucleic acid, or a polypeptide. In some embodiments, the diagnostic agent includes, but is not limited to, a fluorophore, a fluorescent dye, a radionuclide, or an enzyme.

In some embodiments, the payload D is a topoisomerase I inhibitor or a microtubule inhibitor. In some embodiments, the topoisomerase I inhibitor includes, but is not limited to, exatecan or a derivative thereof. In some other embodiments, the microtubule inhibitor includes, but is not limited to, eribulin or a derivative thereof.

In some embodiments, the payload D is a nucleic acid, e.g., an RNA, including siRNA, saRNA, or shRNA.

In some embodiments, the payload D is a radionuclide.

In some embodiments, the payload D is a polypeptide. In some embodiments, a cysteine residue of the polypeptide is linked to the linker L by a covalent bond. In some embodiments, a terminal cysteine residue of the polypeptide is linked to the linker L by a covalent bond. In some embodiments, the polypeptide does not comprise cysteine, and a cysteine residue may be introduced to a terminus or side chain and then linked to the linker L by a covalent bond to form, for example, an antibody-peptide conjugate (APC). In some embodiments, a tyrosine residue of the polypeptide is linked to the linker L by a covalent bond. In some embodiments, a terminal tyrosine residue of the polypeptide is linked to the linker L by a covalent bond. In some embodiments, the polypeptide does not comprise tyrosine, and a tyrosine residue may be introduced to a terminus or side chain and then linked to the linker L by a covalent bond to form, for example, an antibody-peptide conjugate (APC).

The conjugate provided by some embodiments may be an antibody-drug conjugate (ADC) or an antibody-peptide conjugate, e.g., a conjugate of an antibody constant region or a fragment thereof (e.g., Fc) and a drug, or a conjugate of an antibody constant region or a fragment thereof (C_{H}1, hinge, C_{H}2, and/or CL) and a drug. In some specific embodiments, the antibody constant region or the fragment thereof is 1) to 5) described above.

### Preparation Method for Conjugate

The present disclosure provides a method for preparing a conjugate, which comprises:
a) providing a protein comprising an engineered tyrosine residue;
b) contacting with tyrosinase to convert the phenol moiety of the engineered tyrosine residue into an o-quinone moiety, and
c) subjecting the o-quinone moiety to a [4+2] cycloaddition reaction with an alkene or alkyne compound, wherein the alkene or alkyne compound comprises a cycloalkenyl, heterocycloalkenyl, cycloalkynyl, or heterocycloalkynyl moiety;
   the protein is any of the aforementioned proteins provided by the present disclosure, and the protein comprises an antibody constant region or a fragment thereof. Illustratively: In some embodiments, the engineered tyrosine residue in the antibody constant region or the fragment thereof is obtained by mutating (e.g., substituting or replacing) a natural amino acid residue to tyrosine.

In some embodiments, the engineered tyrosine residue is located in a C_{H}1 region, a hinge region, an Fc region, and/or a CL region of the antibody constant region or the fragment thereof. In some embodiments, the residue is located in the C_{H}1 region, the hinge region, a C_{H}2 region, and/or a Cκ region and a Cλ region.

In some embodiments, the engineered tyrosine residue is located at any one or any combination of positions 135, 137, 192, 223, 298, and 329 of the heavy chain HC and position 202 of the light chain LC. In some other embodiments, the engineered tyrosine is located at any one or any combination of positions 135, 137, 192, 223, and 329 of the heavy chain HC and position 202 of the light chain LC.

In some embodiments, the engineered tyrosine is selected from the group consisting of any one or any combination of 135Y, 137Y, 192Y, 223Y, 298Y, and 329Y of the heavy chain HC and 202Y of the light chain LC, for example, it is selected from the group consisting of any one or any combination of T135Y, G137Y, S192Y, T223Y, S298Y, and P329Y, and S202Y of the light chain LC. In some other embodiments, the engineered tyrosine is selected from the group consisting of any one or any combination of 135Y, 137Y, 192Y, 223Y, and 329Y of the heavy chain HC and 202Y of the light chain LC, for example, it is selected from the group consisting of any one or any combination of T135Y, G137Y, S192Y, T223Y, and P329Y, and S202Y of the light chain LC.

In some embodiments, the engineered tyrosine is located at any one or any combination of positions 135, 137, 192, 223, 298, and 329 of IGHG1 (or located at positions in IGHG2, IGHG3, and IGHG4 corresponding to positions 135, 137, 192, 223, 298, and 329 of IGHG1) and position 202 of Cκ. In other embodiments, the engineered tyrosine is located at any one or any combination of positions 135, 137, 192, 223, and 329 of IGHG1 (or located at positions in IGHG2, IGHG3, and IGHG4 corresponding to positions 135, 137, 192, 223, and 329 of IGHG1) and position 202 of Cκ.

In some embodiments, the alkene or alkyne compound comprises a cycloalkenyl, heterocycloalkenyl, cycloalkynyl, or heterocycloalkynyl moiety, and optionally comprises a payload D.

In some embodiments, the alkene or alkyne compound comprises a cycloalkenyl, heterocycloalkenyl, cycloalkynyl, or heterocycloalkynyl moiety, and a payload D.

In some embodiments, the o-quinone moiety in step b) undergoes a [4+2] cycloaddition reaction with the alkene or alkyne compound to form a structure represented by Za or Zb, 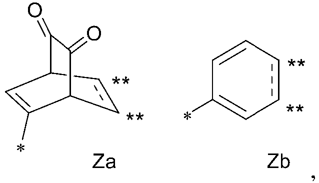 wherein * represents an end linked to Pc, ** represents an end linked to a linker, and ---- represents a single bond or a double bond.

In some embodiments, the structure represented by Za is a structure represented by formula Za1 or Za2, 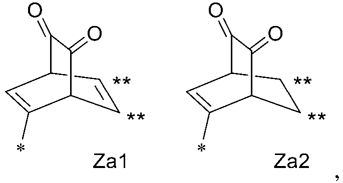 wherein * represents an end linked to Pc, and ** represents an end linked to a linker.

In some embodiments, the structure represented by Zb in the antibody conjugate is a structure represented by formula Zb1 or Zb2, 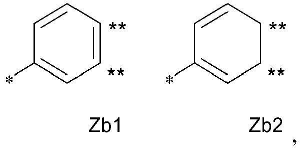 wherein * represents an end linked to Pc, and ** represents an end linked to a linker.

In some embodiments, the aforementioned tyrosinase is tyrosine oxidase.

In some embodiments, the aforementioned tyrosinase is derived from *Bacillus megaterium* or *Verrucomicrobium spinosum.* In some embodiments, the aforementioned tyrosinase is megaTYR derived from *Bacillus megaterium,* with the amino acid sequence set forth in SEQ ID NO: 21 or an amino acid sequence having at least 90% sequence identity thereto; or is VsTYR core derived from *Verrucomicrobium spinosum,* with the amino acid sequence set forth in SEQ ID NO: 22 or an amino acid sequence having at least 90% sequence identity thereto; or is VsTYR sp derived from *Verrucomicrobium spinosum,* with the amino acid sequence set forth in SEQ ID NO: 23 or an amino acid sequence having at least 90% sequence identity thereto. The tyrosinase may be obtained by conventional preparation methods in the art, such as the method in Example 6 of the present application.

The present disclosure further provides a protein comprising an antibody constant region or a fragment thereof, wherein the antibody constant region or the fragment thereof comprises an o-quinone chemical moiety. In some embodiments, the protein is any of the aforementioned proteins provided by the present disclosure.

In some embodiments, the o-quinone chemical moiety is located in a C_{H}1 region, a hinge region, an Fc region, and/or a CL region of the antibody constant region or the fragment thereof. In some embodiments, the o-quinone chemical moiety is located in the C_{H}1 region, the hinge region, a C_{H}2 region, and/or a Cκ region.

In some embodiments, the o-quinone chemical moiety is located at any one or any combination of the amino acids at positions 135, 137, 192, 223, 298, and 329 of the heavy chain HC and the amino acid at position 202 of the light chain LC of the antibody constant region or the fragment thereof. In some other embodiments, the o-quinone chemical moiety is located at any one or any combination of the amino acids at positions 135, 137, 192, 223, and 329 of the heavy chain HC and the amino acid at position 202 of the light chain LC of the antibody constant region or the fragment thereof.

In some embodiments, the o-quinone chemical moiety is located in a non-glycosylation-modified region of the antibody constant region or the fragment thereof, e.g., not located at position 297 (297N) of the heavy chain HC or not located within the range of 10 amino acids near position 297; e.g., not located between the amino acid residues at positions 292 and 302, positions 293 and 303, positions 291 and 301, positions 293 and 302, or positions 292 and 301 of the heavy chain HC.

In some embodiments, the o-quinone chemical moiety is located on an engineered tyrosine residue, and the engineered tyrosine residue is selected from the group consisting of any one or any combination of 135Y, 137Y, 192Y, 223Y, and 329Y of the heavy chain HC and 202Y of the light chain LC, for example, it is selected from the group consisting of any one or any combination of T135Y, G137Y, S192Y, T223Y, and P329Y, and S202Y of the light chain LC.

In some embodiments, the o-quinone chemical moiety is located on an engineered tyrosine residue, and the engineered tyrosine is located at any one or any combination of positions 135, 137, 192, 223, and 329 of IGHG1 (or located at positions in IGHG2, IGHG3, and IGHG4 corresponding to positions 135, 137, 192, 223, and 329 of IGHG1) and position 202 of the Cκ region.

In some embodiments, the o-quinone chemical moiety is obtained by oxidizing a tyrosine residue with tyrosinase.

In some embodiments, the o-quinone chemical moiety is shown below: 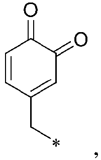 wherein * represents an end linked to Pc.

The present disclosure further provides an isotopically substituted form of the aforementioned conjugate or the pharmaceutically acceptable salt thereof. In some embodiments, the isotopically substituted form is a deuterated form.

In another aspect, the present disclosure further provides a pharmaceutical composition comprising an effective amount of the aforementioned conjugate or pharmaceutically acceptable salt thereof or deuterated form thereof, and a pharmaceutically acceptable excipient.

The pharmaceutically acceptable salt of the antibody-drug conjugate described in the present disclosure may be selected from the group consisting of an inorganic salt and an organic salt.

The compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure. The compounds of the present disclosure containing asymmetric carbon atoms may be separated in optically active pure form or racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

In the chemical structures of the compounds of the present disclosure, the bond " 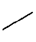 indicates an unspecified configuration; that is, if chiral isomers exist in the chemical structures, the bond " 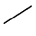 " may be " 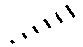 " or " 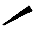 ", or includes both the configurations " 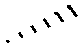 " and " 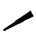 " simultaneously. In the chemical structures of the compounds of the present disclosure, the bond " 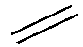 " does not specify a configuration; that is, the configuration for the bond " 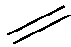 " may be an E configuration or a Z configuration, or includes both the E configuration and the Z configuration simultaneously.

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

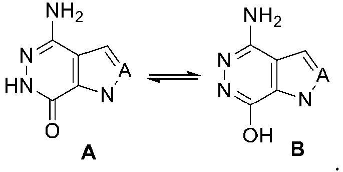

All compounds in the present disclosure can be drawn as form A or form B. All tautomeric forms fall within the scope of the present disclosure. The nomenclature of the compounds does not exclude any tautomer.

The present disclosure also includes some isotopically labeled compounds of the present disclosure that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compound of formula (I). Each available hydrogen atom linked to a carbon atom may be independently replaced by a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

### Polynucleotide and Vector

The present disclosure provides a polynucleotide and vector encoding the protein (e.g., the antibody or the antigen-binding fragment thereof) of the present disclosure. The polynucleotide of the present disclosure may be an RNA, a DNA, or a cDNA. According to some embodiments of the present disclosure, the polynucleotide of the present disclosure is a substantially isolated polynucleotide.

The polynucleotide of the present disclosure may also be in the form of, may be present in, and/or may be part of a vector. The vector is, for example, a plasmid, cosmid, YAC, or viral vector. The vector may especially be an expression vector, i.e., a vector that can provide for the *in vitro* and/or *in vivo* (i.e., in suitable host cells, host organisms, and/or expression systems) expression of the antibody or the antigen-binding fragment thereof and the antibody constant region or the fragment thereof of the present disclosure. The expression vector generally comprises at least one of the polynucleotides of the present disclosure, which is operably linked to one or more suitable expression regulatory elements (e.g., promoters, enhancers, and terminators). The selection of the elements and their sequences for expression in a particular host is within the knowledge of those skilled in the art. Regulatory elements and other elements useful or necessary for expressing the antibody or the antigen-binding fragment thereof of the present disclosure are, for example, promoters, enhancers, terminators, integration factors, selection markers, leader sequences, or reporter genes.

The polynucleotide of the present disclosure may be prepared or obtained by known means (e.g., by automatic DNA synthesis and/or recombinant DNA techniques) based on information on the amino acid sequence encoded by the polynucleotide, and/or may be isolated from a suitable natural source.

### Host Cell

The present disclosure provides a recombinant host cell that expresses or is capable of expressing one or more of the proteins (e.g., antibodies or antigen-binding fragments thereof) of the present disclosure and/or comprises the polynucleotide or vector of the present disclosure. In some embodiments, the host cell is a bacterial cell, a fungal cell, or a mammalian cell.

Bacterial cells include, e.g., cells of gram-negative bacterial strains (e.g., *Escherichia coli* strains, *Proteus* strains, and *Pseudomonas* strains), and gram-positive bacterial strains (*e.g., Bacillus* strains, *Streptomyces* strains, *Staphylococcus* strains, and *Lactococcus* strains).

Fungal cells include, e.g., cells of the species *Trichoderma, Neurospora, and Aspergillus;* or cells of the species *Saccharomyces* (e.g., *Saccharomyces cerevisiae*), *Schizosaccharomyces* (e.g., *Schizosaccharomyces pombe*), *Pichia* (e.g., *Pichia pastoris* and *Pichia methanolica*), and *Hansenula.*

Mammalian cells include, e.g., HEK293 cells, CHO cells, BHK cells, HeLa cells, COS cells, and the like.

However, amphibian cells, insect cells, plant cells, and any other cells used in the art for expressing heterologous proteins may also be used in the present disclosure.

### Composition

The present disclosure provides a composition comprising the aforementioned conjugate (e.g., the antibody-drug conjugate) of the present disclosure.

In some embodiments, a unit dose of the conjugate in the pharmaceutical composition is 0.001 mg-1000 mg.

In certain embodiments, the pharmaceutical composition comprises 0.01-99.99% of the aforementioned conjugate or the pharmaceutically acceptable salt thereof or the isotopically substituted form thereof based on the total weight of the composition. In certain embodiments, the pharmaceutical composition comprises 0.1-99.9% of the aforementioned conjugate or the pharmaceutically acceptable salt thereof or the isotopically substituted form thereof. In certain embodiments, the pharmaceutical composition comprises 0.5%-99.5% of the aforementioned conjugate or the pharmaceutically acceptable salt thereof or the isotopically substituted form thereof. In certain embodiments, the pharmaceutical composition comprises 1%-99% of the aforementioned conjugate or the pharmaceutically acceptable salt thereof or the isotopically substituted form thereof. In certain embodiments, the pharmaceutical composition comprises 2%-98% of the aforementioned conjugate or the pharmaceutically acceptable salt thereof or the isotopically substituted form thereof.

In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

In some embodiments, provided is a pharmaceutical composition comprising an amount of an anti-HER2 antibody-drug conjugate, an anti-LIV-1 antibody-drug conjugate, or an anti-TROP2 antibody-drug conjugate that is effective in treating, alleviating, or preventing a cancer, and at least one pharmaceutically acceptable excipient.

In some embodiments, provided is an article of manufacture or product (as an example, a kit) comprising the antibody-drug conjugate of the present disclosure. Optionally, the article of manufacture comprises a container and a label. Examples of containers are bottles, syringes, and test tubes. The container accommodates a composition effective in treating a disorder. The label on or associated with the container indicates that the composition is used for treating the disorder of choice.

### Treatment Method and Pharmaceutical Use

The present disclosure provides a method for treating, alleviating, preventing, or diagnosing a disease or disorder by using the conjugate of the present disclosure.

In some embodiments, provided is a method for ameliorating, alleviating, treating, or preventing a disease; for example, the method comprises administering to a subject a therapeutically effective amount of an anti-HER2 antibody-drug conjugate or a composition (including a pharmaceutical composition) comprising same for treating a cancer, e.g., breast cancer. For example, the method comprises administering to a subject a therapeutically effective amount of an anti-TROP2 antibody-drug conjugate or a composition (including a pharmaceutical composition) comprising same for treating a cancer. For example, the method comprises administering to a subject a therapeutically effective amount of an anti-LIV-1 antibody-drug conjugate or a composition (including a pharmaceutical composition) comprising same for treating a cancer.

### Definitions of Terms

In order to facilitate the understanding of the present disclosure, certain technical and scientific terms are specifically defined below. Unless otherwise specifically defined in the present disclosure, all other technical and scientific terms used in the present disclosure have the meanings generally understood by those of ordinary skill in the art to which the present disclosure pertains.

The three-letter and single-letter codes for amino acids used in the present disclosure are as described in J. biol. chem., 243, p3558 (1968).

"Engineered" refers to an operation performed on a nucleic acid or polypeptide molecule by synthesis methods, e.g., recombinant technology, *in vitro* peptide synthesis, enzymatic or chemical coupling of peptides, or other methods commonly used in the art.

"Engineered tyrosine residue", in the context of an amino acid sequence, refers to the presence of a non-wild-type amino acid residue. For example, when a wild-type sequence and an engineered form of the sequence are aligned, the "engineered tyrosine residue" is not present in the corresponding wild-type polypeptide sequence, but is introduced into the polypeptide (by mutating (e.g., substituting or replacing) the existing amino acid residue or inserting a tyrosine residue).

"Antibody" encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) as long as they exhibit the desired antigen-binding activity. An antibody may refer to an immunoglobulin, which is of a four-peptide-chain structure formed by two heavy chains and two light chains linked by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3.

The antibodies of the present disclosure may be polyclonal, monoclonal, xenogenic, allogeneic, syngeneic, or modified forms thereof, with the monoclonal antibodies being especially applicable in various examples. Generally, the antibodies of the present disclosure are recombinant antibodies. As used herein, "recombinant" generally refers to such products as a cell, a polynucleotide, a protein, or a vector, and indicates that the cell, the polynucleotide, the protein, or the vector has been modified by introduction of a heterologous polynucleotide or protein or alteration of a natural polynucleotide or protein, or that the cell is derived from a cell modified in this way. For example, recombinant cells express genes that are not found within the native (non-recombinant) cellular form or express native genes that are abnormally expressed, lowly expressed, or not expressed at all.

"Antigen-binding fragment" encompasses a single-chain antibody (i.e., full-length heavy and light chains); a Fab, a modified Fab, a Fab', a modified Fab', a F(ab')2, an Fv, a Fab-Fv, a Fab-dsFv, a single-domain antibody (e.g., VH or VL or VHH), an scFv, a bivalent or trivalent or tetravalent antibody, a Bis-scFv, a diabody, a tribody, a triabody, a tetrabody, and an epitope-binding fragment of any one of the above (see, e.g., Holliger and Hudson, 2005, Nature Biotech. 23(9):1126-1136; Adair and Lawson, 2005, Drug Design Reviews-Online 2(3), 209-217). Methods for producing and preparing such antigen-binding fragments are well known in the art (see, e.g., Verma et al., 1998, Journal of Immunological Methods, 216, 165-181).

For the determination or definition of CDRs, the deterministic depiction of CDRs and identification of residues comprising antigen-binding sites of the antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues do not significantly affect the antigen binding. As used in the present disclosure, a CDR may refer to a CDR defined by any method known in the art, including combinations of methods. The correspondence between the various numbering schemes is well known to those skilled in the art.

"Homology", "identity", or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. In general, when two sequences are aligned, a comparison is performed to obtain the maximum homology percentage.

"Polynucleotide" refers to any DNA or RNA molecule, either single- or double-stranded, and, if single-stranded, the molecule of its complementary sequence, preferably a double-stranded DNA. A polynucleotide is "operably linked" when it is placed into a functional relationship with another polynucleotide sequence. For example, a promoter or enhancer is operably linked to a coding sequence if it affects the transcription of the coding sequence.

"Host cell" includes individual cells or cell cultures, which may be or have been the recipient of a vector for incorporation of a polynucleotide insert. The host cell includes progeny of a single host cell, and the progeny may not necessarily be completely identical (in morphology or genomic DNA complement) to the original parent cell due to natural, accidental, or deliberate mutations. The host cell includes cells transfected and/or transformed *in vivo* with the polynucleotide of the present disclosure. "Cell", "cell line", and "cell culture" are used interchangeably, and all such designations include their progeny. It should also be understood that all progeny may not be precisely identical in DNA content due to intentional or unintentional mutations. The scope of the "host cell" encompasses mutant progeny that have the same function or biological activity as the obtained cell selected in the initially transformed cells.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method, route, and dose of administration, and the severity of side effects. An effective amount may be the maximum dose or administration regimen to avoid significant side effects or toxic effects. The subject of the present disclosure may be an animal or a human subject. "Optional" or "optionally" means that the event or circumstance subsequently described may, but does not necessarily, occur, and this description includes instances where the event or circumstance occurs or does not occur. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless otherwise clearly stated in the context, throughout the description and the claims, the words "comprise", "have", "include", and the like should be understood in an inclusive sense as opposed to an exclusive or exhaustive sense; that is to say, in the sense of "including, but not limited to". In the context of the mutations included in the present disclosure, "/" represents "and"; for example, "T223Y/P329Y" represents "T223Y and P329Y".

The "subject" or "patient" in the present disclosure means a mammal, particularly a primate, and especially a human.

"Pharmaceutical composition" refers to a mixture comprising one or more of the conjugates described herein and other chemical components, as well as other components such as physiologically and pharmaceutically acceptable carriers and excipients. The pharmaceutical composition is intended to promote administration to an organism and facilitate the absorption of the active ingredient so that it can exert its biological activity. "Excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by a regulatory authority as acceptable for use in humans or livestock animals.

"Linker", "linker unit", or "linker fragment" refers to a chemical structural fragment or bond, which is linked to the protein of the present disclosure at one end and linked to a drug at the other end, and also may be linked to other linkers and then linked to the drug. The linker may comprise one or more linker components. Exemplary linker components include 6-maleimidocaproyl (MC), maleimidopropionyl (MP), valine-citrulline (Val-Cit or vc), alanine-phenylalanine (ala-phe), p-aminobenzyloxycarbonyl (PAB), and those derived from coupling to a linker reagent: N-succinimidyl 4-(2-pyridylthio)pentanoate (SPP), N-succinimidyl 4-(N-maleimidomethyl)cyclohexane-1 carboxylate (SMCC, also referred to herein as MCC), and N-succinimidyl(4-iodo-acetyl)aminobenzoate (SIAB). The linker may include a stretcher unit, a spacer unit, an amino acid unit, and an extension unit. The linker may be synthesized using methods known in the art, such as those described in US2005-0238649A1. The linker may be a "cleavable linker" favorable for the release of drugs in cells. For example, the linker comprises acid-labile linkers (e.g., hydrazones), protease-sensitive (e.g., peptidase-sensitive) linkers, photolabile linkers, dimethyl linkers, or disulfide-containing linkers (Chari et al., Cancer Research 52: 127-131(1992); U.S. Patent No. 5,208,020).

"Stretcher unit" refers to a chemical structural fragment that is covalently linked to an antibody by a carbon atom at one end and linked to an amino acid unit, a disulfide moiety, or a polyethylene glycol moiety at the other end, and may also be linked to other linkers and then linked to the aforementioned moieties.

"Spacer unit" is a bifunctional compound structural fragment that can be used to couple an amino acid unit to a cytotoxic drug to ultimately form an antibody-drug conjugate, in such a way that the cytotoxic drug can be selectively linked to the amino acid unit. "Amino acid" refers to an organic compound that contains amino and carboxyl in the molecular structure and in which both amino and carboxyl are directly linked to a -CH-structure. The general formula is H₂NCHRCOOH, where R is H, substituted or unsubstituted alkyl, or the like. Amino acids are classified as α, β, γ, δ, ε...-amino acids according to the position of the carbon atom to which the amino is linked in the carboxylic acid. In the biological field, the amino acids that constitute native proteins have their specific structural characteristics; that is, their amino groups are attached directly to the α-carbon atoms, namely α-amino acids, including Gly (Glycine), Ala (Alanine), Val (Valine), Leu (Leucine), Ile (Isoleucine), Phe (Phenylalanine), Trp (Tryptophan), Tyr (Tyrosine), Asp (Aspartic acid), His (Histidine), Asn (Asparagine), Glu (Glutamic acid), Lys (Lysine), Gln (Glutamine), Met (Methionine), Arg (Arginine), Ser (Serine), Thr (Threonine), Cys (Cysteine), Pro (Proline), etc. Non-natural amino acids are, for example, citrulline. As is well known to those skilled in the art, non-natural amino acids do not constitute natural proteins and are therefore not involved in the synthesis of antibodies in the present disclosure. The three-letter and single-letter codes for amino acids used in the present disclosure are as described in J. Biol. Chem, 243, p3558 (1968).

"Antibody-drug conjugate" means that a ligand is linked to a biologically active drug by a linker unit. In the present disclosure, "antibody-drug conjugate" (ADC) means that a monoclonal antibody or an antibody fragment is linked to a biologically active toxic drug, such as exatecan or a derivative thereof, by a linker unit.

"Therapeutic agent" means a macromolecule or small molecule that can be administered to a subject in need thereof to treat a disorder. The therapeutic agent may be administered to treat or prevent the onset, slow the progression, or ameliorate one or more symptoms of a medical disorder in a subject suffering from the disorder.

"Diagnostic agent" refers to a compound that can be used for *in vivo* imaging studies such as CT, MRI, and X-ray and/or *in vitro* imaging studies. Non-limiting examples of diagnostic agents include fluorophores, fluorescent dyes, radionuclides, and enzymes. "Drug loading" may be represented as the ratio of the number of drug molecules to the number of antibody molecules. The drug loading may range from 1 to 20, preferably from 1 to 10 cytotoxic drug molecules (D) linked per antibody molecule (Pc). In an embodiment of the present disclosure, the drug loading is represented as DAR or y. Low drug loading can reduce efficacy, while high drug loading can negatively affect the pharmacokinetic profile and toxicity. Exemplary drug loading may be an average of 1, 2, 3, 4, 5, 6, 7, 8, 9, 10, 11, 12, 13, 14, 15, 16, 17, 18, 19, 20, or a numerical value between any two of these numerical values, preferably an average of 1-10, and more preferably an average of 1-8, or 2-8, or 2-7, or 3-8, or 3-7, or 3-6, or 4-7, or 4-6, or 4-5. The average number of drug molecules per ADC molecule after the conjugation reaction can be characterized by conventional methods such as UV/visible spectroscopy, mass spectrometry, an ELISA assay, a monoclonal antibody molecular size variant assay (CE-SDS), and HPLC.

The monoclonal antibody molecular size variant assay (CE-SDS) of the present disclosure may be used for quantitatively determining the purity of a recombinant monoclonal antibody product by adopting capillary electrophoresis-sodium dodecyl sulfate (CE-SDS) ultraviolet assay based on the molecular weight under reduced and nonreduced conditions and according to a capillary electrophoresis method (Chinese Pharmacopoeia).

The loading of the antibody-drug conjugate can be controlled by the following non-limiting methods, including:
(1) controlling a molar ratio of a linking reagent to a monoclonal antibody,
(2) controlling reaction time and temperature, and
(3) selecting different reaction reagents.

"Alkyl" refers to a saturated aliphatic hydrocarbon group, which is a linear or branched chain group containing 1 to 12 carbon atoms, preferably an alkyl group containing 1 to 10 carbon atoms, and most preferably an alkyl group containing 1 to 6 carbon atoms. Non-limiting examples include methyl, ethyl, n-propyl, isopropyl, n-butyl, isobutyl, *tert-*butyl, sec-butyl, n-pentyl, 1,1-dimethylpropyl, 1,2-dimethylpropyl, 2,2-dimethylpropyl, 1-ethylpropyl, 2-methylbutyl, and 3-methylbutyl. Alkyl may be substituted or unsubstituted, and when it is substituted, the substituent may be substituted at any available point of attachment, and the substituent is preferably one or more of the following groups independently selected from the group consisting of halogen, C₁₋₃ alkyl, and C₁₋₃ alkoxy.

"Alkenyl" refers to an unsaturated aliphatic linear or branched hydrocarbon group containing one or more carbon-carbon double bonds. Exemplary alkenyl includes C2-C8, C2-C7, C2-C6, C2-C4, C3-C12, and C3-C6 alkenyl, including but not limited to, vinyl, 1-propenyl, 2-propenyl (i.e., allyl), 2-methyl-1-propenyl, 1-butenyl, 2-butenyl (i.e., crotyl), and the like. Alkenyl used in any context herein is optionally substituted in the same manner as alkyl.

"Alkynyl" refers to an unsaturated aliphatic linear or branched hydrocarbon group containing one or more carbon-carbon triple bonds. Exemplary alkynyl includes C2-C8, C2-C7, C2-C6, C2-C4, C3-C12, and C3-C6 alkynyl, including but not limited to, ethynyl, prop-1-ynyl, but-1-ynyl, pent-1-ynyl, pent-4-ynyl, and pent-1,4-diynyl. Alkynyl used in any context herein is optionally substituted in the same manner as alkyl.

The term "cycloalkyl" refers to a saturated monocyclic or polycyclic hydrocarbon substituent; the cycloalkyl ring contains 3 to 20 carbon atoms, preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, etc. Polycyclic cycloalkyl includes spiro-ring, fused-ring, and bridged-ring cycloalkyl. Cycloalkyl may be substituted or unsubstituted, and when it is substituted, the substituent may be substituted at any available point of attachment, and the substituent is preferably one or more of the following groups independently selected from the group consisting of halogen, C₁₋₃ alkyl, and C₁₋₃ alkoxy.

"Cycloalkenyl" refers to an unsaturated monocyclic or polycyclic hydrocarbon substituent containing one or more carbon-carbon double bonds. Cycloalkenyl contains 3 to 20 carbon atoms, including C2-C8, C4-C6, C8-C12, C14-C18, and C16-C20 cycloalkenyl. In addition, cycloalkenyl may be fused to aryl or heteroaryl. Exemplary cycloalkenyl includes, but is not limited to:

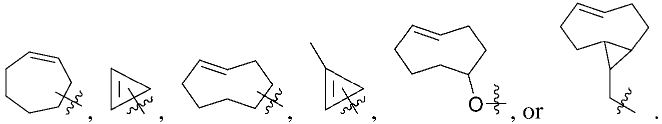

In addition, cycloalkenyl may be substituted or unsubstituted, and when it is substituted, the substituent may be substituted at any available point of attachment, and the substituent is preferably one or more of the following groups independently selected from the group consisting of halogen, C₁₋₃ alkyl, and C₁₋₃ alkoxy.

"Cycloalkynyl" refers to an unsaturated monocyclic or polycyclic hydrocarbon substituent containing one or more carbon-carbon triple bonds. Cycloalkynyl contains 3 to 20 carbon atoms, including C2-C8, C4-C6, C8-C12, C14-C18, and C16-C20 cycloalkynyl. In addition, cycloalkynyl may be fused to aryl or heteroaryl. Exemplary cycloalkynyl includes, but is not limited to:

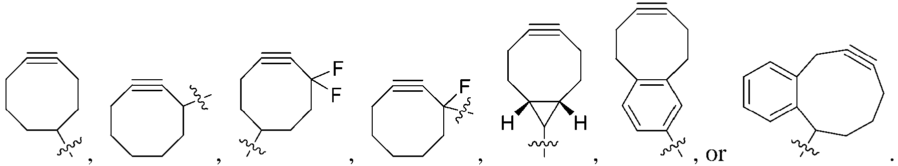

In addition, cycloalkynyl may be substituted or unsubstituted, and when it is substituted, the substituent may be substituted at any available point of attachment, and the substituent is preferably one or more of the following groups independently selected from the group consisting of halogen, C₁₋₃ alkyl, and C₁₋₃ alkoxy.

"Heterocycloalkyl" refers to a saturated monocyclic or polycyclic hydrocarbon substituent containing 3 to 20 ring atoms, wherein one or more ring atoms are heteroatoms selected from the group consisting of nitrogen, oxygen, and S(O)ₘ (where m is an integer of 0 to 2), but excluding a ring moiety of -O-O-, -O-S-, or -S-S-, and the other ring atoms are carbon. Preferably, heterocycloalkyl contains 3 to 12 ring atoms, 1 to 4 of which are heteroatoms; more preferably, heterocycloalkyl contains 3 to 7 ring atoms. In addition, heterocycloalkyl may be fused to aryl or heteroaryl. Non-limiting examples include pyrrolidinyl, piperidinyl, piperazinyl, morpholinyl, thiomorpholinyl, and the like.

"Heterocycloalkenyl" refers to an unsaturated monocyclic or polycyclic hydrocarbon substituent containing 3 to 20 ring atoms, wherein one or more ring atoms are heteroatoms selected from the group consisting of nitrogen, oxygen, and S(O)ₘ (where m is an integer of 0 to 2), but excluding a ring moiety of -O-O-, -O-S-, or -S-S-, and the other ring atoms are carbon and contain one or more carbon-carbon double bonds. Heterocycloalkenyl contains 3 to 20 carbon atoms, preferably including C2-C8, C4-C6, C8-C12, C14-C18, and C16-C20 heterocycloalkenyl. In addition, heterocycloalkenyl may be fused to aryl or heteroaryl. Exemplary heterocyclyl includes, but is not limited to: 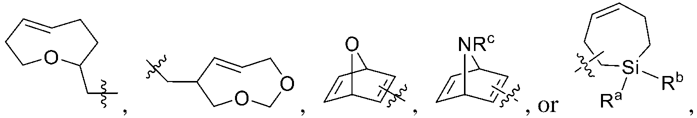 wherein R^{a} and R^{b} are each independently selected from the group consisting of hydrogen, C₁₋₆ alkyl, and aryl; R^{c} is selected from the group consisting of hydrogen and C₁₋₆ alkyl. In addition, heterocycloalkenyl may be substituted or unsubstituted, and when it is substituted, the substituent may be substituted at any available point of attachment, and the substituent is preferably one or more of the following groups independently selected from the group consisting of halogen, C₁₋₃ alkyl, and C₁₋₃ alkoxy.

"Heterocycloalkynyl" refers to an unsaturated monocyclic or polycyclic hydrocarbon substituent containing 3 to 20 ring atoms, wherein one or more ring atoms are heteroatoms selected from the group consisting of nitrogen, oxygen, and S(O)ₘ (where m is an integer of 0 to 2), but excluding a ring moiety of -O-O-, -O-S-, or -S-S-, and the other ring atoms are carbon and contain one or more carbon-carbon triple bonds. Heterocycloalkynyl contains 3 to 20 carbon atoms, including C2-C8, C4-C6, C8-C12, C14-C18, and C16-C20 heterocycloalkynyl. In addition, heterocycloalkynyl may be fused to aryl or heteroaryl. Exemplary heterocycloalkynyl includes, but is not limited to: 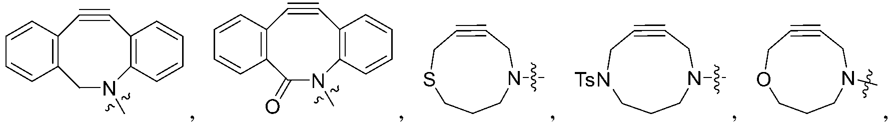 or

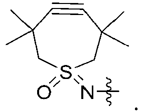

In addition, heterocycloalkynyl may be substituted or unsubstituted, and when it is substituted, the substituent may be substituted at any available point of attachment, and the substituent is preferably one or more of the following groups independently selected from the group consisting of halogen, C₁₋₃ alkyl, and C₁₋₃ alkoxy.

"Alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted, and when it is substituted, the substituent is preferably one or more of the following groups independently selected from the group consisting of halogen, C₁₋₃ alkyl, and C₁₋₃ alkoxy.

"Aryl" refers to a 6- to 14-membered, preferably 6- to 12-membered, all-carbon monocyclic or fused polycyclic (i.e., rings that share a pair of adjacent carbon atoms) group having a conjugated *π*-electron system, such as phenyl and naphthyl. The aryl ring may be fused to a heteroaryl, heterocycloalkyl, or cycloalkyl ring, wherein the ring attached to the parent structure is the aryl ring; its non-limiting examples include:

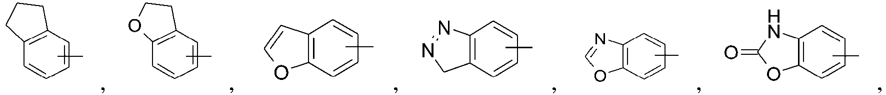

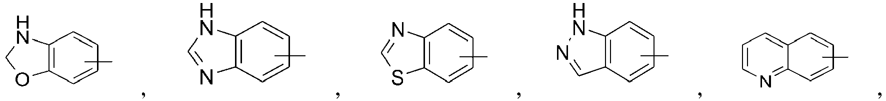

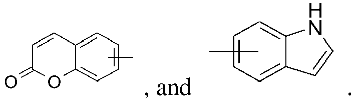

Aryl may be substituted or unsubstituted, and when it is substituted, the substituent is preferably one or more of the following groups independently selected from the group consisting of halogen, C₁₋₃ alkyl, and C₁₋₃ alkoxy.

"Heteroaryl" refers to a heteroaromatic system containing 1 to 4 heteroatoms and 5 to 14 ring atoms, wherein the heteroatoms are selected from the group consisting of oxygen, sulfur, and nitrogen. Preferably, heteroaryl is 6- to 12-membered. More preferably, heteroaryl is 5- or 6-membered. For example, its non-limiting examples include: imidazolyl, furyl, thienyl, pyridyl, pyrimidinyl, pyrazinyl, triazolyl, indazolyl, benzimidazolyl, 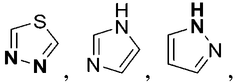 etc.

"Spiro" refers to compounds in which two rings share one atom. Non-limiting examples of spirocycloalkyl include:

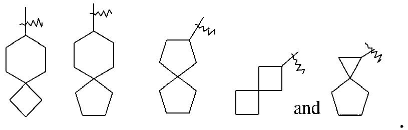

"Fused" refers to compounds formed by fusing two or more rings by sharing two adjacent atoms. Non-limiting examples of fused cycloalkyl include:

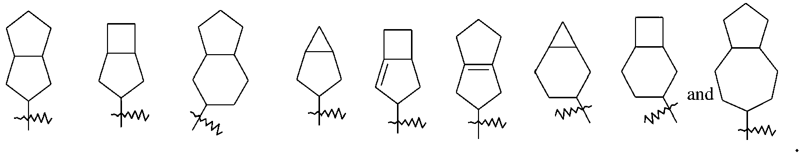

"Bridged" refers to a structure formed by two or more cyclic structures sharing two non-adjacent ring atoms. According to the number of constituent rings, it may be bicyclic, tricyclic, tetracyclic, or polycyclic bridged cycloalkyl, preferably bicyclic, tricyclic, or tetracyclic, and more preferably bicyclic or tricyclic. Non-limiting examples of bridged cycloalkyl include:

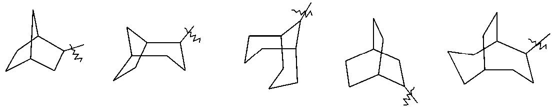

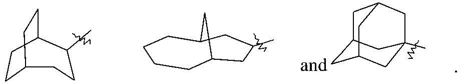

"Hydroxy" refers to an -OH group.

"Halogen" refers to fluorine, chlorine, bromine, or iodine.

"Cyano" refers to -CN.

"Amino" refers to -NH₂.

"Nitro" refers to -NO₂.

"Oxo" refers to an =O substituent.

"Substituted" means that one or more, preferably up to 5, more preferably 1 to 3 hydrogen atoms in the group are independently substituted with a corresponding number of substituents. When the substituent is ketone or oxo (i.e., =O), two (2) hydrogens on the atom are replaced.

Technical solution 1. An antibody-drug conjugate, comprising a structure represented by formula I or a pharmaceutically acceptable salt thereof:

Pc-[L-(D)ₓ]_{y} I,

wherein Pc is an antibody or an antigen-binding fragment thereof;
L is a linker covalently linking Pc to D, and L is linked to an amino acid in Pc; the amino acid is located at any one or any combination of positions 135, 137, 192, 223, 298, and 329 of a heavy chain HC and position 202 of a light chain LC of the antibody Pc or the antigen-binding fragment thereof, or the amino acid is not located at a N-glycosylation site or within 10 amino acids near the N-glycosylation site;
x is 1 to 20;
y is 1 to 20;
-D is a payload;
preferably, the N-glycosylation site is 297N.

Technical solution 2. The antibody-drug conjugate according to technical solution 1, wherein L and Pc form a structure represented by Za or Zb: 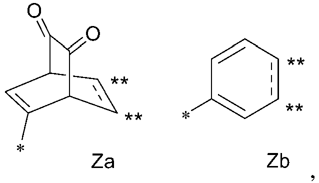 wherein * represents an end linked to Pc, ** represents an end linked to a linker, and ---- represents a single bond or a double bond.

Technical solution 3. The antibody-drug conjugate according to technical solution 1 or 2, wherein an amino acid linked to L is tyrosine and is oxidized by tyrosinase to o-quinone. Technical solution 4. The antibody-drug conjugate according to technical solution 3, wherein L-(D)ₓ is a chemical moiety represented by the following formulas: 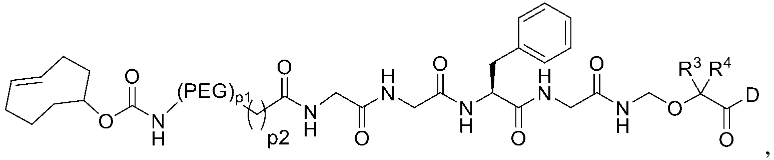 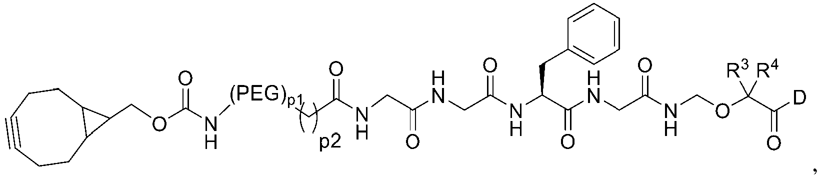 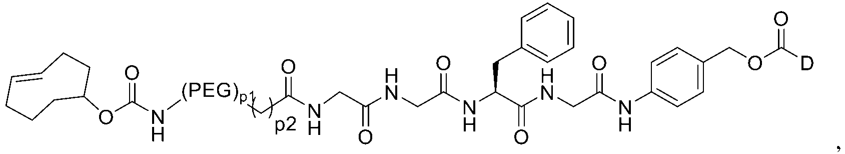 or 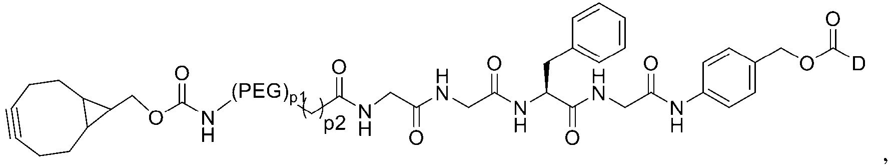 wherein R³ is selected from the group consisting of hydrogen, C₃₋₆ cycloalkylalkyl, and C₃₋₆ cycloalkyl, preferably hydrogen; R⁴ is selected from the group consisting of hydrogen, haloalkyl, and C₃₋₆ cycloalkyl, preferably hydrogen; or R³ and R⁴, together with the carbon atom to which they are attached, form C₃₋₆ cycloalkyl; p1 is selected from the group consisting of 2, 4, 6, and 8, and p2 is selected from the group consisting of 0, 1, and 2.

Technical solution 5. The antibody-conjugate according to technical solution 3 or 4, wherein the antibody-conjugate is represented by the following formula: 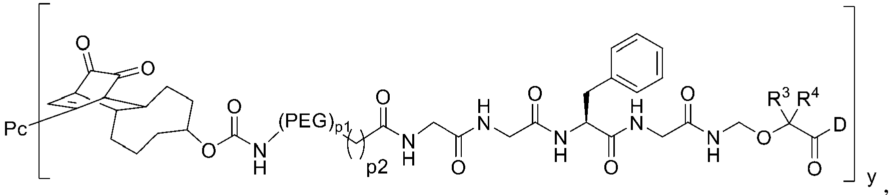 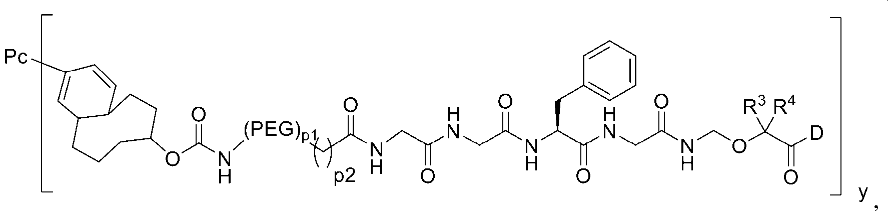 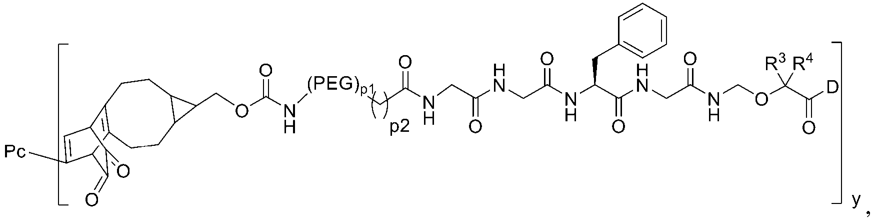 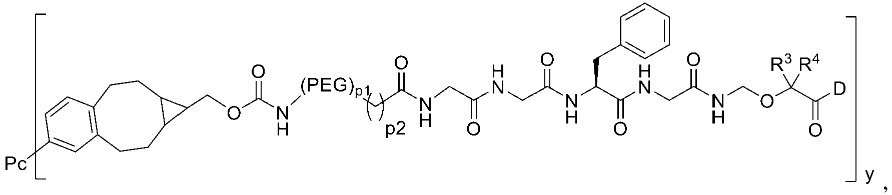 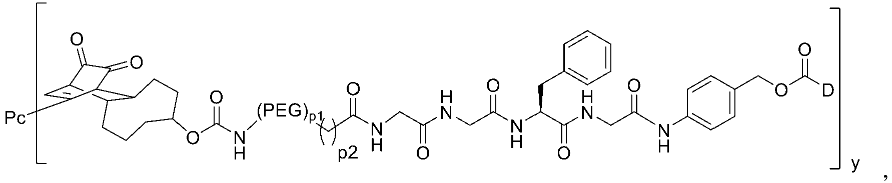 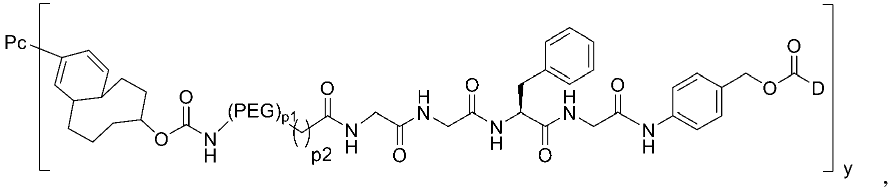 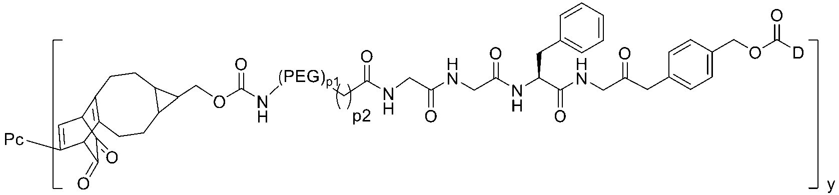 or 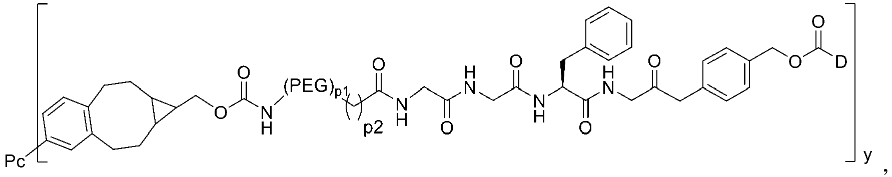 wherein R³ is selected from the group consisting of hydrogen, C₃₋₆ cycloalkylalkyl, and C₃₋₆ cycloalkyl, preferably hydrogen; R⁴ is selected from the group consisting of hydrogen, haloalkyl, and C₃₋₆ cycloalkyl, preferably hydrogen; or R³ and R⁴, together with the carbon atom to which they are attached, form C₃₋₆ cycloalkyl; y is selected from the group consisting of 1 to 20 and may be an integer or a decimal; p1 is selected from the group consisting of 2, 4, 6, and 8, and p2 is selected from the group consisting of 0, 1, and 2; Pc is an antibody or an antigen-binding fragment thereof.

Technical solution 6. A method for preparing an antibody-drug conjugate, comprising:
a) providing an antibody or an antigen-binding fragment thereof comprising an exposed tyrosine residue, wherein the exposed tyrosine residue is located at any one or any combination of positions 135, 137, 192, 223, 298, and 329 of a heavy chain HC and position 202 of a light chain LC of the antibody Pc or the antigen-binding fragment thereof, or not located at a N-glycosylation site or within 10 amino acids near the N-glycosylation site,
b) contacting with tyrosinase to convert the phenol moiety of the exposed tyrosine residue to an o-quinone moiety, and
c) subjecting the o-quinone moiety to a [4+2] cycloaddition reaction with an alkene or alkyne compound, wherein the alkene or alkyne compound comprises a cycloalkenyl, heterocycloalkenyl, cycloalkynyl, or heterocycloalkynyl moiety.

The method according to technical solution 6, wherein the tyrosinase is derived from *Bacillus megaterium* or *Verrucomicrobium spinosum.*

Technical solution 7. The method according to technical solution 6, wherein the alkene or alkyne compound comprises a cycloalkenyl, heterocycloalkenyl, cycloalkynyl, or heterocycloalkynyl moiety, and optionally comprises a payload D.

Technical solution 8. The method according to any one of technical solution 7, wherein the o-quinone moiety in step b) undergoes a [4+2] cycloaddition reaction with the alkene or alkyne compound to form a structure represented by Za or Zb, 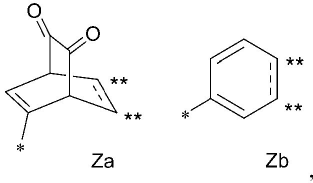 wherein * represents an end linked to Pc, ** represents an end linked to a linker, and ---- represents a single bond or a double bond.

### BRIEF DESCRIPTION OF THE DRAWINGS

FIGs. 1A to 1C show SDS-PAGE results of recombinantly expressed and purified tyrosinases, wherein FIG. 1A shows SDS-PAGE results of megaTYR; FIG. 1B shows SDS-PAGE results of VsTYR core; and FIG. 1C shows SDS-PAGE results of VsTYR sp.
FIG. 2 shows a schematic diagram of a one-pot conjugation reaction mediated by catalysis by tyrosinase.
FIGs. 3A and 3B show deconvolved mass spectrometry results for tyrosinase-mediated conjugates of single-point mutated antibodies and small molecules. FIG. 3A shows deconvolved mass spectrometry results for the VsTYR core-mediated conjugate of 0102 H30 and L-2, and FIG. 3B shows deconvolved mass spectrometry results for the VsTYR core-mediated conjugate of 0102 L15 and L-2.
FIGs. 4A to 4C show deconvolved mass spectrometry results for tyrosinase-mediated conjugates of multi-site mutated antibodies and a small molecule, wherein FIG. 4A shows deconvolved mass spectrometry results for the VsTYR core-mediated conjugate of Her HH3 and L-2; FIG. 4B shows deconvolved mass spectrometry results for the VsTYR core-mediated conjugate of Her HH8 and L-2; and FIG. 4C shows deconvolved mass spectrometry results for the VsTYR core-mediated conjugate of Her HH9 and L-2.
FIG. 5 shows the result of the purification of an antibody-peptide conjugate by HiLoad 16/600 Superdex 200 pg.
FIG. 6 shows the ELISA results of the binding of CBP-ADA-mut, ADA mut, and adalimumab to human TNFα.
FIG. 7 shows the results of the binding of CBP-ADA-mut and ADA mut to chicken type II collagen.
FIG. 8 shows *in vitro* cell killing results for ADCs.
FIG. 9 shows a sequence alignment of IGHG1, IGHG2, IGHG3, and IGHG4.

### DETAILED DESCRIPTION

The present disclosure is further described below with reference to examples, but these examples are not intended to limit the scope of the present disclosure. Experimental procedures without specific conditions specified in the examples of the present disclosure were generally conducted under conventional conditions such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

Unless otherwise specified, the CDRs of the antibodies in the examples of the present disclosure were defined using the Kabat numbering scheme.

The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument, with dimethyl sulfoxide-D6 (DMSO-*d₆*), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters, MS model: waters ACQuity Qda Detector/waters SQ Detector), and THERMO Ultimate 3000-Q Exactive (manufacturer: THERMO, MS model: THERMO Q Exactive).

The high performance liquid chromatography (HPLC) analyses were performed using an Agilent HPLC 1200DAD, Agilent HPLC 1200VWD, or Waters HPLC e2695-2489 highpressure liquid chromatograph.

The chiral HPLC analyses were performed using an Agilent 1260 DAD high performance liquid chromatograph.

The preparative high performance liquid chromatography used Waters 2545-2767, Waters 2767-SQ Detecor2, Shimadzu LC-20AP, and Gilson GX-281 preparative chromatographs. The preparative chiral chromatography used a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm to 0.2 mm, and those used in the thin-layer chromatography separation and purification had a layer thickness of 0.4 mm to 0.5 mm.

The silica gel column chromatography generally used Yantai Huanghai 200-300 mesh silica gel as the carrier.

Known starting materials in the present disclosure may be synthesized by using or following methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed in an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C to 30 °C, unless otherwise specified.

The monitoring of the reaction progress in the examples used thin-layer chromatography (TLC). The developing solvent used in the reactions, the eluent systems used in the column chromatography purification, the developing solvent systems used in the thin-layer chromatography, and the volume ratio of the solvents were adjusted depending on the polarity of the compound, or a small amount of basic or acidic reagents such as triethylamine and acetic acid could be added for adjustment.

### Example 1. Design of Mutation of Solvent Exposure Sites in IgG Antibodies into Tyrosine

With trastuzumab Fab (PDB: 6MH2) and trastuzumab Fc (PDB: 3D6G) as templates, the solvent accessible surface area (SASA (Å²)) of each amino acid in the constant regions of the antibodies and the solvent accessible surface area of the mutant tyrosine after substitution with tyrosine were calculated using bioinformatic methods, and the possibility of site-specific modification of potential mutable sites was evaluated based on secondary structure information about the mutation sites. The heavy chain HC sequence of trastuzumab is detailed in SEQ ID NO: 1, and its light chain LC sequence is detailed in SEQ ID NO: 2.

Jorick J. Bruins et al. used mushroom-derived tyrosinase to perform site-specific modification on Y296 and Y300 near N297 (Bruins JJ, et al. Bioconjug Chem. 2021 Oct 20;32(10):2167-2172). According to the SASA calculations, Tyr296 was the most exposed of all Tyrs, with a numerical value of 248, and the numerical value for Tyr300 was only 41. Therefore, the SASA screening value was set to 40. Meanwhile, based on the spatial conformations of Tyr296 and Tyr300, sites with relatively large structural flexibility in or near the loop were selected as candidate sites.

The selected sites of the IGHG1 antibody are shown in Table 1. For other types of IGHG, PDB: 4L4J, PDB: 2QSC, PDB: 5W5N, and PDB: 3EO1 were used as templates, and similar calculation methods and screening were adopted. Selectable sites for the light chain are shown in Table 2. In addition, the hinge region linking the Fab and the Fc has relatively large flexibility and is therefore a potential site for site-specific modification. The position numbers in the present disclosure are all defined based on the EU numbering scheme.

**Table 1. The selected sites of the IGHG1 antibody**

| Position number | Original amino acid | SASA_mut(Å²) | SASA_ori(Å²) |
|---|---|---|---|
| 118 | Ala | 64.99 | 43.69 |
| 119 | Ser | 181.04 | 92.84 |
| 121 | Lys | 134.76 | 117.24 |
| 135 | Thr | 123.65 | 64.3 |
| 137 | Gly | 174.27 | 76.21 |
| 138 | Gly | 194.5 | 142.23 |
| 162 | Ala | 111.63 | 92.27 |
| 166 | Gly | 203.21 | 148.98 |
| 191 | Ser | 125.41 | 110.74 |
| 192 | Ser | 180.76 | 124.51 |
| 195 | Thr | 157.08 | 103.58 |
| 197 | Thr | 110.62 | 111.05 |
| 207 | Ser | 167.7 | 124.92 |
| 208 | Asn | 196.41 | 146.32 |
| 214 | Lys | 154.5 | 135.05 |
| 254 | Ser | 225.59 | 106.15 |
| 256 | Thr | 208.99 | 85.76 |
| 267 | Ser | 13.05 | 38.04 |
| 268 | His | 242.46 | 104.93 |
| 270 | Asp | 112.22 | 37.14 |
| 271 | Pro | 144.76 | 33.30 |
| 280 | Asp | 191.10 | 79.17 |
| 281 | Gly | 214.03 | 55.44 |
| 282 | Val | 247.89 | 110.13 |
| 283 | Glu | 251.06 | 64.13 |
| 290 | Lys | 86.18 | 100.43 |
| 298 | Ser | 205.28 | 75.84 |
| 318 | Glu | 72.83 | 69.82 |
| 326 | Lys | 152.90 | 178.59 |
| 327 | Ala | 65.79 | 44.27 |
| 329 | Pro | 226.57 | 151.38 |
| 330 | Ala | 158.90 | 63.91 |
| 337 | Ser | 50.85 | 29.36 |
| 342 | Gln | 202.39 | 167.20 |
| 355 | Arg | 293.65 | 229.71 |
| 384 | Asn | 131.23 | 124.91 |
| 385 | Gly | 290.26 | 53.45 |
| 389 | Asn | 177.41 | 132.42 |
| 402 | Gly | 87.10 | 38.42 |
| 413 | Asp | 158.62 | 68.77 |
| 434 | Asn | 230.74 | 128.97 |
| 442 | Ser | 123.73 | 34.91 |
| 444 | Ser | 64.94 | 93.18 |

| | | | |
|---|---|---|---|
| Note: SASA_ori (Å²) is the original SASA (Å²), and SASA_mut (Å²) is the SASA (Å²) after mutation; calculations cannot be performed for the hinge region. | | | |

**Table 2. The selected sites of the light chains of antibodies**

| Format | Position number | Original amino acid | SASA_mut(Å²) | SASA_ori(Å²) |
|---|---|---|---|---|
| | 109 | Thr | 160.23 | 119.07 |
| | 110 | Val | 90.5 | 82.05 |
| Kappa LC | 143 | Glu | 144.57 | 139.47 |
| | 152 | Asn | 142.92 | 120.28 |
| | 153 | Ala | 106.43 | 54.18 |
| | 157 | Gly | 220.86 | 91.29 |
| | 169 | Lys | 225.97 | 223.45 |
| | 190 | Lys | 115.18 | 156.36 |
| | 191 | Val | 31.34 | 57.01 |
| | 197 | Thr | 57.44 | 62.19 |
| | 202 | Ser | 196.93 | 133.18 |
| | 210 | Asn | 127.08 | 73.98 |
| | 213 | Glu | 150.88 | 135.83 |

### Example 2. Expression and Identification of Wild-Type Antibodies (wt) and Single-Point Mutated Antibodies

The mutation sites in Example 1 were specifically mutated on the sequences of trastuzumab and 0102. The mutations were generated using QuikChange XL of Stratagene according to the manufacturer's scheme. Corresponding plasmid DNA was constructed on the pTT5 vector, and the required mutations were confirmed by DNA sequencing. The antibodies were expressed using ExpiCHO-S cells and purified. Specifically, ExpiCHO-S cells (Thermo, A29133) in the logarithmic growth phase and good conditions were diluted to 6 × 10⁶ cells/mL with culture medium, and 50 mL of the cell suspension was inoculated. 40 µL of plasmids was diluted with 2 mL of culture medium, with LC:HC being 3:2, and after thorough mixing, solution 1 was obtained. 160 µL of transfection reagent was diluted with 1.84 mL of culture medium, and after thorough mixing, solution 2 was obtained. Solution 2 was added to solution 1, and after 1-5 min of gentle mixing at room temperature, a mixed transfection solution was obtained. The mixed transfection solution was then added dropwise to the cell culture with shaking to ensure thorough mixing. The shake flask was placed on a shaker at 37 °C with 8% CO₂. After 18-22 h of culture, 8 mL of Feed (Thermo, A29133) and 0.3 mL of Enhancer (Thermo, A29133) were added, and the culture conditions were adjusted to 5% CO₂ and 32 °C. The supernatant of the fermentation broth was centrifugally collected on days 12-14 and purified by one-step affinity chromatography (MabSelectSuRe column, GE, 175438). The resulting antibody was purified and buffer-exchanged into a phosphate buffer (pH 5.5).

The expressed antibodies were identified using LC-MS. Specifically, the purified antibody was diluted to 1 mg/mL, and formic acid (final concentration: 0.1%) was added to enhance the protonation of the sample. The sample was analyzed using a ThermoFisher QE plus mass spectrometer. The chromatography column was equilibrated with 90% mobile phase A (0.1% formic acid/water solution)-10% mobile phase B (0.1% formic acid/acetonitrile) before sample injection, and gradient elution was then performed. After the sampling was completed, mass spectrometry data of the target peak were calculated using BioPharma Finder software.

Related information about some of the expressed antibodies is shown in Table 3. The expressed molecular weights were all consistent with the calculated values.

**Table 3. The expression and identification of wild-type antibodies (wt) and antibodies with single tyrosine point mutations and their combinations**

| Antibody No. | Mutation(s) | Heavy chain HC | Light chain LC | Calculated molecular weight Da | Found molecular weight Da |
|---|---|---|---|---|---|
| | | | | (+2*G0F) | (+2*G0F) |

| Wild type | | | | | |
|---|---|---|---|---|---|
| Her H1 | / | SEQ ID NO:1 | SEQ ID NO:2 | 148055.89 | 148056.34 |
| 0102 | / | SEQ ID NO:12 | SEQ ID NO: 13 | 148288.10 | 148287.41 |

| With mutation(s) in IGHG1 | | | | | |
|---|---|---|---|---|---|
| Her H9 | T135Y | SEQ ID NO:3 | SEQ ID NO:2 | 148180.03 | 148179.06 |
| Her H10 | G137Y | SEQ ID NO:4 | | 148268.14 | 148266.54 |
| Her H14 | T197Y | SEQ ID NO:5 | | 148180.03 | 148177.39 |
| Her H19 | T223Y | SEQ ID NO:6 | | 148180.03 | 148178.28 |
| Her H30 | P329Y | SEQ ID NO:7 | | 148188.01 | 148185.77 |
| Her H33 | Q342Y | SEQ ID NO:8 | | 148125.98 | 148125.08 |
| Her H53 | S192Y | SEQ ID NO:9 | | 148208.08 | 148206.64 |
| Her H26* | S298Y/Y296F/ Y300F/N297G | SEQ ID NO:10 | | 145139.98 | 145137.69 |
| 0102 H9 | T135Y | SEQ ID NO:14 | SEQ ID NO: 13 | 148412.24 | 148412.00 |
| 0102 H10 | G137Y | SEQ ID NO:15 | | 148550.34 | 148498.27 |
| 0102 H19 | T223Y | SEQ ID NO:16 | | 148412.24 | 148411.63 |
| 0102 H30 | P329Y | SEQ ID NO: 17 | | 148420.22 | 148418.89 |
| 0102 H53 | S192Y | SEQ ID NO:18 | | 148440.29 | 148439.11 |
| 0102 H26* | S298Y/Y296F/ Y300F/N297G | SEQ ID NO:19 | | 145372.19 | 145370.22 |

| With a mutation in Cκ | | | | | |
|---|---|---|---|---|---|
| Her L15 | S202Y | SEQ ID NO:1 | SEQ ID NO:11 | 148208.08 | 148206.91 |
| 0102 L15 | S202Y | SEQ ID NO:12 | SEQ ID NO:20 | 148440.29 | 148438.36 |

| | | | | | |
|---|---|---|---|---|---|
| * The antibodies contain mutation N297G. Since the antibodies can be glycosylated at 297N, glycosylation will no longer occur after position 297 is mutated into G. The corresponding molecular weights are all non-glycosylated molecular weights. | | | | | |

### Example 3. Synthesis of Compound L-1

### Step 1: Synthesis of compound 1b

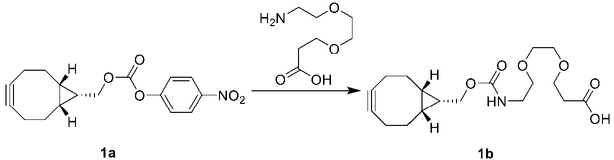

Compound **1a** (prepared with reference to the method in J. Am. Chem. Soc. 2020, 142, 9285-9301) (100 mg, 0.317 mmol) was dissolved in DMF (5 mL), and Et₃N (96 mg, 0.951 mmol) and 3-[2-(2-aminoethoxy)ethoxy]-propionic acid (56 mg, 0.317 mmol) were added with stirring at room temperature. The reaction was substantially completed at room temperature. The reaction mixture was concentrated to dryness under reduced pressure, and the crude product was purified by silica gel column chromatography (DCM/MeOH/HOAc = 15:1:0.01) to give compound **1b** (63 mg, 56% yield).

Ms (ESI): m/z 376.1 [M+Na]⁺.

### Step 2: Compound 1e

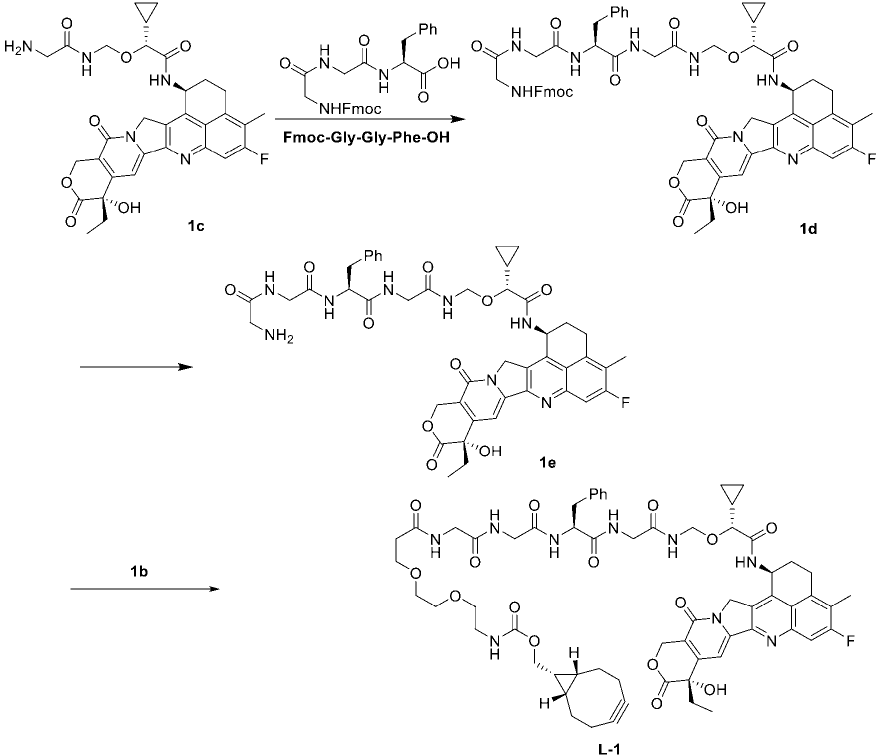

Compound **1c** (prepared with reference to the method in WO2022/161385; 73.6 mg, 0.119 mmol) and **Fmoc-Gly-Gly-Phe-OH** (65.6 mg, 0.131 mmol) were added to a 50 mL reaction flask, and THF (2 mL) and MeOH (3 mL) were added. The mixture was cooled in an ice-water bath. DMTMM (4-(4,6-dimethoxytriazin-2-yl)-4-methylmorpholine hydrochloride, 49.3 mg, 0.178 mmol) was added, and the ice-water bath was removed. The reaction was substantially completed at room temperature. The reaction mixture was concentrated to dryness under reduced pressure to give a crude product. The crude product was purified by silica gel column chromatography (DCM/MeOH = 15:1) to give compound **1d** (110 mg).

Ms (ESI): m/z 1103.4 [M+H]⁺.

Compound **1d** (150 mg, 0.136 mmol) was dissolved in anhydrous THF (3 mL). The solution was cooled in an ice-salt bath in an Ar atmosphere. With the internal temperature controlled at -10 °C, DBU (62 mg, 0.41 mmol) was added. With the internal temperature controlled at -10 °C, the mixture was stirred for 2 h. tert-Butyl dimethyl ether (MTBE, 10 mL) was added, and the mixture was filtered, followed by washing with MTBE (10 mL). After drying, crude compound **1e** (156 mg) was obtained. The crude product was directly used in the next step.

Ms (ESI): m/z 881.3 [M+H]⁺.

### Step 3: Synthesis of compound L-1

Compound **1b** (60 mg, 0.17 mmol) and compound **1e** (60% HPLC purity, 156 mg) were dissolved in a mixed solvent of DCM/MeOH (6 mL/2 mL), and the temperature was controlled at -30 °C. DMTMM (75 mg, 0.254 mmol, 1.5 eq) was then added, and the mixture was stirred for 2 h with the internal temperature controlled at -17 °C. Water (15 mL) was added to quench the reaction, and extraction was performed with DCM (8 mL × 2). The organic phases were combined, washed once with a saturated NaCl solution (20 mL), dried over Na₂SO₄, filtered, and concentrated to dryness under reduced pressure to give a crude product. The crude product was purified by preparative liquid chromatography (acetonitrile/water, 20 mM NH₄HCO₃) to give compound **L-1** (41.9 mg, HPLC: 93.0%).

Ms (ESI): m/z 1216.4 [M+H]⁺.

¹H NMR (400 MHz, CDCl₃): δ 8.40-8.10 (m, 1H), 8.00-7.85 (m, 1H), 7.85-7.30 (m, 6H), 7.25-7.05 (m, 5H), 5.70-5.50 (m, 2H), 5.45-5.10 (m, 2H), 5.05-4.80 (m, 2H), 4.75-4.60 (m, 1H), 4.55-4.25 (m, 2H), 4.20-4.05 (m, 2H), 3.95-3.40 (m, 15H), 3.35-2.85 (m, 6H), 2.55-2.10 (m, 12H), 1.65-1.20 (m, 5H), 1.05-0.85 (m, 6H), 0.70-0.55 (m, 3H), 0.55-0.30 (m, 1H).

### Example 4. Synthesis of Compound L-2

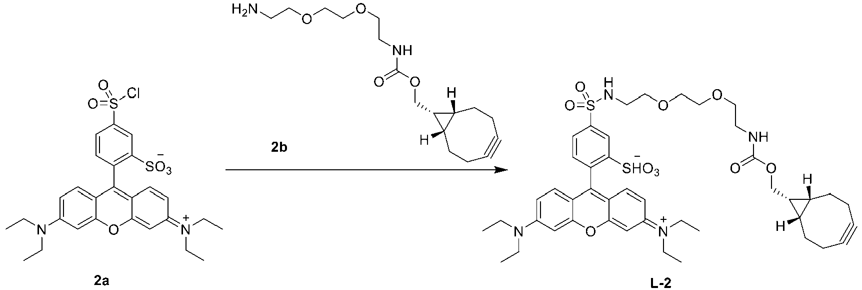

Compound **2b** (prepared with reference to the method in Angew. Chem. Int. Ed. Eng. 2010, 49, 9422-9425) (83.7 mg, 0.258 mmol) and Et₃N (104 mg, 1.03 mmol) were dissolved in CH₂Cl₂ (5 mL), and the solution was cooled in an ice-water bath in an Ar atmosphere. A solution of compound **2a** (74.7 mg, 0.129 mmol) in CH₂Cl₂ (5 mL) was added. The mixture was stirred overnight at room temperature and concentrated to dryness under reduced pressure, and the crude product was purified by preparative liquid chromatography (acetonitrile/water, 20 mM NH₄HCO₃) to give compound **L-2** (63 mg). Ms (ESI): m/z 865.3 [M+H]⁺.

¹H NMR (400 MHz, DMSO-d⁶) δ 7.86 (d, J = 1.6 Hz, 1H), 7.32 (dd, J = 8.0 2.0 Hz, 1H), 6.70 (d, J = 8.0 Hz, 1H), 6.31 (d, J = 9.6 Hz, 2H), 6.25-6.15 (m, 2H), 6.13 (d, J = 2.4 Hz, 1H), 3.30 (d, J = 8.4 Hz, 2H), 2.87 (q, J = 7.2 Hz, 8H), 2.83-2.69 (m, 8H), 2.48-2.39 (m, 5H), 1.48-1.28 (m, 6H), 0.85-0.68 (m, 2H), 0.49 (t, J = 7.2 Hz, 12H), 0.16-0.05 (m, 2H).

### Example 5. Synthesis of Compound L-3

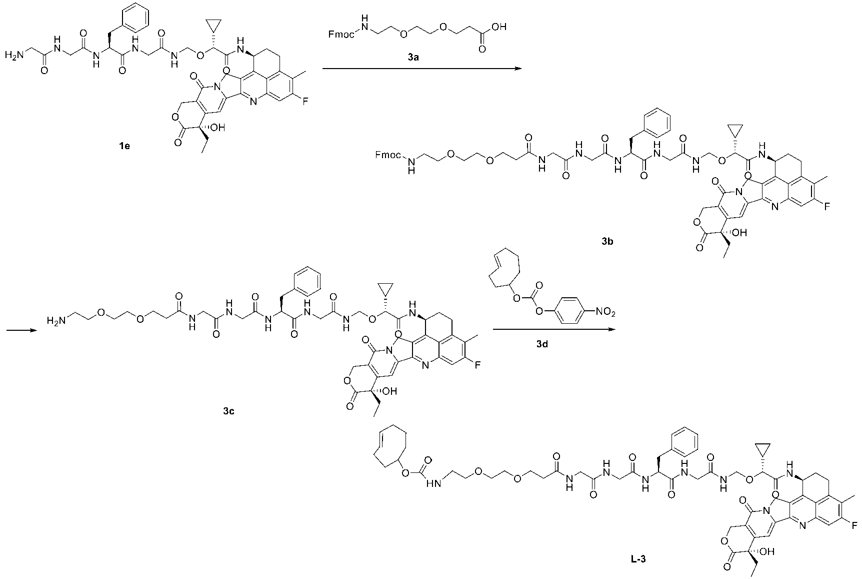

### Step 1: Synthesis of compound 3b

In a 100 mL single-necked flask, substrate **1e** (240 mg, 0.272 mmol, 1.0 eq, prepared with reference to the method in Example 18 of EP3858386A1) and compound **3a** (141 mg, 0.356 mmol, 1.3 eq) were dissolved in DCM (18 mL) and MeOH (6 mL), and the solution was purged three times with nitrogen and cooled to -30 °C. DMTMM (113 mg, 0.408 mmol, 1.5 eq) was added, and the mixture was then warmed to -17 °C and stirred until LCMS monitoring indicated the completion of the reaction. The reaction mixture was purified by column chromatography (MeOH/DCM) to give a white solid (380 mg, directly used in the next step).

Ms (ESI): m/z 1262.6 [M+H]⁺.

### Step 2: Synthesis of compound 3c

In a 10 mL single-necked flask, substrate **3b** (60 mg, 0.048 mmol, 1.0 eq) was dissolved in THF (1 mL), and the solution was cooled to -10 °C. DBU (30 mg, 0.197 mmol, 4.1 eq) was then added. After the addition, the mixture was stirred at -10 °C until LCMS monitoring indicated the completion of the reaction. MTBE (5 mL) was added to the reaction mixture, and the mixture was stirred for 5 min with the temperature maintained and was then directly filtered. The filter cake was washed twice with MTBE and dissolved in DCM (5 mL), and the solution was concentrated to dryness by rotary evaporation to give a yellow solid product (60 mg, crude, directly used in the next step). Ms (ESI): m/z 1040.8 [M+H]⁺.

### Step 3: Synthesis of compound L-3

In a 10 mL single-necked flask, substrates 2c (60 mg, crude, ~0.048 mmol, 1.0 eq) and 1b (15 mg, 0.051 mmol, 1.1 eq, purchased from Confluore, lot No. BCT-3-210428) were dissolved in DCM (1 mL), and DIEA (41 mg, 0.317 mmol, 6.6 eq) was added. The mixture was then stirred overnight at 20 °C. LCMS monitoring indicated the completion of the reaction. The reaction mixture was directly concentrated to dryness by rotary evaporation, purified twice by reversed-phase preparative chromatography (acetonitrile/water, 20 mM NH₄HCO₃), and lyophilized to give a yellow solid product (5 mg, 78.5% purity).

Ms (ESI): m/z 1192.6 [M+H]⁺.

¹H-NMR (400 MHz, CD3OD) δ 7.81 (s, 0.6H), 7.62-7.58 (m, 1H), 7.24-7.13 (m, 5H), 6.73-6.65 (m, 0.4H), 5.70-5.64 (m, 1H), 5.60-5.53 (m, 1H), 5.48-5.44 (m, 1H), 5.40-5.32 (m, 1H), 5.17-5.09 (m, 1H), 4.93-4.90 (m, 4H), 4.67 (d, J = 10.0 Hz, 1H), 4.59 (s, 1H), 4.42-4.35 (m, 1H), 4.28-4.23 (m, 1H), 3.83-3.77 (m, 4H), 3.72-3.67 (m, 3H), 3.64-3.60 (m, 1H), 3.54-3.44 (m, 6H), 3.26-3.13 (m, 4H), 3.07-2.96 (m, 1H), 2.90-2.78 (m, 1H), 2.50-2.46 (m, 2H), 2.39 (s, 3H), 2.36-2.17 (m, 6H), 2.02-1.86 (m, 4H), 1.73-1.64 (m, 2H), 1.58-1.50 (m, 1H), 1.36-1.25 (m, 3H), 1.07-0.88 (m, 3H), 0.71-0.48 (m, 4H).

### Example 6. Expression and Purification of Tyrosinase

Gene sequences of three tyrosinases-megaTYR, which is derived from *Bacillus megaterium,* VsTYR core, which is derived from *Verrucomicrobium spinosum,* and VsTYR sp-with a His6 purification tag fused to the C-terminus or N-terminus were separately synthesized onto pet28a+ vectors, and the vectors were electroporated into *E. coli* BL21 (DE3) to construct genetically engineered bacteria.

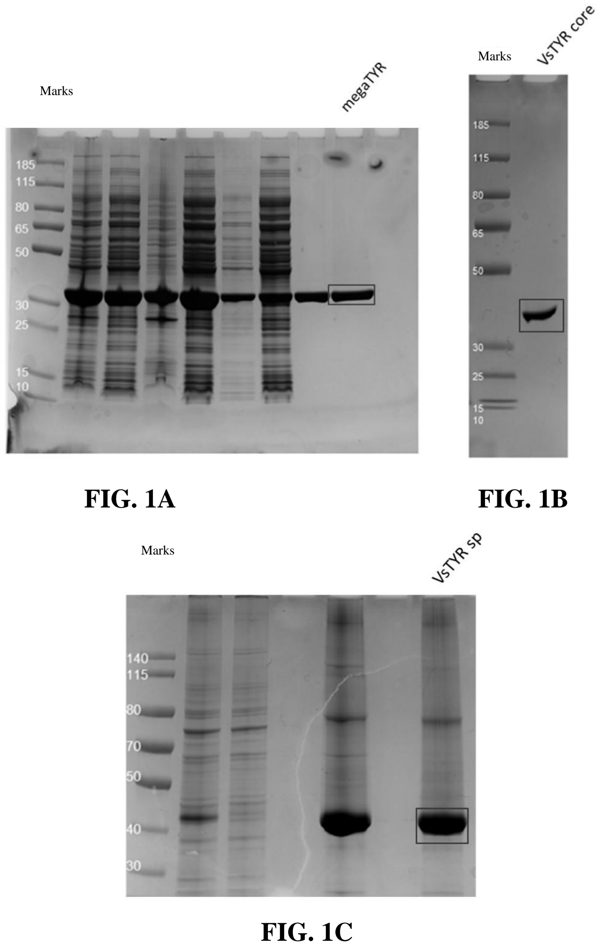

The genetically engineered *E. coli* BL21 (DE3) bacteria constructed to contain the fusion protein genes were cultured in 2*LB culture medium or other culture media commonly used for E. coli expression at 37 °C to 0.8-1.0 OD, and 0.2 mM IPTG was added. After 20 h of culture at 20 °C and 220 RPM and 10 min of centrifugation at 10,000 g, bacterial cells were obtained. The cells were resuspended in a wash buffer (500 mM NaCl, 20 mM imidazole, 20 mM Tris-HCl pH 7.5) and then disrupted by ultrasonication. After 30 min of centrifugation at 10,000 g at 4 °C, the supernatants were collected and purified using a Ni column. The elution buffer was 500 mM NaCl, 500 mM imidazole, 20 mM Tris-HCl, 0.02 mM CuSO₄, pH 7.4. Each of the purified samples was buffer-exchanged into a storage buffer (PBS, 15% glycerol, 0.02 mM CuSO₄). The purity was determined by SDS-PAGE.
Table 4 shows specific information about the three tyrosinases. All three tyrosinases were correctly expressed in *E. coli.* FIGs. 1A to 1C are SDS-PAGE images.

**Table 4. Specific information about tyrosinases**

| Tyrosinase | Species | Amino acid sequence | SDS-PAGE purity |
|---|---|---|---|
| megaTYR | *Bacillus megaterium* | SEQ ID NO:21 | >95% |
| VsTYR core | *Verrucomicrobium spinosum* | SEQ ID NO:22 | >95% |
| VsTYR sp | *Verrucomicrobium spinosum* | SEQ ID NO:23 | >80% |

### Example 7. Tyrosinase-Mediated Conjugation of Single-Point Mutated and Wild-Type Antibodies to Small Molecules

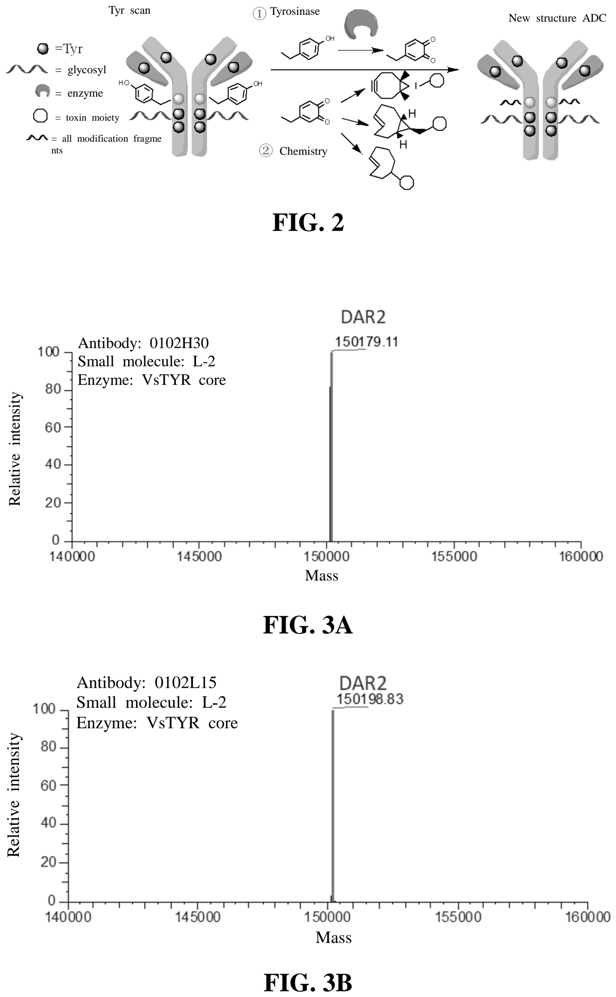

The expressed antibodies in Example 2 and the synthesized compounds in Examples 3-5 were subjected to one-pot conjugation reactions mediated by catalysis by the expressed tyrosinases in Example 6. The specific reactions are shown in FIG. 2. After an additional tyrosine is introduced into the antibody by mutation, tyrosinase can recognize it and oxidize it to quinone. The small molecule in the reaction system rapidly undergoes a 4+2 Diels Alder reaction with the quinone through a linker, thereby forming an antibody conjugate.

Specific reaction conditions: The antibody concentration was controlled to be about 2.5 mg/mL. The compound was fed in a molar ratio of greater than 5:1. The small molecule was dissolved in DMSO, and the final concentration of DMSO was controlled to be not greater than 10%. After pre-cooling on ice, tyrosinase (final concentration: 1 mg/mL) was added. The reaction pH was controlled to be about 5.5. The reaction was performed at 4 °C for 16 h. The final reaction mixture was stored at -40 °C for later analysis. RP-HPLC analysis was performed on the conjugate sample using conventional high performance liquid chromatography to determine how the HC/LC containing the corresponding mutation site(s) reacted. The conjugate sample was diluted to 1 mg/mL with PBS (pH 8.0), and dithiothreitol (DTT) (final concentration: 0.25 mol/L) was added. After 10 min of reduction at 70 °C, acetonitrile (final concentration: 20%) and 0.1% formic acid were added, and HPLC analysis was performed. The chromatography column was Agilent PLRP-S 1000A, 8 µm. The mobile phases were as follows: A: 0.1% TFA/H₂O; B: 0.1% TFA/ACN. The elution gradient is shown in Table 5. Ultraviolet absorption was measured at 280 nm.

**Table 5. The chromatography column elution gradient**

| Time (min) | A(%) | B(%) |
|---|---|---|
| 0 | 70 | 30 |
| 4 | 70 | 30 |
| 4.10 | 68 | 32 |
| 32 | 58 | 42 |
| 32.10 | 40 | 60 |
| 36 | 40 | 60 |
| 36.10 | 70 | 30 |
| 45 | 70 | 30 |

The reaction mixture of conjugation was diluted to 1 mg/mL, and formic acid (final concentration: 0.1%) was added to enhance the protonation of the sample. The sample was analyzed using a ThermoFisher QE plus mass spectrometer. The chromatography column was equilibrated with 90% mobile phase A (0.1% formic acid/water solution)-10% mobile phase B (0.1% formic acid/acetonitrile) before sample injection, and gradient elution was then performed. After the sampling was completed, mass spectrometry data of the target peak were calculated using BioPharma Finder software.

The DAR value was calculated based on the unconjugated and conjugated mass spectrometry signal intensities, and the total DAR was calculated using the following formula: total DAR = (0 * Height DO + 1 * Height D1 +...+ n * Hight Dn) / (Height DO + Height D1 +...+ Height Dn). The calculated DAR value for single-point mutated antibodies is 2.

Table 6 shows the LC-MS and RP-HPLC results after conjugation. After being modified by VsTYR, the wild-type trastuzumab molecule (Her H1) had a DAR of 0.20, and the wt 0102 molecule had a DAR of 0.01, indicating that the background modification of the trastuzumab and wild-type 0102 molecules by VsTYR was very low. However, the introduction of a single-point mutation such as T135Y, G137Y, S192Y, T223Y, S298Y (meanwhile, N297G was introduced to eliminate the influence of the N sugar on the enzymatic reaction, and Y296F and Y300F were introduced to eliminate the possibility of the enzyme catalyzing the natural tyrosines at these positions), or P329Y into the heavy chains of the two antibody molecules, or the introduction of single-point mutation S202Y into the light chains of the two antibody molecules achieved complete modification of the corresponding antibody heavy and light chains. In addition, the introduction of mutation Q342Y into the heavy chain increased the ratio of modification of the mutant of trastuzumab by VsTYR to DAR 1.16; however, the introduction of T197Y into the heavy chain did not significantly increase the modification ratio. FIGs. 3A and 3B illustratively show deconvolved mass spectrometry results for some of the tyrosinase-mediated conjugates of single-point mutated antibodies and small molecules. In addition, the mushroom-derived tetrameric tyrosinase (Sigma Aldrich T3824) was not able to modify the wild-type 0102 molecule or the molecule with G137Y introduced into the heavy chain of 0102.

**Table 6. The results of the tyrosinase-mediated conjugation of single-point mutated and wild-type antibodies to small molecules**

| Antibody No. | Enzyme | Compound | RP monitoring of reaction | Calculated increase in molecular weight Da (+1 Drug) | DAR value (calculated by MS) |
|---|---|---|---|---|---|
| Her H1 | VsTYR core | L-1 | LC did not react/HC reacted very little | 1231.30 | 0.20 |
| Her H9 | VsTYR core | L-1 | HC reacted completely | 1231.30 | 2.23 |
| Her H10 | VsTYR core | L-1 | HC reacted completely | 1231.30 | 2.25 |
| Her H19 | VsTYR core | L-1 | HC reacted completely | 1231.30 | 2.10 |
| Her H30 | VsTYR core | L-2 | HC reacted completely | 880.06 | 2.10 |
| Her H53 | VsTYR core | L-3 | HC reacted completely | 1206.27 | 2.39 |
| Her H26 | VsTYR core | L-2 | HC reacted completely | 880.06 | 2.25 |
| Her L15 | VsTYR core | L-2 | LC reacted completely | 880.06 | 2.25 |
| Her H14 | VsTYR core | L-2 | No significant increase in reaction compared to wt | 880.06 | 0.38 |
| Her H33 | VsTYR core | L-2 | HC reaction increased | 880.06 | 1.16 |
| Her H10 | VsTYR sp | L-2 | HC reacted completely | 880.06 | 2.00 |
| Her H10 | Mega TYR | L-2 | HC reacted completely | 880.06 | 2.24 |
| 0102 | VsTYR core | L-2 | Both LC/HC did not react | 880.06 | 0.01 |
| 0102 | Mushroo m-derived tetrameric tyrosinase | L-2 | Both LC/HC did not react | 880.06 | 0.00 |
| 0102 H9 | VsTYR core | L-2 | HC reacted completely | 880.06 | 1.81 |
| 0102 H10 | VsTYR core | L-2 | HC reacted completely | 880.06 | 1.96 |
| 0102 H19 | VsTYR core | L-2 | HC reacted completely | 880.06 | 1.60 |
| 0102 H30 | VsTYR core | L-2 | HC reacted completely | 880.06 | 2.00 |
| 0102 H53 | VsTYR core | L-2 | HC reacted completely | 880.06 | 1.84 |
| 0102 L15 | VsTYR core | L-2 | LC reacted completely | 880.06 | 2.00 |
| 0102 H26 | VsTYR core | L-2 | HC reacted completely | 880.06 | 2.00 |
| 0102 H10 | Mushroo m-derived tetrameric tyrosinase | L-2 | Both LC/HC did not react | 880.06 | 0.00 |

### Example 8. Expression and Identification of Multi-tyrosine Combination Mutated Antibodies

The additionally introduced highly reactive tyrosines demonstrated in Example 7 were subjected to multi-site combination and introduced into trastuzumab. Meanwhile, the highly reactive tyrosines were additionally introduced into trastuzumab, and a GGGGY sequence was included at the C-terminus of the LC. Antibody expression was performed according to the antibody expression and purification method in Example 2. Meanwhile, LC-MS identification was performed according to the method in Example 2. Related information about some of the expressed antibodies is shown in Table 7. The expressed molecular weights were all consistent with the calculated values.

**Table 7. The expression and identification of multi-site mutated antibodies (all of which are of the IGHG1 and Cκ types)**

| Antibody No. | Mutations | Heavy chain HC | Light chain LC | Calculated molecular weight Da (+2*G0F) | Found molecular weight Da (+2*G0F) |
|---|---|---|---|---|---|
| Her HH3 | T135Y/T223Y/P329Y of HC and S202Y of CL | SEQ ID NO:24 | SEQ ID NO:11 | 148588.48 | 148586.19 |
| Her HH4 | T135Y/T223Y/P329Y/S192Y of HC | SEQ ID NO:25 | SEQ ID NO:2 | 148588.48 | 148586.11 |
| Her HH7 | T135Y/T223Y/P329Y of HC | SEQ ID NO:24 | SEQ ID NO:2 | 148436.29 | 148433.52 |
| Her HH8 | T135Y/P329Y of HC and S202Y of CL | SEQ ID NO:26 | SEQ ID NO:11 | 148464.34 | 148461.84 |
| Her HH9 | T223Y/P329Y of HC | SEQ ID NO:27 | SEQ ID NO:2 | 148312.15 | 148309.81 |
| Her HH11 | P329Y of HC and S202Y of CL | SEQ ID NO:7 | SEQ ID NO:11 | 148340.20 | 148338.88 |
| Her HH12 | S192Y of HC and S202Y of CL | SEQ ID NO:9 | SEQ ID NO:11 | 148360.28 | 148359.17 |
| Her HH13 | G137Y of HC and terminal GGGGY of CL | SEQ ID NO:4 | SEQ ID NO:67 | 149052.16 | 149051.08 |

### Example 9. Tyrosinase-Mediated Conjugation of Multi-Site Mutated Antibodies to Compounds

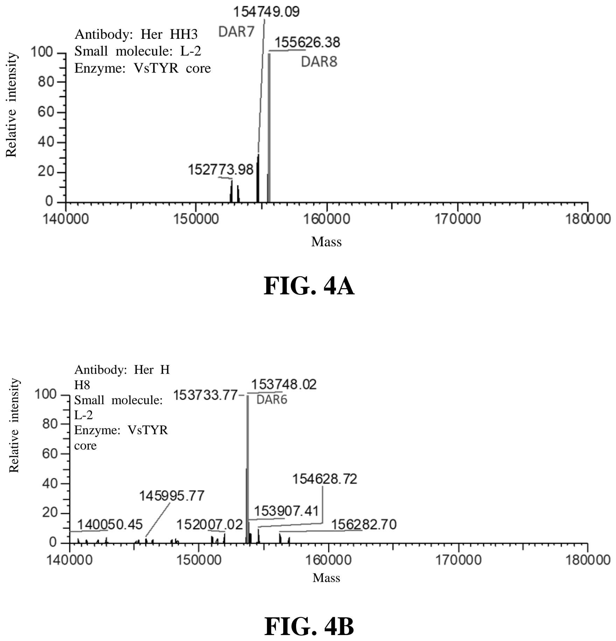

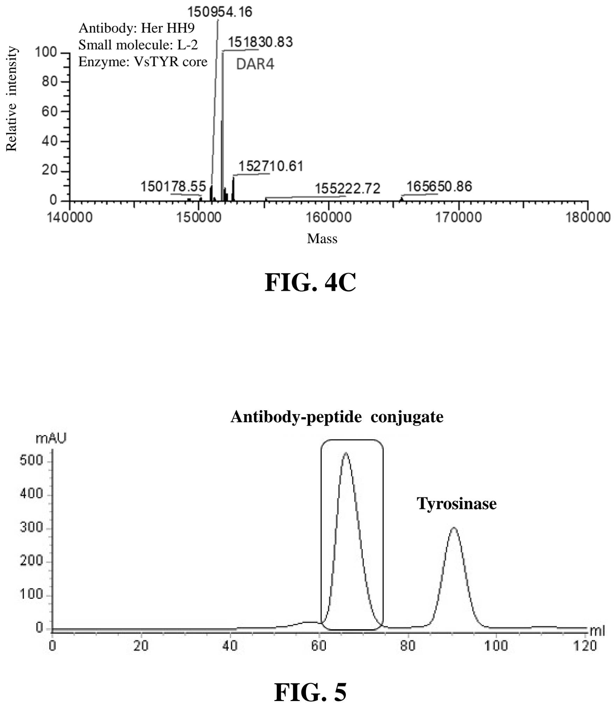

In this example, the expressed antibodies in Example 8 and the synthesized compounds in Examples 3-5 were subjected to one-pot conjugation reactions mediated by catalysis by the VsTYR core enzyme. The specific conjugation conditions and LC-MS detection were the same as those in Example 7. Meanwhile, DAR values were calculated. Table 8 shows the LC-MS results after conjugation. The found DAR values were consistent with the calculated ones, indicating that the simultaneous introduction of multiple highly reactive tyrosine mutations into the antibody constant region (as described in Example 7) enabled complete modification at multiple sites through the VsTYR tyrosinase-catalyzed reaction. FIGs. 4A to 4C illustratively show deconvolved mass spectrometry results for some of the tyrosinase-mediated conjugates of multi-site mutated antibodies and a small molecule.

**Table 8. The results of the tyrosinase-mediated conjugation of multi-site mutated antibodies to a small molecule**

| Antibody No. | Compound | Calculated increase in molecular weight Da (+1 drug molecule) | Calculated DAR | DAR value (calculated by MS) |
|---|---|---|---|---|
| Her HH3 | L-2 | 880.06 | 8 | 7.76 |
| Her HH4 | L-2 | 880.06 | 8 | 7.19 |
| Her HH7 | L-2 | 880.06 | 6 | 5.73 |
| Her HH8 | L-2 | 880.06 | 6 | 6.07 |
| Her HH9 | L-2 | 880.06 | 4 | 4.05 |
| Her HH11 | L-2 | 880.06 | 4 | 4.13 |
| Her HH12 | L-2 | 880.06 | 4 | 4.05 |
| Her HH13 | L-2 | 880.06 | 4 | 4.08 |

### Example 10. Melting Point (TM) Analysis of Single-Point Mutated and Wild-Type Antibodies

A differential scanning calorimetry (DSC) experiment was performed using a MicroCal PEAQ-DSC Automated microcalorimeter to determine the melting points of some of the single-point mutated antibodies in Example 2 and to test the stability of the antibodies after the mutations. Samples (350 µL, with the concentration controlled at 1.0 mg/mL) were placed in a 96-well plate. Sample bottles were placed in the sample compartment, and the position numbers of the samples were recorded. For each sample, two groups were set: a blank group and a control group. The blank group was generally PBS. The running sequence included the first three wells being blank and each of the following wells containing a sample, with an injection of wash buffer inserted after every five samples. With the blank group as a baseline, the fitting model was confirmed, and the corresponding melting points were calculated using software. Table 9 shows the specific results, which indicate that the tested single-point mutation sites had little effect on the thermal stability of the proteins.

**Table 9. Melting point (TM) analysis of single-point mutated and wild-type antibodies**

| Antibody No. | Mutation | Tonset (°C) | TM1 (°C) | TM2 (°C) |
|---|---|---|---|---|
| Her H1 | / | 64.99 | 71.90 | 82.08 |
| Her H9 | T135Y | 64.80 | 71.65 | 81.99 |
| Her H10 | G137Y | 64.57 | 71.50 | 81.91 |
| Her H19 | T223Y | 64.60 | 71.35 | 81.96 |
| Her H30 | P329Y | 62.29 | 68.70 | 81.94 |
| Her H53 | S192Y | 64.47 | 71.43 | 81.89 |
| Her L15 | S202Y | 64.52 | 71.58 | 81.77 |

### Example 11. Analysis of Binding of Single-Point Mutated and Wild-Type Antibodies to Human FcRn

The affinities of some of the mutant antibodies of trastuzumab in Example 2 and wild-type trastuzumab (Her H1) for FcRn were determined. The experiment was performed using a Biacore 8K system (GE Healthcare). PBS-P+ buffer solution (0.2 M PBS, 27 mM KCl, 1.37 M NaCl, 0.05% surfactant P20) was used as the mobile phase. Human FcRn (Acro Biosystems, FCN-H52W7) protein was prepared in PBS-P+ buffer solution as a ligand and captured using the Anti-His antibody protein on chip channels. Each test antibody was used as an analyte and prepared in PBS-P+ buffer solution, and the analyte was serially diluted 2-fold from 500 nM to obtain a total of 7 concentration points and was allowed to flow through the experimental and reference channels at a flow rate of 30 µL/min, with an association time of 60 s and a dissociation time of 90 s. The regeneration buffer (10 mM Glycine pH 1.5 (GE Healthcare, 29238268-AA)) was run at a flow rate of 10 µL/min for 30 s. Data were processed using Biacore 8K Evaluation analysis software. The corresponding reference channel (Fc 1) signal values were subtracted from the detection channel (Fc 2) signal values to obtain corrected signal curves. Affinity kinetic curves were fitted according to the 1:1 Langmuir binding model, and the association rate Ka, the dissociation rate Kd, and the dissociation constant (i.e., the affinity KD value) were calculated.

Table 10 shows that there was no significant difference in FcRn affinity between the single-point mutated and wild-type antibodies tested.

**Table 10. The results of the binding of single-point mutated and wild-type antibodies to human FcRn**

| Ligand | Analyte | ka (1/Ms) | kd (1/s) | KD (M) | Binding model |
|---|---|---|---|---|---|
| Human FcRn | Her H1 | 9.69E+05 | 1.68E-01 | 1.73E-07 | 1: 1 |
| | Her H9 | 1.09E+06 | 2.19E-01 | 2.01E-07 | |
| | Her H10 | 1.01E+06 | 1.93E-01 | 1.91E-07 | |
| | Her H19 | 4.02E+05 | 9.41E-02 | 2.34E-07 | |
| | Her H26 | 4.37E+05 | 9.94E-02 | 2.27E-07 | |
| | Her H30 | 3.91E+05 | 6.66E-02 | 1.70E-07 | |
| | Her H53 | 3.98E+05 | 7.81E-02 | 1.96E-07 | |
| | Her L15 | 1.81E+06 | 3.86E-01 | 2.13E-07 | |

### Example 12. Analysis of Binding of Single-Point Mutated and Wild-Type Antibodies to Human CD16a

The affinities of some of the mutant antibodies of trastuzumab in Example 2 and wild-type trastuzumab (Her H1) for CD16a were determined. The experiment was performed using a Biacore 8K system (GE Healthcare). An anti-His sensor chip was selected. For the detection of the human CD16a-F176/human CD16a-V176 (Acro Biosystems, CDA-H5220, CD8-H52H4) receptors, HBS-EP⁺ buffer solution (10 mM HEPES, 150 mM NaCl, 3 mM EDTA, 0.05% surfactant P20) was used as the mobile phase. The human CD16a-F176 & human CD16a-V176 receptors were separately prepared in HBS-EP⁺ buffer solution as ligands and captured using the anti-His antibody protein on chip channels. Each test antibody was used as an analyte and prepared in HBS-EP⁺ buffer solution, and the analyte was serially diluted 2-fold from 2000 nM to obtain a total of 7 concentration points and was allowed to flow through the experimental and reference channels at a flow rate of 30 µL/min, with an association time of 60 s and a dissociation time of 60 s. The regeneration buffer (10 mM Glycine pH 1.5 (GE Healthcare, 29238268-AA)) was run at a flow rate of 10 µL/min for 30 s. Data were processed using Biacore 8K Evaluation analysis software. The corresponding reference channel (Fc 1) signal values were subtracted from the detection channel (Fc 2) signal values to obtain corrected signal curves. Affinity kinetic curves were fitted according to the 1:1 Langmuir binding model, and the association rate Ka, the dissociation rate Kd, and the dissociation constant (i.e., the affinity KD value) were calculated.

Table 11 shows that the two antibodies Her H26, in which a glycan chain capable of binding to CD16a is removed, and Her H30, in which the mutated P329 is a key amino acid for CD16a binding, did not bind to CD16a, which is in line with the expectation reported in the literature (Bogen JP, et al. Design of a Trispecific Checkpoint Inhibitor and Natural Killer Cell Engager Based on a 2+1 Common Light Chain Antibody Architecture. Front Immunol. 2021 May 10;12:669496). There was no significant difference in CD16a affinity between the remaining single-point mutated and wild-type antibodies tested.

**Table 11. The results of the binding of single-point mutated and wild-type antibodies to human CD16a**

| Ligand | Analyte | ka (1/Ms) | kd (1/s) | KD (M) | Binding model |
|---|---|---|---|---|---|
| CD16a-F176 | Her H1 | 8.63E+04 | 5.95E-02 | 6.90E-07 | |
| | Her H9 | 7.95E+04 | 4.83E-02 | 6.07E-07 | |
| | Her H10 | 7.97E+04 | 4.02E-02 | 5.05E-07 | |
| | Her H19 | 7.91E+04 | 5.35E-02 | 6.76E-07 | |
| | Her H26 | / | / | No binding | |
| | Her H30 | / | / | No binding | |
| | Her H53 | 7.41E+04 | 4.08E-02 | 5.51E-07 | |
| | Her L15 | 8.24E+04 | 5.87E-02 | 7.12E-07 | 1: 1 |
| CD16a-V176 | Her H1 | 8.64E+04 | 1.73E-02 | 2.00E-07 | |
| | Her H9 | 8.46E+04 | 1.56E-02 | 1.85E-07 | |
| | Her H10 | 8.84E+04 | 1.56E-02 | 1.76E-07 | |
| | Her H19 | 7.97E+04 | 1.59E-02 | 1.99E-07 | |
| | Her H26 | / | / | No binding | |
| | Her H30 | / | / | No binding | |
| | Her H53 | 8.37E+04 | 1.56E-02 | 1.87E-07 | |
| | Her L15 | 8.33E+04 | 1.63E-02 | 1.95E-07 | |

### Example 13. Design, Expression, and Identification of Single-Point Mutated Antibodies with Other IGHG Forms

Table 12 shows selectable sites for other IGHG forms (IGHG2, IGHG3, and IGHG4) selected based on calculations similar to those in Example 1. Moreover, the hinge region linking the Fab and the Fc has relatively large flexibility and is therefore a potential site for site-specific modification.

**Table 12. The selected sites of antibodies with other IGHG forms (IGHG2, IGHG3, and IGHG4)**

| IGHG form | Position number^{[1]} | Position number corresponding to IGHG1 | Original amino acid | SASA_mut(Å²) | SASA_ori(Å²) |
|---|---|---|---|---|---|
| IGHG2 | 133 | 135 | Thr | 151.81 | 91.88 |
| IGHG2 | 135 | 137 | Glu | 67.75 | 70.25 |
| IGHG2 | 197 | 192 | Asn | 142.24 | 77.89 |
| IGHG2 | 298 | 298 | Ser | 163.59 | 103.3 |
| IGHG2 | 329 | 329 | Pro | 84.29 | 76.95 |
| IGHG3 | 298 | 298 | Ser | 121.18 | 71.20 |
| IGHG3 | 329 | 329 | Pro | 176.88 | 150.49 |
| IGHG4 | 138 | 135 | Thr | 199.83 | 134.05 |
| IGHG4 | 140 | 137 | Glu | 27.17 | 47.63 |
| IGHG4 | 195 | 192 | Ser | 60.94 | 44.27 |
| IGHG4 | 298 | 298 | Ser | 127.87 | 92.85 |
| IGHG4 | 329 | 329 | Pro | 206.71 | 137.91 |

| | | | | | |
|---|---|---|---|---|---|
| Note: SASA_ori (Å²) is the original SASA (Å²), and SASA_mut (Å²) is the SASA (Å²) after mutation. [1] For IGHG2, numbering was performed with PDB: 4L4J and PDB: 2QSC as template references; for IGHG4, numbering was performed with PDB: 5W5N and PDB: 3EO1 as template references; the Fc region of IGHG3 was defined based on the EU numbering scheme for IGHG1. | | | | | |

The designed mutation sites of different IGHG forms were subjected to single-point specific mutation on the sequence of the 0102 molecule. Meanwhile, the non-mutated wild types (wt) were used as controls. Expression and identification were performed in the manner described in Example 2.

Related information about some of the expressed antibodies is shown in Table 13. The expressed molecular weights were all consistent with the calculated values.

**Table 13. The expression and identification of wild-type antibodies (wt) and single-point mutated antibodies**

| Antibody No. | IGHG form | Mutation | Heavy chain HC | Light chain LC | Calculated molecular weight Da (+2*G0F) | Found molecular weight Da (+2*G0F) |
|---|---|---|---|---|---|---|
| Wild type | | | | | | |
| 0102 H73 | IGHG2 | / | SEQ ID NO:28 | SEQ ID NO:13 | 147810.68 | 147811 |
| 0102 H74 | IGHG3 | / | SEQ ID NO:29 | | 158537.29 | 158537 |
| 0102 H40 | IGHG4 | / | SEQ ID NO:30 | | 147914.60 | 147915 |

| With mutation in IGHG | | | | | | |
|---|---|---|---|---|---|---|
| 0102 H75 | IGHG2 | T133Y | SEQ ID NO:31 | SEQ ID NO:13 | 147934.82 | 147936 |
| 0102 H76 | IGHG2 | N197Y | SEQ ID NO:32 | | 147908.83 | 147910 |
| 0102 H77 | IGHG3 | P329Y | SEQ ID NO:33 | | 158669.41 | 158670 |
| 0102 H78 | IGHG4 | T138Y | SEQ ID NO:34 | | 148038.74 | 148052 |
| 0102 H79 | IGHG4 | P329Y | SEQ ID NO:35 | | 148046.72 | 148068 |

### Example 14. Tyrosinase-Mediated Conjugation of Single-Point Mutated and Wild-Type Antibodies with Other IGHG Forms to Small Molecules

In this example, the expressed antibodies in Example 13 and the synthesized compounds in Examples 3-5 were subjected to one-pot conjugation reactions mediated by catalysis by the VsTYR core enzyme. The specific conjugation conditions and LC-MS detection were the same as those in Example 7. Meanwhile, DAR values were calculated. Table 14 shows the DAR values calculated by LC-MS after conjugation. After being modified by VsTYR core, the 0102 H73 molecule, which contains wild-type IGHG2, had a DAR of 0.08. After being modified by VsTYR core, the 0102 H74 molecule, which contains wild-type IGHG3, had a DAR of 0.00. After being modified by VsTYR core, the 0102 H40 molecule, which contains wild-type IGHG4, had a DAR of 0.36, indicating that VsTYR exhibited a certain background modification effect on wild-type IGHG4. The introduction of a single-point mutation such as T133Y or N197Y into IGHG2 and the introduction of a single-point mutation such as T138Y or P329Y into IGHG4 increased the ratio of modification of the corresponding mutant molecules of 0102 by VsTYR. The introduction of single-point mutation P329Y into IGHG3 achieved complete modification of the corresponding antibody heavy and light chains.

**Table 14. The results of the tyrosinase-mediated conjugation of single-point mutated and wild-type antibodies to a small molecule**

| Antibody No. | IGHG form | Compound | Calculated increase in molecular weight Da (+1 Drug) | DAR value (calculated by MS) |
|---|---|---|---|---|
| Wild type | | | | |
| 0102 H73 | IGHG2 | L-2 | 880.06 | 0.08 |
| 0102 H74 | IGHG3 | L-2 | 880.06 | 0.00 |
| 0102 H40 | IGHG4 | L-2 | 880.06 | 0.36 |

| With mutation in IGHG | | | | |
|---|---|---|---|---|
| 0102 H75 | IGHG2 | L-2 | 880.06 | 0.40 |
| 0102 H76 | IGHG2 | L-2 | 880.06 | 0.33 |
| 0102 H77 | IGHG3 | L-2 | 880.06 | 1.85 |
| 0102 H78 | IGHG4 | L-2 | 880.06 | 1.46 |
| 0102 H79 | IGHG4 | L-2 | 880.06 | 1.73 |

### Example 15. Expression and Identification of Other Single (Multiple)-Site Mutated Antibodies

Expression and identification of other single (multiple)-site mutated antibodies were performed in the manner described in Example 2. c-met H10 and c-met L15 were monoclonal antibodies targeting c-met. ADA mut was obtained by performing site-specific mutations HC P329Y and LC S202Y on the sequence of adalimumab. 1921 H10 L15 was a monoclonal antibody targeting TROP2. Table 15 shows that the molecular weights of the expressed antibodies were all consistent with the calculated values.

**Table 15. The expression and identification of other single (multiple)-site mutated antibodies**

| Antibody No. | Mutation(s) | Heavy chain HC | Light chain LC | Calculated molecular weight Da (+2*G0F) | Found molecular weight Da (+2*G0F) |
|---|---|---|---|---|---|
| c-met H10 | G137Y of IGHG1 HC | SEQ ID NO:36 | SEQ ID NO:37 | 148836.23 | 148837 |
| c-met L15 | S202Y of CL | SEQ ID NO:38 | SEQ ID NO:39 | 148775.95 | 148777 |
| ADA mut | P329Y of IGHG1 HC and S202Y of CL | SEQ ID NO:40 | SEQ ID NO:41 | 148429.56 | 148431 |
| 1921 mut | G137Y of IGHG1 HC and S202Y of CL | SEQ ID NO:42 | SEQ ID NO:43 | 148529.94 | 148530 |

### Example 16. Synthesis of Polypeptide P-1

### P-1 peptide sequence: EQKLISEEDLGGSC (TCO-Mal) (SEQ ID NO: 80)

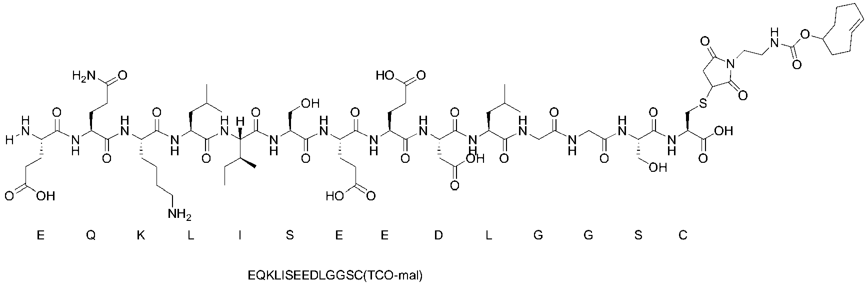

### Step 1: Synthesis of resin-peptide

1.1. 2-Cl Resin (degree of substitution: 0.3 mmol/g) was selected. According to the above peptide sequence, from the C-terminus to the N-terminus (from right to left), the amino acids were sequentially subjected to condensation using the Fmoc-process until the peptide chain condensation was completed. 1 g of 2-Cl resin was added to a clean reactor, and 10 mL of DCM was added. The resin was soaked and swollen for 20 min, and the liquid was then removed by filtration under reduced pressure.

1.2. 0.3 mmol of Fmoc-Cys(Trt)-OH and 0.6 mmol of HoBt were accurately weighed out and dissolved in 6 mL of DMF, and 0.6 mmol of DIC was added dropwise. After 10 min of activation, the mixture was added to a reaction column. After 2 h, the reaction was stopped. Washing was performed 3 times with a proper amount of DMF, and 2 mL of DIEA, 2 mL of methanol, and 6 mL of DCM were added. After 35 min of reaction, capping was completed. The reaction liquid was removed under reduced pressure, and the resin was washed 3 times with DMF.

### 1.3. Removal of FMOC:

10 mL of 20% piperidine/DMF solution was added, and the mixture was stirred for 10 min. The liquid was removed under reduced pressure. Another 10 mL of 20% piperidine/DMF solution was added, and the mixture was stirred for 5 min. The liquid was removed under reduced pressure, and washing was performed 5 times with DMF. A sample was taken and subjected to a ninhydrin color development test, and the color (blue) was recorded.

### 1.4. Condensation reaction

Feeding was performed in an amount that was 3 times the molar quantity of the degree of substitution of the resin. 0.9 mmol of Fmoc-Ser(tbu)-OH and 0.9 mmol of HoBt were weighed out and dissolved in 6 mL of DMF, and 0.9 mmol of DIC was added dropwise. After 10 min of activation, the mixture was added to a reaction column. After 1.5 h of reaction (a ninhydrin color development test was performed, and no color development was observed), the liquid was removed under reduced pressure. Washing was performed 5 times with DMF.

### 1.5. Cycle reaction

The procedures in steps 1.4 to 1.5 were repeated until the condensation of the last glutamic acid was completed. Washing was performed 3 times with DMF, and a sample was taken and subjected to a ninhydrin color development test. The sample showed no color development, indicating that the reaction was complete. After the condensation was completed, Fmoc was removed according to the procedures in step 1.3, and the residue was washed 3 times with MeOH and filtered under reduced pressure to dryness.

### Step 2: Cleavage of polypeptide

Preparation of cleavage reagent: The volume of the cleavage reagent used was calculated using a ratio of the resin-peptide to the cleavage solution of 1 g:15 mL:
TFA:TIS:EDT:H2O = 95:2:2:1. The required cleavage reagent H₂O and TFA were sequentially added to a cleavage reaction flask, and the temperature of the cleavage reagent was controlled at 0 to 10 °C. The cleavage reagent was added to the peptide-resin with stirring. After the temperature of the system stabilized, the temperature was then controlled at 20 to 25 °C, and the mixture was stirred for 2 h.

The cleavage solution was collected by filtration and subjected to precipitation using an amount of cold diethyl ether that was 10 times the volume of the filtrate. The precipitate was centrifugally washed 4 times and dried at room temperature under reduced pressure to give a crude product (0.7 g).

### Step 3: Purification of naked peptide

With a 0.1% aqueous TFA solution as mobile phase A and 0.1% TFA in acetonitrile as mobile phase B, a liquid chromatography system was prepared. A 10 µm reversed-phase C18 column (50 × 250 mm) was used and packed. The ultraviolet detector was set at 220 nm. The flow rate was adjusted to 40 mL/min. The column was equilibrated with 10% acetonitrile for 10 min. The crude peptide was ground and crushed and then completely dissolved in purified water under microwaves. The solution was filtered through a 0.45 µm filter membrane and injected.

Elution was performed using a 10% 0.01 min - 45% 40 min acetonitrile concentration gradient at room temperature. Changes in absorbance were noted, the impurity peaks were removed, and the target product was collected. According to analysis, a >95% purified liquid was obtained. The purified liquid was lyophilized to give the target product (white powder, 100 mg). After the polypeptide was lyophilized, purity and MS analyses were performed again. The results showed a purity of 95.684% and an MS molecular weight of 1507.62. The calculated yield of the naked peptide was 22.11%.

### Step 4: Modification of naked peptide with TCO-mal

30 mg of the naked peptide was dissolved in 10 mL of 10% aqueous acetonitrile solution, and 10 mg of TCO-Mal was added. The solution was stirred at room temperature, and a sample of the solution was taken for MS analysis once every 30 min until the molecular weight of the naked peptide (1507.62) disappeared.

### Step 5: Purification of target peptide

With a 0.1% aqueous TFA solution as mobile phase A and 0.1% TFA in acetonitrile as mobile phase B, a liquid chromatography system was prepared. A 10 µm reversed-phase C18 column (50 × 250 mm) was used and packed. The ultraviolet detector was set at 220 nm. The flow rate was adjusted to 40 mL/min. The column was equilibrated with 10% acetonitrile for 10 min. The reaction mixture was subjected to elution with a 10% 0.01 min - 45% 40 min acetonitrile concentration gradient, and the target product was collected. According to analysis, a >95% purified liquid was obtained. The collected purified liquid was lyophilized to give the target product (white powder, 15 mg). After the polypeptide was lyophilized, purity and MS analyses were performed again. The results showed a purity of 95.051% and an MS molecular weight of 1799.962. The yield was 39.82%.

### Example 17. Synthesis of Polypeptide P-2

### P-2 peptide sequence: LRELHLNNNC (TCO-mal) (SEQ ID NO: 81)

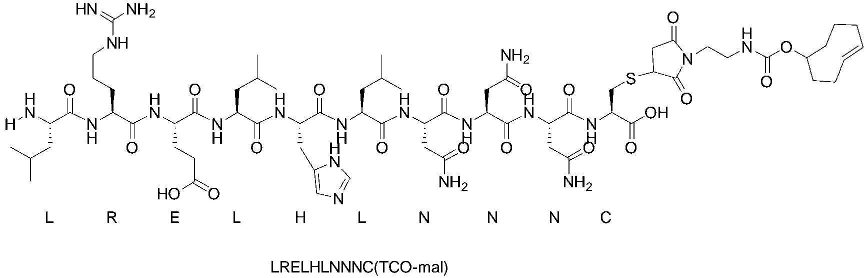

### Step 1: Synthesis of resin-peptide

1.1. 2-CL resin (degree of substitution: 0.3 mmol/g) was selected. According to the above peptide sequence, from the C-terminus to the N-terminus (from right to left), the amino acids were sequentially subjected to condensation using the Fmoc-process until the peptide chain condensation was completed. 2 g of 2-CL resin was added to a clean reactor, and 20 mL of DCM was added. The resin was soaked and swollen for 20 min, and the liquid was then removed by filtration under reduced pressure.

1.2. 0.6 mmol of Fmoc-Cys(trt)-OH and 0.9 mmol of HoBt were accurately weighed out and dissolved in 10 mL of DMF, and 0.9 mmol of DIC was added dropwise. After 10 min of activation, the mixture was added to a reaction column. After 2 h, the reaction was stopped. Washing was performed 3 times with a proper amount of DMF, and 3 mL of DIEA, 3 mL of methanol, and 10 mL of DCM were added. After 35 min of reaction, capping was completed. The reaction liquid was removed under reduced pressure, and the resin was washed 3 times with DMF.

### 1.3. Removal of FMOC

15 mL of 20% piperidine/DMF solution was added, and the mixture was stirred for 10 min. The liquid was removed under reduced pressure. Another 15 mL of 20% piperidine/DMF solution was added, and the mixture was stirred for 5 min. The liquid was removed under reduced pressure, and washing was performed 5 times with DMF. A sample was taken and subjected to a ninhydrin color development test, and the color (blue) was recorded.

### 1.4. Condensation reaction

Feeding was performed in an amount that was 3 times the molar quantity of the degree of substitution of the resin. 1.8 mmol of Fmoc-Asn(trt)-OH and 1.8 mmol of HoBt were weighed out and dissolved in 10 mL of DMF, and 2.7 mmol of DIC was added dropwise. After 10 min of activation, the mixture was added to a reaction column. After 1.5 h of reaction (a ninhydrin color development test was performed, and no color development was observed), the liquid was removed under reduced pressure. Washing was performed 5 times with DMF.

### 1.5. Cycle reaction

The procedures in steps 1.4 to 1.5 were repeated until the condensation of the last leucine was completed. Washing was performed 3 times with DMF, and a sample was taken and subjected to a ninhydrin color development test. The sample showed no color development, indicating that the reaction was complete. After the condensation was completed, Fmoc was removed according to the procedures in step 1.3, and the residue was washed 3 times with MeOH and filtered under reduced pressure to dryness.

### Step 2: Cleavage of polypeptide

Preparation of cleavage reagent: The volume of the cleavage reagent used was calculated using a ratio of the resin-peptide to the cleavage solution of 1 g:15 mL: TFA:TIS:EDT:H2O = 95:2:2:1. The required cleavage reagent H₂O and TFA were sequentially added to a cleavage reaction flask, and the temperature of the cleavage reagent was controlled at 0 to 10 °C. The cleavage reagent was added to the peptide-resin with stirring. After the temperature of the system stabilized, the temperature was then controlled at 20 to 25 °C, and the mixture was stirred for 2 h. The cleavage solution was collected by filtration and subjected to precipitation using an amount of cold diethyl ether that was 10 times the volume of the filtrate. The precipitate was centrifugally washed 4 times and dried at room temperature under reduced pressure to give a crude product (1.3 g).

### Step 3: Purification of naked peptide

With a 0.1% aqueous TFA solution as mobile phase A and 0.1% TFA in acetonitrile as mobile phase B, a liquid chromatography system was prepared. A 10 µm reversed-phase C18 column (50 × 250 mm) was used and packed. The ultraviolet detector was set at 220 nm. The flow rate was adjusted to 40 mL/min. The column was equilibrated with 10% acetonitrile for 10 min. The crude peptide was ground and crushed and then dissolved in a 10% aqueous acetonitrile solution. The resulting solution was microwaved until the peptide was completely dissolved. The solution was filtered through a 0.45 µm filter membrane and injected. Elution was performed with a 10% 0.01 min - 45% 40 min acetonitrile concentration gradient at room temperature. Changes in absorbance were noted, the impurity peaks were removed, and the target product was collected. According to analysis, a >95% purified liquid was obtained. The purified liquid was lyophilized to give the target product (white powder, 210 mg). After the polypeptide was lyophilized, purity and MS analyses were performed again. The results showed a purity of 95.853% and an MS molecular weight of 1225.376. The calculated yield of the naked peptide was 27.38%.

### Step 4: Modification of naked peptide with TCO-mal

100 mg of the naked peptide was dissolved in 20 mL of 10% aqueous acetonitrile solution, and 30 mg of TCO-Mal was added. The solution was stirred at room temperature, and a sample of the solution was taken for MS analysis once every 30 min until the molecular weight of the naked peptide (1225.376) disappeared.

### Step 5: Purification of target peptide

With a 0.1% aqueous TFA solution as mobile phase A and 0.1% TFA in acetonitrile as mobile phase B, a liquid chromatography system was prepared. A 10 µm reversed-phase C18 column (50 × 250 mm) was used and packed. The ultraviolet detector was set at 220 nm. The flow rate was adjusted to 40 mL/min. The column was equilibrated with 10% acetonitrile for 10 min. The reaction mixture was subjected to elution with a 10% 0.01 min - 45% 40 min acetonitrile concentration gradient, and the target product was collected. According to analysis, a >95% purified liquid was obtained. The collected purified liquid was lyophilized to give the target product (white powder, 50 mg). After the polypeptide was lyophilized, purity and MS analyses were performed again. The results showed a purity of 98.821% and an MS molecular weight of 1517.72. The yield was 40.36%.

### Example 18. Tyrosinase-Mediated Conjugation of Single (Multiple)-Site Mutated Antibodies to Polypeptides

In this example, the expressed antibodies in Example 15 and the synthesized polypeptides in Examples 16 and 17 were subjected to one-pot conjugation reactions mediated by catalysis by the VsTYR core enzyme. The specific conjugation conditions and LC-MS detection were the same as those in Example 7. Meanwhile, DAR values were calculated.

Table 16 shows the results of the calculation of DAR values after conjugation, which indicate that the antibodies containing mutation sites can be conjugated to the related polypeptides.

**Table 16. The results of the tyrosinase-mediated conjugation of single (multiple)-site mutated antibodies to polypeptides**

| Antibody No. | Polypeptide | Calculated increase in molecular weight Da (+1 Drug) | Calculated DAR | DAR value (calculated by MS) |
|---|---|---|---|---|
| c-met H10 | P-1 | 1813.96 | 2 | 2.00 |
| c-met L15 | P-1 | 1813.96 | 2 | 2.00 |
| ADA mut | P-2 | 1531.72 | 4 | 4.12 |

### Example 19. Preparation of Antibody-Peptide Conjugate Using VsTYR core

In this example, a conjugate of the ADA mut antibody of Example 18 and the P-2 polypeptide (CBP-ADA-mut) was prepared. The specific conjugation conditions and LC-MS detection were the same as those in Example 7. Meanwhile, the DAR value was calculated. The conjugation reaction mixture was purified using HiLoad 16/600 Superdex 200 pg (120 mL). The column was equilibrated with a PBS 5.5 buffer, and elution was performed. The peak of the target antibody conjugate was collected, and the enzyme and small molecule were removed. FIG. 5 shows a typical chromatogram of purification and collection. According to SEC-HPLC analysis, the purity of the collected antibody-peptide conjugate was >95%.

### Example 20. Assay for In Vitro Binding Activity of Antibody-Peptide Conjugate

The prepared antibody-peptide conjugate CBP-ADA-mut in Example 19 was subjected to an *in vitro* binding activity test. The antigen-binding sequence of the antibody end of CBP-ADA-mut is adalimumab, which can bind to TNFα, and the sequence of the polypeptide end is CBP (collagen-binding peptide), which can bind to collagen (Katsumata K, et al. Targeting inflammatory sites through collagen affinity enhances the therapeutic efficacy of anti-inflammatory antibodies. Sci Adv. 2019 Nov 6;5(11):eaay1971).

An ELISA was performed. Plates were coated with human TNFα antigen (Sino Biological, 10602 HNAE) and chicken type II collagen (Chondrex, #20012). After a washing-blocking-washing process, the test sample was added and incubated. After another wash, anti-human IgG, HRP (BETHYL, A80 319P) and anti-human IgG (Fab specific) peroxidase antibody (Sigma, A0293) were separately added as secondary antibodies. After washing, the TMB chromogenic solution was added. After 5-10 min of incubation, the stop solution was added, and OD450 values were measured.

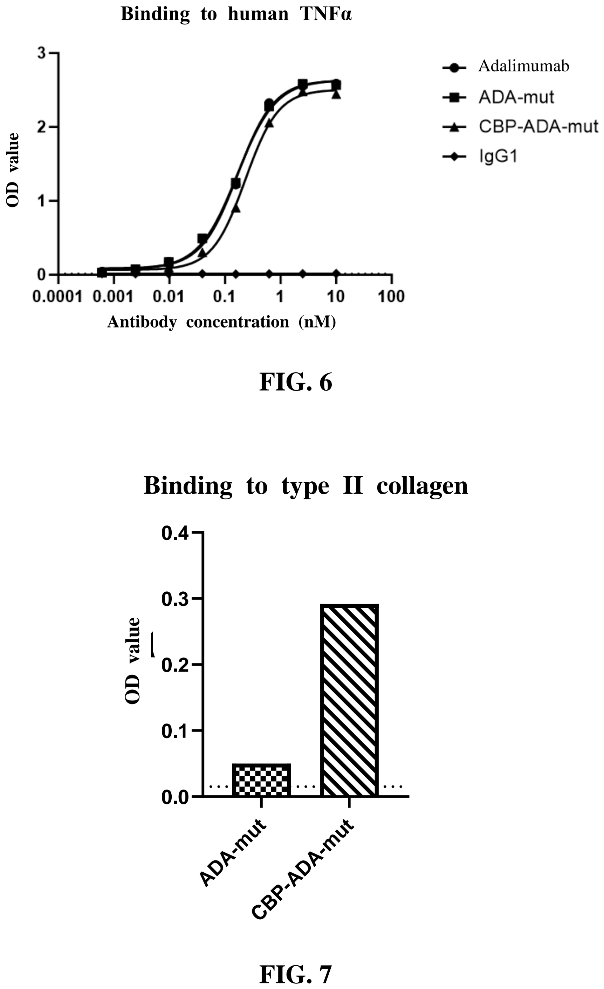

FIG.6 shows that CBP-ADA-mut was able to bind to TNFα and was comparable to ADA mut and adalimumab in binding activity. FIG. 7 shows that the antibody-peptide conjugate CBP-ADA-mut was able to bind to type II collagen via the conjugated CBP polypeptide, while ADA mut, a monoclonal antibody not conjugated to the CBP polypeptide, was not able to bind to type II collagen and thus did not have the corresponding biological function.

### Example 21. Assay for In Vitro Killing Activity of Antibody-Drug Conjugates

With reference to the conjugation conditions in Example 7, the expressed antibody TROP2 mut in Example 15 was conjugated to the synthesized L-1 small molecule in Example 3. According to LC-MS calculations, the DAR value of the resulting product ADC-1 was 4.00. Preparative purification was performed by a means similar to that in Example 19. The prepared ADC-1 was subjected to an *in vitro* cell experiment. Meanwhile, ADC-2 (interchain cys conjugation) was prepared as a positive control, and isotype IgG1 DAR4 (interchain cys conjugation) was prepared as a negative control. ADC-2 has the following structure:

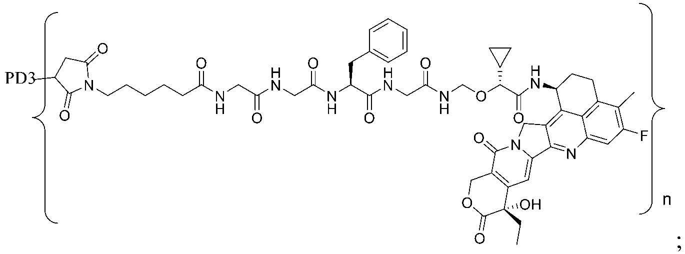

ADC-2 (n = 4.0) was prepared with reference to WO2021147993A1. The heavy and light chain sequences of the PD3 antibody (a TROP2 antibody) are set forth in SEQ ID NOs: 44 and 45 in the present disclosure. hIgG1 DAR4 differs from ADC-2 in that the VH and VL of the antibody end are the VH and VL of an unrelated antibody.

The killing ability of ADC-1 against cells with different TROP2 expression levels was verified by *in vitro* cell killing. A431 cells (with high expression of TROP) were cultured in a DMEM culture medium containing 10% FBS and passaged every three days in a passage ratio of 1:3 to 1:8. OVCAR-3 cells (with moderate expression of TROP) were cultured in an RPMI-1640 culture medium containing 10% FBS and passaged every three days in a passage ratio of 1:2 to 1:4. HEK-293 cells were cultured in a DMEM culture medium containing 10% FBS and passaged every three days in a passage ratio of 1:6 to 1:10. During passages, the culture medium was removed using a pipette; the cells were washed with PBS and then digested with 2 mL of 0.25% trypsin at 37 °C for 5 min; then a fresh culture medium was added to stop the digestion, and the cells were resuspended. 50 µL of the cell suspension was added to a 96-well cell culture plate, with each well containing 2 × 10³ cells. The culture medium was DMEM with 10% FBS. Only PBS buffer was added to the periphery of the 96-well plate. The culture plate was incubated overnight in an incubator (37 °C, 5% CO₂).

The next day, the test ADC drugs were prepared to an initial concentration of 200 nM and then serially diluted with the complete culture medium in a 1:3 ratio to obtain 9 concentration points, and an additional 0 concentration point was set. The dilutions (50 µL) were added to the above cell plate, and the plate was incubated in an incubator (37 °C, 5% CO2) for 6 days. 100 µL of Cell Titer-Glo reagent was added to each well of the 96-well cell culture plate. The plate was placed in a dark place at room temperature for 10-15 min. The chemiluminescence signals were measured using a PE Envision instrument, and the data were processed using GraphPad software. Plotting was performed with the ADC or toxin concentration as the X-axis and the cell killing rate as the Y-axis.

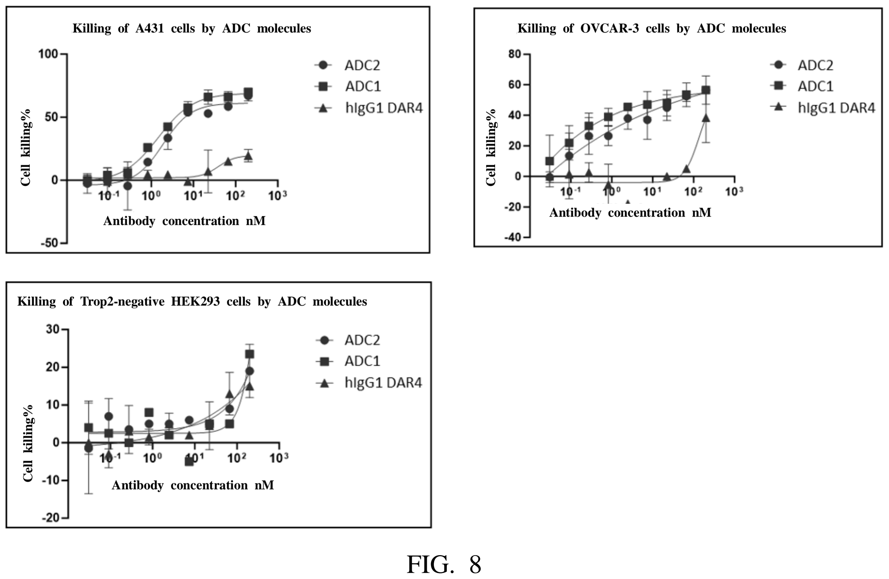

The results show that against A431 cells (with high expression of TROP2) and OVCAR-3 cells (with moderate expression), the killing ability of ADC-1 was comparable to that of ADC-2; there was no killing of TROP2-negative HEK-293 cells, and non-specific killing occurred only at high concentrations (FIG. 8). The data are shown in Table 17.

**Table 17. In vitro cell killing results for ADCs**

| | | | | | | |
|---|---|---|---|---|---|---|
| | A431 cells | | OVCAR-3 cells | | HEK-293 cells | |
| TROP2 expression level | High expression | | Moderate expression | | Low expression | |

| ADC No. | EC₅₀ (nM) | Maximum killing (%) | EC₅₀ (nM) | Maximum killing (%) | EC₅₀ (nM) | Maximum killing (%) |
|---|---|---|---|---|---|---|
| ADC-1 | 1.47 | 70.5 | / | 56.5 | / | 23.5 |
| ADC-2 | 1.91 | 67 | / | 56.5 | / | 19 |
| hIgG1 DAR4 | 38.91 | 19.5 | / | 38.5 | / | 13 |

| | | | | | | |
|---|---|---|---|---|---|---|
| Both ADC-1 and ADC-2 have a DAR of 4. | | | | | | |

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

Sequences of the present disclosure:
> Her wt HC (SEQ ID NO: 1) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> Her wt LC (SEQ ID NO: 2) 
DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKH
KVYACEVTHQGLSSPVTKSFNRGEC
> Her H9 HC (SEQ ID NO: 3) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSYSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> Her H10 HC (SEQ ID NO: 4) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
 
STKGPSVFPLAPSSKSTSYGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> Her **H14** HC (SEQ ID NO: 5) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> **Her H19** HC (SEQ ID NO: 6) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKYHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> Her H30 HC (SEQ ID NO: 7) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALYAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> Her H33 HC (SEQ ID NO: 8) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
 
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGYPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> Her H53 HC (SEQ ID NO: 9) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYS
LSSVVTVPSSYLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> Her H26 HC (SEQ ID NO: 10) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQFGYTFRVVS
VLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> Her L15 LC (SEQ ID NO: 11) 
DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKH
KVYACEVTHQGLYSPVTKSFNRGEC
> 0102 wt HC (SEQ ID NO: 12) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREEMTKNQVSLTCLVK
 
GFYPSDIAVEWESNGQPENNYKTTPPVLDSDGSFFLYSKLTVDKSRWQQGNVFSCSVMHEA
LHNHYTQKSLSLSPGK
> 0102 wt LC (SEQ ID NO: 13) 
DVVMTQSPLSLPVTPGEPASISCKSTQSLLYSDGETYLNWLLQKPGQSPQRLIYLVSKLDSGV
PDRFSGSGSGTDFTLKISRVEAEDVGVYYCWQGSHFPQTFGGGTKVEIKRTVAAPSVFIFPPS
DEQLKSGTASVVCLLNNFYPREAKVQWKVDNALQSGNSQESVTEQDSKDSTYSLSSTLTLS
KADYEKHKVYACEVTHQGLSSPVTKSFNRGEC
> 0102 H9 HC (SEQ ID NO: 14) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPSSKSYSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFSCSVMHEA
LHNHYTQKSLSLSPGK
> 0102 **H10** HC (SEQ ID NO: 15) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPSSKSTSYGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFSCSVMHEA
LHNHYTQKSLSLSPGK
> 0102 **H19** HC (SEQ ID NO: 16) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKYHTCPPCPAPELLGGPSVFLFPPKPKD
TLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFSCSVMHEA
LHNHYTQKSLSLSPGK
> 0102 H30 HC (SEQ ID NO: 17) EVQLVESGGGLVKPGGSLRLSCAASGFTFSTYAMSWVRQAPEKRLEWVATISDSDYYADNV 
KGRFTISRDNAKNSLYLQMNSLRAEDTAVYYCVRDDWDGFDYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVL
HQDWLNGKEYKCKVSNKALYAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFSCSVMHEA
LHNHYTQKSLSLSPGK
> 0102 H53 HC (SEQ ID NO: 18) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPSSKSTSGGTAALGCLVKDYFPEPVTVSWNSGALTSGVHTFPAVLQSSGLYSLSSVV
TVPSSYLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFSCSVMHEA
LHNHYTQKSLSLSPGK
> 0102 H26 HC (SEQ ID NO: 19) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQFGYTFRVVSVLTVLH
QDWLNGKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSVMHEAL
HNHYTQKSLSLSPGK
> 0102 L15 LC (SEQ ID NO: 20) 
DVVMTQSPLSLPVTPGEPASISCKSTQSLLYSDGETYLNWLLQKPGQSPQRLIYLVSKLDSGV
PDRFSGSGSGTDFTLKISRVEAEDVGVYYCWQGSHFPQTFGGGTKVEIKRTVAAPSVFIFPPS
DEQLKSGTASVVCLLNNFYPREAKVQWKVDNALQSGNSQESVTEQDSKDSTYSLSSTLTLS
KADYEKHKVYACEVTHQGLYSPVTKSFNRGEC
> megaTYR (SEQ ID NO: 21) 
MSNKYRVRKNVLHLTDTEKRDFVRTVLILKEKGIYDRYIAWHGAAGKFHTPPGSDRNAAH
MSSAFLPWHREYLLRFERDLQSINPEVTLPYWEWETDAQMQDPSQSQIWSADFMGGNGNPI
KDFIVDTGPFAAGRWTTIDEQGNPSGGLKRNFGATKEAPTLPTRDDVLNALKITQYDTPPWD
MTSQNSFRNQLEGFINGPQLHNRVHRWVGGQMGVVPTAPNDPVFFLHHANVDRIWAVWQII
HRNQNYQPMKNGPFGQNFRDPMYPWNTTPEDVMNHRKLGYVYDIELRKSKRSSLEHHHH
 HH
> VsTYR core (SEQ ID NO: 22) 
MAKYHRLNLQNPAAAPFLESYKKAITVMLQLPPSDARNWYRNAFIHTLDCPHGNWWFVV
WHRGYTGWFERTVRELSGDPNFAFPYWDWTALPQVPDSFFNGVLDPNNPAFIASYNEFYSQ
LSNPMSALWNSFSTAQLQQMRNRGFQSVNDVWQAVRDSPMFFPRGRARTLTRQNPGFDAT
TRRAVSIGTIRNALAPTDFITFGSGKTANHSESATQGILESQPHNNVHNNIGGFMQDLLSPTDP
VFFAHHSNIDRLWDVWTRKQQRLGLPTLPTGANLPLWANEPFLFFIGPDGKPVAKNKAGDY
ATIGDFEYNYEPGSGEAVIPAASRPGEMNNKHHHHHH
> VsTYR sp (SEQ ID NO: 23) 
MRGSHHHHHHGMASMTGGQQMGRDLYDDDDKDPSSRSAAGTMAKYHRLNLQNPAAAPF
LESYKKAITVMLQLPPSDARNWYRNAFIHTLDCPHGNWWFVVWHRGYTGWFERTVRELS
GDPNFAFPYWDWTALPQVPDSFFNGVLDPNNPAFIASYNEFYSQLSNPMSALWNSFSTAQLQ
QMRNRGFQSVNDVWQAVRDSPMFFPRGRARTLTRQNPGFDATTRRAVSIGTIRNALAPTDFI
TFGSGKTANHSESATQGILESQPHNNVHNNIGGFMQDLLSPTDPVFFAHHSNIDRLWDVWTR
KQQRLGLPTLPTGANLPLWANEPFLFFIGPDGKPVAKNKAGDYATIGDFEYNYEPGSGEAV
> **Her HH3** HC (SEQ ID NO: 24) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSYSGGTAALGCLVKDYFPEPVTVSWNSGALTSGVHTFPAVLQSSGLYS
LSSVVTVPSSSLGTQTYICNVNHKPSNTKVDKKVEPKSCDKYHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALYAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> Her **HH4** HC (SEQ ID NO: 25) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSYSGGTAALGCLVKDYFPEPVTVSWNSGALTSGVHTFPAVLQSSGLYS
LSSVVTVPSSYLGTQTYICNVNHKPSNTKVDKKVEPKSCDKYHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALYAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> **Her HH8** HC (SEQ ID NO: 26) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
 
STKGPSVFPLAPSSKSYSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALYAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> **Her HH9** HC (SEQ ID NO: 27) 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKYHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALYAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGK
> 0102 H73 HC (SEQ ID NO: 28) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPCSRSTSESTAALGCLVKDYFPEPVTVSWNSGALTSGVHTFPAVLQSSGLYSLSSVV
TVPSSNFGTQTYTCNVDHKPSNTKVDKTVERKCCVECPPCPAPPVAGPSVFLFPPKPKDTLM
ISRTPEVTCVVVDVSHEDPEVQFNWYVDGVEVHNAKTKPREEQFNSTFRVVSVLTVVHQD
WLNGKEYKCKVSNKGLPAPIEKTISKTKGQPREPQVYTLPPSREEMTKNQVSLTCLVKGFYP
SDISVEWESNGQPENNYKTTPPMLDSDGSFFLYSKLTVDKSRWQQGNVFSCSVMHEALHNH
YTQKSLSLSPGK
> 0102 H74 HC (SEQ ID NO: 29) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPCSRSTSGGTAALGCLVKDYFPEPVTVSWNSGALTSGVHTFPAVLQSSGLYSLSSVV
TVPSSSLGTQTYTCNVNHKPSNTKVDKRVELKTPLGDTTHTCPRCPEPKSCDTPPPCPRCPEP
KSCDTPPPCPRCPEPKSCDTPPPCPRCPAPELLGGPSVFLFPPKPKDTLMISRTPEVTCVVVDV
SHEDPEVQFKWYVDGVEVHNAKTKPREEQFNSTFRVVSVLTVLHQDWLNGKEYKCKVSN
KALPAPIEKTISKTKGQPREPQVYTLPPSREEMTKNQVSLTCLVKGFYPSDIAVEWESSGQPE
NNYNTTPPMLDSDGSFFLYSKLTVDKSRWQQGNIFSCSVMHEALHNRFTQKSLSLSPGK
> 0102 **H40** HC (SEQ ID NO: 30) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
 
SVFPLAPCSRSTSESTAALGCLVKDYFPEPVTVSWNSGALTSGVHTFPAVLQSSGLYSLSSVV
TVPSSSLGTKTYTCNVDHKPSNTKVDKRVESKYGPPCPPCPAPEFLGGPSVFLFPPKPKDTLM
ISRTPEVTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLTCLVKGFYP
SDIAVEWESNGQPENNYKTTPPVLDSDGSFFLYSRLTVDKSRWQEGNVFSCSVMHEALHNH
YTQKSLSLSLGK
> 0102 H75 HC (SEQ ID NO: 31) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPCSRSYSESTAALGCLVKDYFPEPVTVSWNSGALTSGVHTFPAVLQSSGLYSLSSVV
TVPSSNFGTQTYTCNVDHKPSNTKVDKTVERKCCVECPPCPAPPVAGPSVFLFPPKPKDTLM
ISRTPEVTCVVVDVSHEDPEVQFNWYVDGVEVHNAKTKPREEQFNSTFRVVSVLTVVHQD
WLNGKEYKCKVSNKGLPAPIEKTISKTKGQPREPQVYTLPPSREEMTKNQVSLTCLVKGFYP
SDISVEWESNGQPENNYKTTPPMLDSDGSFFLYSKLTVDKSRWQQGNVFSCSVMHEALHNH
YTQKSLSLSPGK
> 0102 H76 HC (SEQ ID NO: 32) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPCSRSTSESTAALGCLVKDYFPEPVTVSWNSGALTSGVHTFPAVLQSSGLYSLSSVV
TVPSSYFGTQTYTCNVDHKPSNTKVDKTVERKCCVECPPCPAPPVAGPSVFLFPPKPKDTLM
ISRTPEVTCVVVDVSHEDPEVQFNWYVDGVEVHNAKTKPREEQFNSTFRVVSVLTVVHQD
WLNGKEYKCKVSNKGLPAPIEKTISKTKGQPREPQVYTLPPSREEMTKNQVSLTCLVKGFYP
SDISVEWESNGQPENNYKTTPPMLDSDGSFFLYSKLTVDKSRWQQGNVFSCSVMHEALHNH
YTQKSLSLSPGK
> 0102 H77 HC (SEQ ID NO: 33) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPCSRSTSGGTAALGCLVKDYFPEPVTVSWNSGALTSGVHTFPAVLQSSGLYSLSSVV
TVPSSSLGTQTYTCNVNHKPSNTKVDKRVELKTPLGDTTHTCPRCPEPKSCDTPPPCPRCPEP
KSCDTPPPCPRCPEPKSCDTPPPCPRCPAPELLGGPSVFLFPPKPKDTLMISRTPEVTCVVVDV
SHEDPEVQFKWYVDGVEVHNAKTKPREEQFNSTFRVVSVLTVLHQDWLNGKEYKCKVSN
KALYAPIEKTISKTKGQPREPQVYTLPPSREEMTKNQVSLTCLVKGFYPSDIAVEWESSGQPE
NNYNTTPPMLDSDGSFFLYSKLTVDKSRWQQGNIFSCSVMHEALHNRFTQKSLSLSPGK
> 0102 H78 HC (SEQ ID NO: 34) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
 
SVFPLAPCSRSYSESTAALGCLVKDYFPEPVTVSWNSGALTSGVHTFPAVLQSSGLYSLSSVV
TVPSSSLGTKTYTCNVDHKPSNTKVDKRVESKYGPPCPPCPAPEFLGGPSVFLFPPKPKDTLM
ISRTPEVTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLTCLVKGFYP
SDIAVEWESNGQPENNYKTTPPVLDSDGSFFLYSRLTVDKSRWQEGNVFSCSVMHEALHNH
YTQKSLSLSLGK
> 0102 H79 HC (SEQ ID NO: 35) 
EVQLVESGGGLVKPGGSLRLSCAASGFTFSTYAMSWVRQAPEKRLEWVATISDSDYYADNV
KGRFTISRDNAKNSLYLQMNSLRAEDTAVYYCVRDDWDGDFDYWGQGTLVTVSSASTKGP
SVFPLAPCSRSTSESTAALGCLVKDYFPEPVTVSWNSGALTSGVHTFPAVLQSSGLYSLSSVV
TVPSSSLGTKTYTCNVDHKPSNTKVDKRVESKYGPPCPPCPAPEFLGGPSVFLFPPKPKDTLM
ISRTPEVTCVVVDVSQEDPEVQFNWYVDGVEVHNAKTKPREEQFNSTYRVVSVLTVLHQD
WLNGKEYKCKVSNKGLYSSIEKTISKAKGQPREPQVYTLPPSQEEMTKNQVSLTCLVKGFYP
SDIAVEWESNGQPENNYKTTPPVLDSDGSFFLYSRLTVDKSRWQEGNVFSCSVMHEALHNH
YTQKSLSLSLGK
> c-met H10 HC (SEQ ID NO: 36) 
QVQLVESGGGVVQPGRSLRLSCAASGFSLSNYGVHWVRQAPGKGLEWLAVIWSGGSTNYA
AAFVSRLTISKDNSKNTVYLQMNSLRAEDTAVYYCARNHDNPYNYAMDYWGQGTTVTVSS
ASTKGPSVFPLAPSSKSTSYGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFSCS
VMHEALHNHYTQKSLSLSPGK
> c-met H10 LC (SEQ ID NO: 37) 
DIVLTQSPDSLAVSLGERATINCRADKSVSTSTYNYLHWYQQKPGQPPKLLIYLASNLASGVP
DRFSGSGSGTDFTLTISSLQAEDVAVYYCQHSRDLPPTFGQGTKLEIKRTVAAPSVFIFPPSDE
QLKSGTASVVCLLNNFYPREAKVQWKVDNALQSGNSQESVTEQDSKDSTYSLSSTLTLSKA
DYEKHKVYACEVTHQGLSSPVTKSFNRGEC
> c-met L15 HC (SEQ ID NO: 38) 
QVQLVESGGGVVQPGRSLRLSCAASGFSLSNYGVHWVRQAPGKGLEWLAVIWSGGSTNYA
AAFVSRLTISKDNSKNTVYLQMNSLRAEDTAVYYCARNHDNPYNYAMDYWGQGTTVTVSS
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREEMTKNQVS
 
LTCLVKGFYPSDIAVEWESNGQPENNYKTTPPVLDSDGSFFLYSKLTVDKSRWQQGNVFSCS
VMHEALHNHYTQKSLSLSPGK
> c-met L15 LC (SEQ ID NO: 39) 
DIVLTQSPDSLAVSLGERATINCRADKSVSTSTYNYLHWYQQKPGQPPKLLIYLASNLASGVP
DRFSGSGSGTDFTLTISSLQAEDVAVYYCQHSRDLPPTFGQGTKLEIK
RTVAAPSVFIFPPSDEQLKSGTASVVCLLNNFYPREAKVQWKVDNALQSGNSQESVTEQDSK
DSTYSLSSTLTLSKADYEKHKVYACEVTHQGLYSPVTKSFNRGEC
> ADA **mut** HC (SEQ ID NO: 40) 
DIQMTQSPSSLSASVGDRVTITCRASQGIRNYLAWYQQKPGKAPKLLIYAASTLQSGVPSRFS
GSGSGTDFTLTISSLQPEDVATYYCQRYNRAPYTFGQGTKVEIKASTKGPSVFPLAPSSKSTSG
GTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEYKC
KVSNKALYAPIEKTISKAKGQPREPQVYTLPPSREEMTKNQVSLTCLVKGFYPSDIAVEWESN
GQPENNYKTTPPVLDSDGSFFLYSKLTVDKSRWQQGNVFSCSVMHEALHNHYTQKSLSLSP
GK
> ADA **mut** LC (SEQ ID NO: 41) 
DAVMTQTPLSLSVTPGQPASISCRSSQSLERSTGNTFLNWYLQKPGQSPQLLIYRVSTRFSGV
PDRFSGSGSGTDFTLKISRVEAEDVGVYYCLQLTHVPLTFGGGTKVEIKRTVAAPSVFIFPPSD
EQLKSGTASVVCLLNNFYPREAKVQWKVDNALQSGNSQESVTEQDSKDSTYSLSSTLTLSK
ADYEKHKVYACEVTHQGLYSPVTKSFNRGEC
> trop2 mut HC (SEQ ID NO: 42) 
EVQLVESGGGLVQPGRSLRLSCAASGFTFDDYAMHWVRQAPGKGLEWVSAITWNSGHIDY
ADSVEGRFTISRDNAKNSLYLQMNSLRAEDTAVYYCAKVSYLSTASSLDYWGQGTLVTVSS
ASTKGPSVFPLAPSSKSTSYGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFSCS
VMHEALHNHYTQKSLSLSPGK
> trop2 **mut** LC (SEQ ID NO: 43) 
DIQMTQSPSSLSASVGDRVTITCRASQGIRNYLAWYQQKPGKAPKLLIYAASTLQSGVPSRFS
GSGSGTDFTLTISSLQPEDVATYYCQRYNRAPYTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLYSPVTKSFNRGEC
> ADC-2 **HC** (SEQ ID NO: 44) 
EVQLVESGGGLVQPGRSLRLSCAASGFTFDDYAMHWVRQAPGKGLEWVSAITWNSGHIDY
ADSVEGRFTISRDNAKNSLYLQMNSLRAEDTAVYYCAKVSYLSTASSLDYWGQGTLVTVSS
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVV
SVLTVLHQDWLNGKEYKCKVSNKALYAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFSCS
VMHEALHNHYTQKSLSLSPGK
> ADC-2 **LC** (SEQ ID NO: 45) 
DIQMTQSPSSLSASVGDRVTITCRASQGIRNYLAWYQQKPGKAPKLLIYAASTLQSGVPSRFS
GSGSGTDFTLTISSLQPEDVATYYCQRYNRAPYTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC
> CH1 (SEQ ID NO: 46) 
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKV
> Hinge (SEQ ID NO: 47)
   EPKSCDKTHTCPPCP
> CH2 (SEQ ID NO: 48) 
APELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAK
> CH3 (SEQ ID NO: 49) 
GQPREPQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK
> Cκ (SEQ ID NO: 50)
> **Her HH13** LC (SEQ ID NO: 67) 
DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKH
KVYACEVTHQGLSSPVTKSFNRGECGGGGY.
   YGGGG (SEQ ID NO: 76);
   YGGGGS (SEQ ID NO: 77);
   GGGGY (SEQ ID NO: 78);
   SGGGGY (SEQ ID NO: 79);
   P-1: EQKLISEEDLGGSC (SEQ ID NO: 80);
   P-2: LRELHLNNNC (SEQ ID NO: 81).
> CH1 T135Y (SEQ ID NO: 82) 
ASTKGPSVFPLAPSSKSYSGGTAALGCLVKDYFPEPVTVSWNSGALTSGVHTFPAVLQSSGLY
SLSSVVTVPSSSLGTQTYICNVNHKPSNTKVDKKV
> **CH1** G137Y (SEQ ID NO: 83) 
ASTKGPSVFPLAPSSKSTSYGTAALGCLVKDYFPEPVTVSWNSGALTSGVHTFPAVLQSSGLY
SLSSVVTVPSSSLGTQTYICNVNHKPSNTKVDKKV
> CH1 S192Y (SEQ ID NO: 84) 
ASTKGPSVFPLAPSSKSTSGGTAALGCLVKDYFPEPVTVSWNSGALTSGVHTFPAVLQSSGLY
SLSSVVTVPSSYLGTQTYICNVNHKPSNTKVDKKV
> Hinge T223Y (SEQ ID NO: 85)
   EPKSCDKYHTCPPCP
> CH2 P329Y (SEQ ID NO: 86) 
APELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALYAPIEKTISKAK
> Cκ S202Y (SEQ ID NO: 87) 
RTVAAPSVFIFPPSDEQLKSGTASVVCLLNNFYPREAKVQWKVDNALQSGNSQESVTEQDSK
DSTYSLSSTLTLSKADYEKHKVYACEVTHQGLYSPVTKSFNRGEC
> **CH1** T135Y/S192Y (SEQ ID NO: 88) 
ASTKGPSVFPLAPSSKSYSGGTAALGCLVKDYFPEPVTVSWNSGALTSGVHTFPAVLQSSGLY
SLSSVVTVPSSYLGTQTYICNVNHKPSNTKVDKKV
> CH1 G137Y/S192Y (SEQ ID NO: 89) 
ASTKGPSVFPLAPSSKSTSYGTAALGCLVKDYFPEPVTVSWNSGALTSGVHTFPAVLQSSGLY
SLSSVVTVPSSYLGTQTYICNVNHKPSNTKVDKKV
> CH1 T135Y/G137Y (SEQ ID NO: 90)

ASTKGPSVFPLAPSSKSYSYGTAALGCLVKDYFPEPVTVSWNSGALTSGVHTFPAVLQSSGLY
SLSSVVTVPSSSLGTQTYICNVNHKPSNTKVDKKV

## Claims

1. A conjugate, comprising a structure represented by formula I or a pharmaceutically acceptable salt thereof:
Pc-[L-(D)ₓ]_{y} I,
wherein:
Pc is a protein comprising an antibody constant region or a fragment thereof;
L is a linker covalently linking Pc to D, and L is linked to an engineered tyrosine (Tyr) residue in the antibody constant region or the fragment thereof; the engineered tyrosine residue is not located near a N-glycosylation site, and Pc does not require removal/reduction of glycosylation modification before reacting with tyrosinase;
D is a payload;
x is 1 to 20;
y is 1 to 20;
preferably, the N-glycosylation site is 297N as defined according to the EU numbering scheme;
preferably, the phenol moiety of the engineered tyrosine residue is oxidizable by monomeric tyrosinase to an o-quinone moiety but is not oxidizable by multimeric tyrosinase to an o-quinone moiety.

2. The conjugate according to claim 1, wherein the monomeric tyrosinase is derived from *Bacillus megaterium* or *Verrucomicrobium spinosum;* the multimeric tyrosinase is a tetrameric enzyme, and the tetrameric enzyme is preferably derived from a mushroom.

3. The conjugate according to claim 1 or 2, wherein the engineered tyrosine residue is not located between the amino acid residues at positions 292 and 302 of a heavy chain HC.

4. A conjugate, comprising a structure represented by formula I or a pharmaceutically acceptable salt thereof:
Pc-[L-(D)ₓ]_{y} I,
wherein:
Pc is a protein comprising an antibody constant region or a fragment thereof;
L is a linker covalently linking Pc to D, and L is linked to an engineered tyrosine residue in the antibody constant region or the fragment thereof; the engineered tyrosine residue is located at any one or any combination of positions 135, 137, 192, 223, and 329 of a heavy chain HC and position 202 of a light chain LC of Pc, wherein the amino acid residue positions are defined according to the EU numbering scheme;
D is a payload;
x is 1 to 20;
y is 1 to 20.

5. The conjugate according to any one of claims 1-4, wherein the engineered tyrosine residue in Pc is located at any one or any combination of positions 135, 137, 192, 223, and 329 of the heavy chain HC and position 202 of the light chain LC of Pc and is preferably selected from the group consisting of the following combinations:
202Y of the LC and 135Y of the HC;
202Y of the LC and 137Y of the HC;
202Y of the LC and 192Y of the HC;
202Y of the LC and 223Y of the HC;
202Y of the LC and 329Y of the HC;
135Y/137Y of the HC;
135Y/192Y of the HC;
135Y/223Y of the HC;
135Y/329Y of the HC;
137Y/192Y of the HC;
137Y/223Y of the HC;
137Y/329Y of the HC;
192Y/223Y of the HC;
192Y/329Y of the HC;
223Y/329Y of the HC;
135Y/137Y of the HC and 202Y of the LC;
135Y/192Y of the HC and 202Y of the LC;
135Y/223Y of the HC and 202Y of the LC;
135Y/329Y of the HC and 202Y of the LC;
137Y/192Y of the HC and 202Y of the LC;
137Y/223Y of the HC and 202Y of the LC;
137Y/329Y of the HC and 202Y of the LC;
192Y/223Y of the HC and 202Y of the LC;
192Y/329Y of the HC and 202Y of the LC;
223Y/329Y of the HC and 202Y of the LC;
135Y/137Y/192Y of the HC;
135Y/137Y/223Y of the HC;
135Y/137Y/329Y of the HC;
135Y/137Y/192Y of the HC and 202Y of the LC;
135Y/137Y/223Y of the HC and 202Y of the LC;
135Y/137Y/329Y of the HC and 202Y of the LC;
135Y/192Y/223Y of the HC;
135Y/192Y/329Y of the HC;
135Y/192Y/223Y of the HC and 202Y of the LC;
135Y/192Y/329Y of the HC and 202Y of the LC;
137Y/192Y/223Y of the HC;
137Y/192Y/329Y of the HC;
137Y/192Y/223Y of the HC and 202Y of the LC;
137Y/192Y/329Y of the HC and 202Y of the LC;
137Y/223Y/329Y of the HC;
137Y/223Y/329Y of the HC and 202Y of the LC;
192Y/223Y/329Y of the HC;
192Y/223Y/329Y of the HC and 202Y of the LC;
135Y/223Y/329Y of the HC; and
135Y/223Y/329Y of the HC and 202Y of the LC.

6. The conjugate according to any one of the preceding claims, wherein L and Pc form a structure represented by Za or Zb: 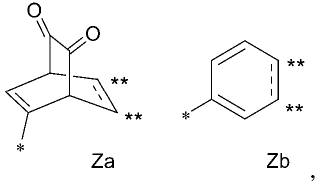
wherein * represents an end linked to Pc;
** represents an end linked to L;
---- is a single bond or a double bond.

7. The conjugate according to any one of the preceding claims, wherein an amino acid linked to L is the engineered tyrosine residue, and the phenol moiety of the engineered tyrosine residue is oxidized by tyrosinase to an o-quinone moiety.

8. The conjugate according to any one of the preceding claims, wherein L comprises a cleavable peptide moiety.

9. The conjugate according to any one of the preceding claims, wherein L comprises an amino acid unit, and the amino acid unit preferably comprises a peptide residue consisting of 2 to 7 amino acids selected from the group consisting of phenylalanine, glycine, valine, lysine, citrulline, serine, glutamic acid, and aspartic acid, and more preferably valine-citrulline (Val-Cit), alanine-alanine-asparagine (Ala-Ala-Asn), glycine-glycine-lysine (Gly-Gly-lys), valine-lysine (Val-lys), valine-alanine (Val-Ala), valine-phenylalanine (Val-Phe), or glycine-glycine-phenylalanine-glycine (Gly-Gly-Phe-Gly).

10. The conjugate according to any one of the preceding claims, wherein L comprises a spacer unit linked to D, and the spacer unit preferably comprises p-aminobenzyloxycarbonyl (PAB),

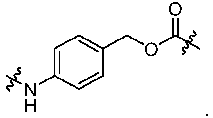

11. The conjugate according to any one of the preceding claims, wherein L comprises a spacer unit linked to D, and the spacer unit comprises a structure selected from the group consisting of the following:
-(CR¹R²)ₘ₁-O(CR¹R²)ₘ₂-CR³R⁴-C(O)-,
-(CR¹R²)ₘ₁NH-(CR¹R²)ₘ₂-CR³R⁴-C(O)-,
-(CR¹R²)ₘ₁O-CR³R⁴(CR¹R²)ₘ₂-,
-(CR¹R²)ₘ₁OCR³R⁴-C(O)-,
-(CR¹R²)ₘ₁-O-(CR¹R²)ₘ₂C(O)-, and -(CR¹R²)ₘ₁-S-(CR¹R²)ₘ₂-CR³R⁴-C(O)-,
wherein:
R¹ and R² are identical or different and are each independently selected from the group consisting of hydrogen, halogen, and alkyl;
R³ is selected from the group consisting of hydrogen, C₃₋₆ cycloalkylalkyl, and C₃₋₆ cycloalkyl;
R⁴ is selected from the group consisting of hydrogen, haloalkyl, and C₃₋₆ cycloalkyl,
preferably hydrogen;
or R³ and R⁴, together with the carbon atom to which they are attached, form C₃₋₆ cycloalkyl;
m1 and m2 are each independently selected from the group consisting of 0, 1, 2, and 3.

12. The conjugate according to claim 11, wherein the spacer unit comprises a structure selected from the group consisting of the following: -(CH₂)₃-C(O)-, -CH₂-O-CH₂-C(O)-, -(CH₂)₂-O-CH₂-C(O)-,

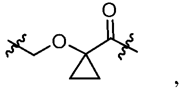

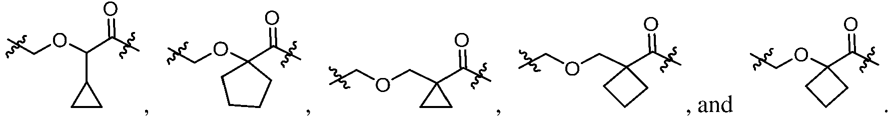

13. The conjugate according to any one of the preceding claims, wherein L comprises a stretcher unit (Str) covalently linked to Pc; the Str undergoes a [4+2] cycloaddition reaction with o-quinone; the o-quinone is obtained by oxidizing a tyrosine residue with tyrosinase.

14. The conjugate according to claim 13, wherein the Str comprises cycloalkenyl, cycloalkynyl, heterocycloalkenyl, or heterocycloalkynyl.

15. The conjugate according to claim 13 or 14, wherein the Str comprises a group selected from the group consisting of the following:

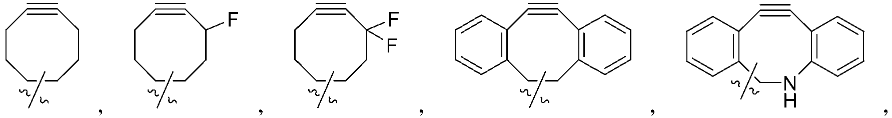

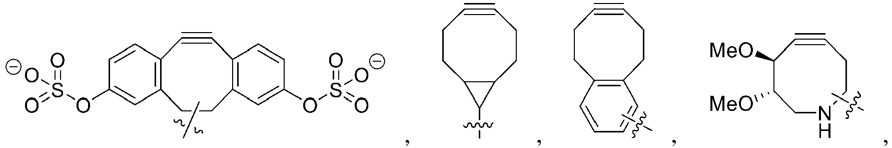

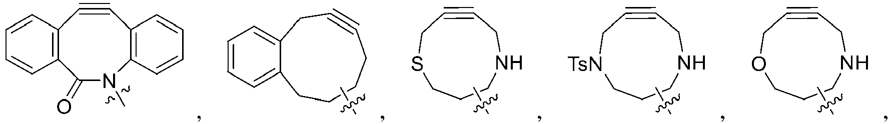

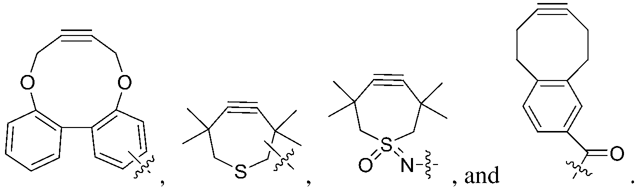

16. The conjugate according to claim 15, wherein the Str and Pc form the following structure: 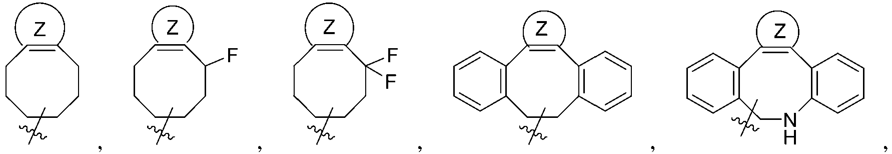 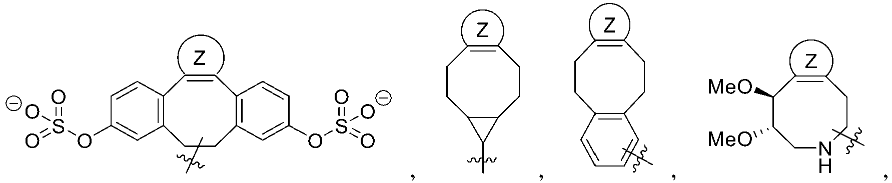 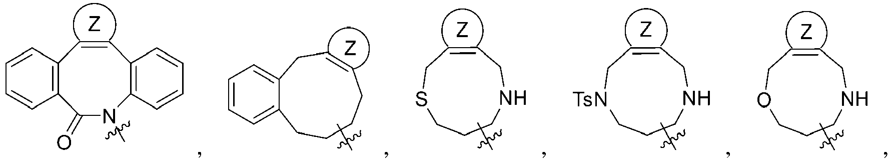 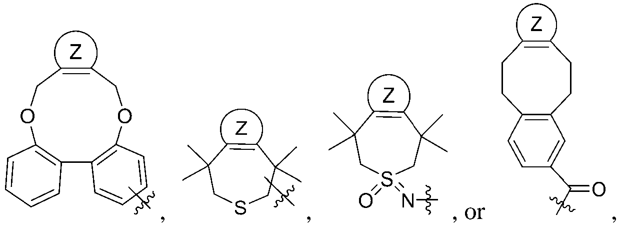 wherein ring Z is

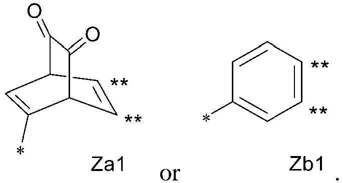

17. The conjugate according to claim 13 or 14, wherein the Str comprises a group selected from the group consisting of the following: 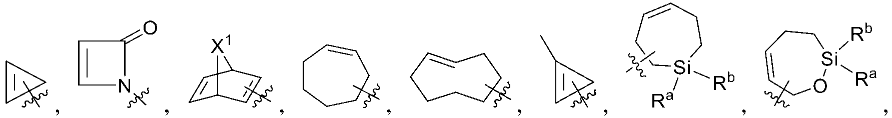 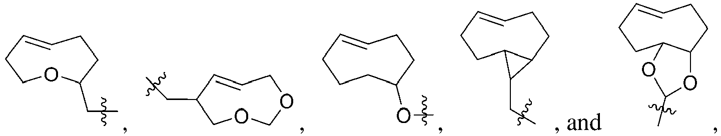
wherein R^{a} and R^{b} are each independently selected from the group consisting of hydrogen, C₁₋₆ alkyl, and aryl;
X¹ is selected from the group consisting of C₁₋₃ alkylene, NR^{c}, and O, and R^{c} is selected from the group consisting of hydrogen and C₁₋₆ alkyl.

18. The conjugate according to claim 17, wherein the Str and Pc form the following structure: 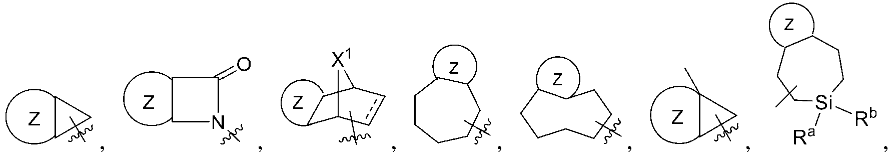 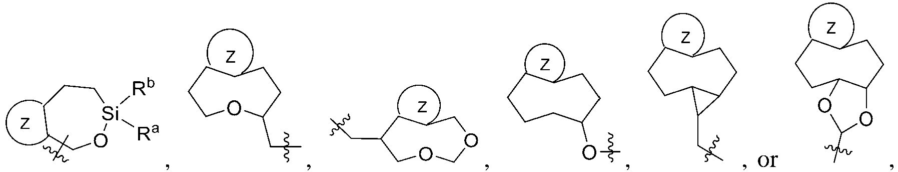
wherein R^{a}, R^{b}, and X¹ are as previously defined;
ring Z is

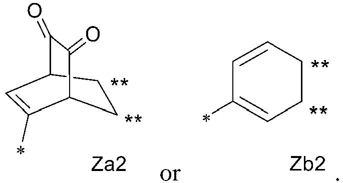

19. The conjugate according to any one of the preceding claims, wherein L-(D)ₓ is a chemical moiety represented by the following formula:
-Str-(Pep)-Sp-D
wherein Str is a stretcher unit covalently linking to Pc;
Sp is a spacer unit;
Pep is a cleavable peptide moiety.

20. The conjugate according to any one of the preceding claims, wherein L-(D)ₓ comprises the following chemical moiety: 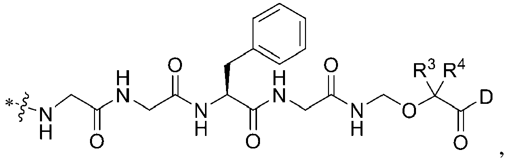 or 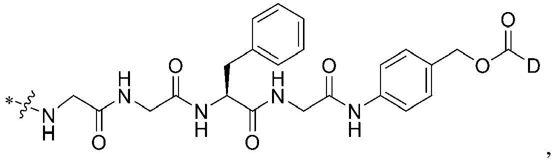 wherein:
R³ is selected from the group consisting of hydrogen, C₃₋₆ cycloalkylalkyl, and C₃₋₆ cycloalkyl, preferably hydrogen;
R⁴ is selected from the group consisting of hydrogen, haloalkyl, and C₃₋₆ cycloalkyl,
preferably hydrogen;
or R³ and R⁴, together with the carbon atom to which they are attached, form C₃₋₆ cycloalkyl;
* represents an end linked to Pc.

21. The conjugate according to any one of the preceding claims, wherein L-(D)ₓ is a chemical structure represented by the following formula: 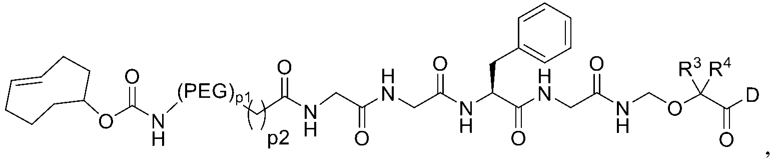 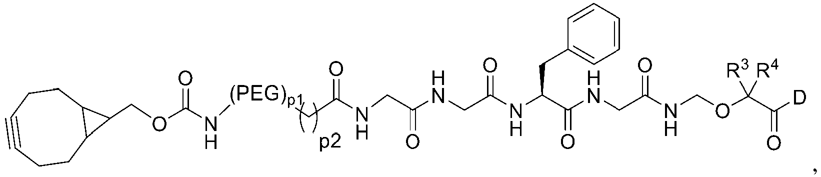 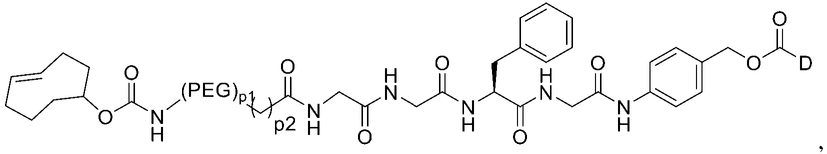 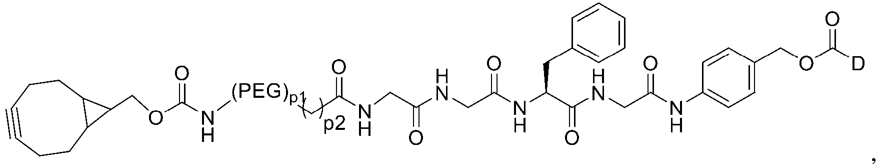 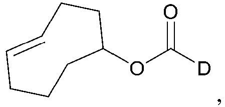 or 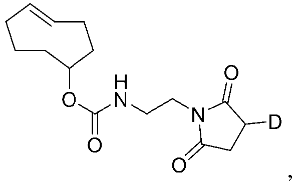
wherein R³ is selected from the group consisting of hydrogen, C₃₋₆ cycloalkylalkyl, and C₃₋₆ cycloalkyl, preferably hydrogen;
R⁴ is selected from the group consisting of hydrogen, haloalkyl, and C₃₋₆ cycloalkyl, preferably hydrogen;
or R³ and R⁴, together with the carbon atom to which they are attached, form C₃₋₆ cycloalkyl;
p1 is selected from the group consisting of 2, 4, 6, and 8;
p2 is selected from the group consisting of 0, 1, and 2.

22. The conjugate according to any one of the preceding claims, wherein the conjugate is represented by the following formula: 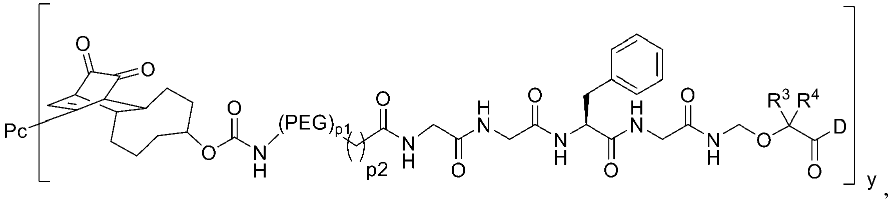 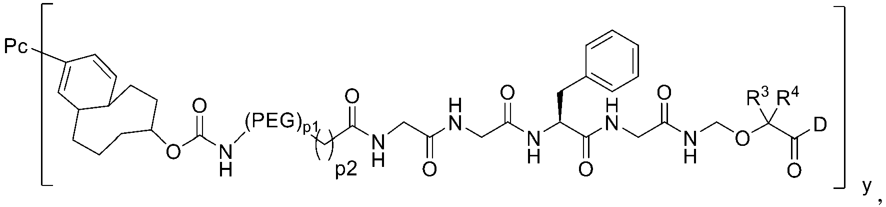 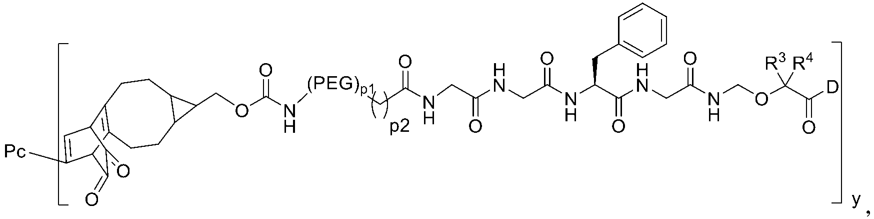 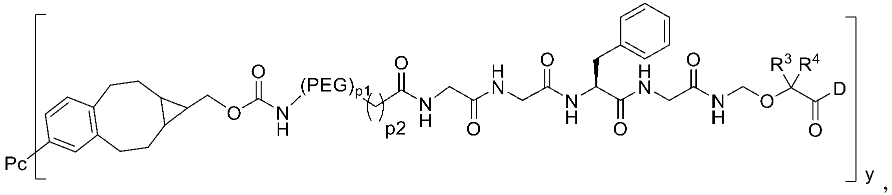 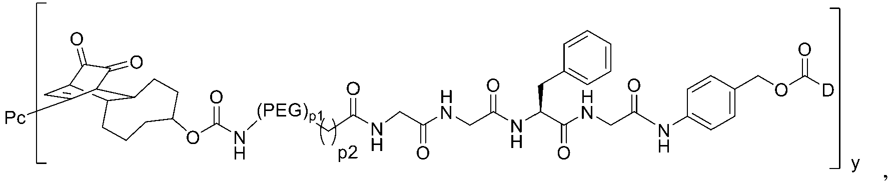 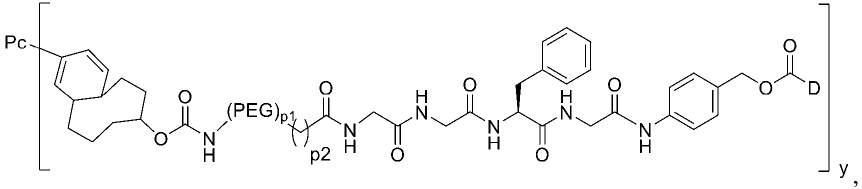 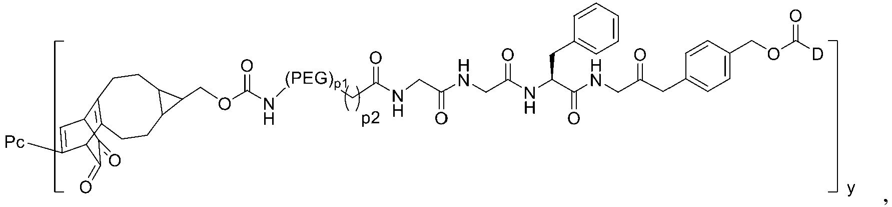 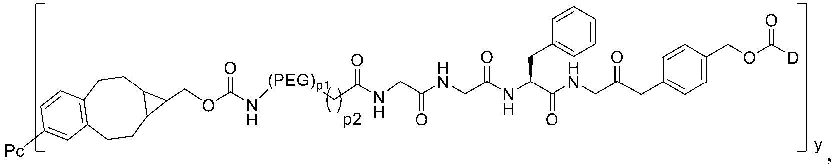 or 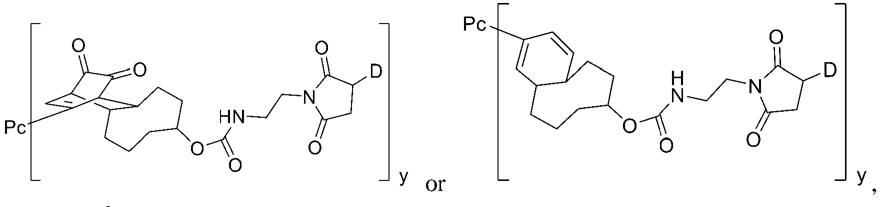 wherein R³ is selected from the group consisting of hydrogen, C₃₋₆ cycloalkylalkyl, and C₃₋₆ cycloalkyl, preferably hydrogen;
R⁴ is selected from the group consisting of hydrogen, haloalkyl, and C₃₋₆ cycloalkyl, preferably hydrogen;
or R³ and R⁴, together with the carbon atom to which they are attached, form C₃₋₆ cycloalkyl;
y is selected from the group consisting of 1 to 20 and may be an integer or a decimal; p1 is selected from the group consisting of 2, 4, 6, and 8;
p2 is selected from the group consisting of 0, 1, and 2.

23. The conjugate according to any one of the preceding claims, wherein the antibody constant region or the fragment thereof in Pc is derived from an IgG, IgA, IgM, IgD, or IgE constant region or a fragment thereof; the IgG is preferably IgG1, IgG2, IgG3, or IgG4, and more preferably IGHG1, IGHG2, IGHG3, or IGHG4.

24. The conjugate according to any one of the preceding claims, wherein the antibody constant region or the fragment thereof in Pc is a heavy chain constant region or a fragment thereof and/or a light chain constant region or a fragment thereof;
preferably, the heavy chain constant region is selected from the group consisting of any one or any combination of a C_{H}1 region, a hinge region, and a C_{H}2 region, and the light chain constant region is a Cκ region;
preferably, the heavy chain constant region comprises a C_{H}3 region.

25. The conjugate according to any one of the preceding claims, wherein the antibody constant region or the fragment thereof in Pc is glycosylated or non-glycosylated; preferably, the glycosylation is 297N glycosylation.

26. The conjugate according to any one of the claims, wherein Pc is selected from the group consisting of an antibody or an antigen-binding fragment thereof, an Fc region or a fragment thereof, and a fusion protein; the antibody or the antigen-binding fragment thereof is preferably a monospecific, bispecific, or multispecific antibody or an antigen-binding fragment thereof.

27. The conjugate according to claim 26, wherein Pc is an anti-tumor antibody or an antigen-binding fragment thereof; the antibody is preferably an anti-HER2 antibody, an anti-LIV-1 antibody, or an anti-TROP2 antibody.

28. The conjugate according to claim 26 or 27, wherein Pc is selected from the group consisting of a murine antibody, a chimeric antibody, a humanized antibody, and a fully human antibody or an antigen-binding fragment thereof.

29. The conjugate according to any one of the preceding claims, wherein Pc is a monomer or a multimer, and the multimer is preferably a dimer, a trimer, or a tetramer.

30. The conjugate according to any one of the preceding claims, wherein Pc comprises a polypeptide fragment at a N-terminus and/or a C-terminus, and the fragment comprises one or more tyrosine residues;
preferably, the fragment is linked to the N-terminus and/or the C-terminus of Pc, either directly or by a linker;
more preferably, the linker is a GS or polyG linker;
most preferably, the N-terminus of Pc comprises YGGGG or YGGGGS, and/or the C-terminus comprises GGGGY or SGGGGY.

31. The conjugate according to any one of the preceding claims, wherein D is a therapeutic agent or a diagnostic agent;
preferably, D is a radioactive agent, a cytotoxic agent, a nucleic acid, a polypeptide, a fluorophore, a fluorescent dye, a radionuclide, an enzyme, an antibiotic, a chelating agent, a lipid, or an antibody or an antigen-binding fragment thereof;
more preferably, D is exatecan or a derivative thereof or eribulin or a derivative thereof.

32. The conjugate according to any one of the preceding claims, wherein x is 1, and y is 1-8.

33. A protein, comprising an antibody constant region or a fragment thereof, wherein the antibody constant region or the fragment thereof comprises one or more engineered tyrosine residues, and the phenol moieties of the engineered tyrosine residues are oxidizable by monomeric tyrosinase to o-quinone moieties but are not oxidizable by multimeric tyrosinase to o-quinone moieties;
preferably, the monomeric tyrosinase is derived from *Bacillus megaterium* or *Verrucomicrobium spinosum;* the multimeric tyrosinase is a tetrameric enzyme, and the tetrameric enzyme is preferably derived from a mushroom.

34. The protein according to claim 33, wherein the engineered tyrosine residues are not located near a N-glycosylation site, and/or Pc does not require removal/reduction of glycosylation modification before reacting with tyrosinase;
preferably, the N-glycosylation site is 297N as defined according to the EU numbering scheme;
more preferably, the engineered tyrosine residues are not located between the amino acid residues at positions 292 and 302 of a heavy chain HC.

35. The protein according to claim 34, wherein the removal/reduction of glycosylation modification is selected from the group consisting of:
1) contacting the protein with an amidase to obtain a protein from which a glycan is removed, wherein the amidase is preferably PNGase F;
2) contacting the protein with an endoglycosidase such that the protein is modified to form a N-glycoprotein having a glycan of the structure -GlcNAc(Fuc)b, wherein b is 0 or 1; and
3) mutating the protein such that 297N is substituted with a non-glycosylated amino acid.

36. A protein, comprising an antibody constant region or a fragment thereof, wherein the antibody constant region or the fragment thereof comprises one or more engineered tyrosine residues, and the engineered tyrosine residues are located at any one or any combination of positions 135, 137, 192, 223, and 329 of a heavy chain HC and position 202 of a light chain LC.

37. The protein according to any one of claims 33-36, wherein the engineered tyrosine residues in Pc are located at any one or any combination of positions 135, 137, 192, 223, and 329 of the heavy chain HC and position 202 of the light chain LC and are preferably selected from the group consisting of the following combinations:
202Y of the LC and 135Y of the HC;
202Y of the LC and 137Y of the HC;
202Y of the LC and 192Y of the HC;
202Y of the LC and 223Y of the HC;
202Y of the LC and 329Y of the HC;
135Y/137Y of the HC;
135Y/192Y of the HC;
135Y/223Y of the HC;
135Y/329Y of the HC;
137Y/192Y of the HC;
137Y/223Y of the HC;
137Y/329Y of the HC;
192Y/223Y of the HC;
192Y/329Y of the HC;
223Y/329Y of the HC;
135Y/137Y of the HC and 202Y of the LC;
135Y/192Y of the HC and 202Y of the LC;
135Y/223Y of the HC and 202Y of the LC;
135Y/329Y of the HC and 202Y of the LC;
137Y/192Y of the HC and 202Y of the LC;
137Y/223Y of the HC and 202Y of the LC;
137Y/329Y of the HC and 202Y of the LC;
192Y/223Y of the HC and 202Y of the LC;
192Y/329Y of the HC and 202Y of the LC;
223Y/329Y of the HC and 202Y of the LC;
135Y/137Y/192Y of the HC;
135Y/137Y/223Y of the HC;
135Y/137Y/329Y of the HC;
135Y/137Y/192Y of the HC and 202Y of the LC;
135Y/137Y/223Y of the HC and 202Y of the LC;
135Y/137Y/329Y of the HC and 202Y of the LC;
135Y/192Y/223Y of the HC;
135Y/192Y/329Y of the HC;
135Y/192Y/223Y of the HC and 202Y of the LC;
135Y/192Y/329Y of the HC and 202Y of the LC;
137Y/192Y/223Y of the HC;
137Y/192Y/329Y of the HC;
137Y/192Y/223Y of the HC and 202Y of the LC;
137Y/192Y/329Y of the HC and 202Y of the LC;
137Y/223Y/329Y of the HC;
137Y/223Y/329Y of the HC and 202Y of the LC;
192Y/223Y/329Y of the HC;
192Y/223Y/329Y of the HC and 202Y of the LC;
135Y/223Y/329Y of the HC; and
135Y/223Y/329Y of the HC and 202Y of the LC.

38. The protein according to any one of claims 32-37, wherein the antibody constant region or the fragment thereof is derived from an IgG, IgA, IgM, IgD, or IgE constant region or a fragment thereof; the IgG is preferably IgG1, IgG2, IgG3, or IgG4, and more preferably IGHG1, IGHG2, IGHG3, or IGHG4.

39. The protein according to any one of claims 32-38, wherein the antibody constant region or the fragment thereof is a heavy chain constant region or a fragment thereof and/or a light chain constant region or a fragment thereof;
preferably, the heavy chain constant region is selected from the group consisting of any one or any combination of a C_{H}1 region, a hinge region, and a C_{H}2 region, and the light chain constant region is a Cκ region;
preferably, the heavy chain constant region comprises a C_{H}3 region.

40. The protein according to claim 39, wherein the amino acid sequence of the C_{H}1 region is set forth in any one of SEQ ID NOs: 84 and 88-90, the amino acid sequence of the hinge region is set forth in SEQ ID NO: 85, the amino acid sequence of the C_{H}2 region is set forth in SEQ ID NO: 86, and the amino acid sequence of the Cκ region is set forth in SEQ ID NO: 87.

41. The protein according to any one of claims 32-40, wherein the antibody constant region or the fragment thereof in Pc is glycosylated or non-glycosylated; preferably, the glycosylation is 297N glycosylation.

42. The protein according to any one of claims 32-41, wherein the protein is selected from the group consisting of an antibody or an antigen-binding fragment thereof, an Fc region or a fragment thereof, and a fusion protein; the antibody or the antigen-binding fragment thereof is preferably a monospecific, bispecific, or multispecific antibody or an antigen-binding fragment thereof.

43. The protein according to claim 42, wherein the protein is an anti-tumor antibody or an antigen-binding fragment thereof; the anti-tumor antibody is preferably an anti-HER2 antibody, an anti-LIV-1 antibody, or an anti-TROP2 antibody;
more preferably, the anti-HER2 antibody or the antigen-binding fragment thereof comprises a VH and a VL, wherein the VH comprises a HCDR1, a HCDR2, and a HCDR3 of the amino acid sequences set forth in SEQ ID NOs: 51-53, and the VL comprises a LCDR1, a LCDR, and a LCDR3 of the amino acid sequences set forth in SEQ ID NOs: 54-56; or the VH comprises an amino acid sequence having at least 90% identity to SEQ ID NO: 57, and the VL comprises an amino acid sequence having at least 90% identity to SEQ ID NO: 58;
more preferably, the anti-LIV-1 antibody or the antigen-binding fragment thereof comprises a VH and a VL, wherein the VH comprises a HCDR1, a HCDR2, and a HCDR3 of the amino acid sequences set forth in SEQ ID NOs: 59-61, and the VL comprises a LCDR1, a LCDR, and a LCDR3 of the amino acid sequences set forth in SEQ ID NOs: 62-64; or the VH comprises an amino acid sequence having at least 90% identity to SEQ ID NO: 65, and the VL comprises an amino acid sequence having at least 90% identity to SEQ ID NO: 66;
more preferably, the anti-TROP2 antibody or the antigen-binding fragment thereof comprises a VH and a VL, wherein the VH comprises a HCDR1, a HCDR2, and a HCDR3 of the amino acid sequences set forth in SEQ ID NOs: 68-70, and the VL comprises a LCDR1, a LCDR, and a LCDR3 of the amino acid sequences set forth in SEQ ID NOs: 71-73; or the VH comprises an amino acid sequence having at least 90% identity to SEQ ID NO: 74, and the VL comprises an amino acid sequence having at least 90% identity to SEQ ID NO: 75.

44. The protein according to claim 42 or 43, wherein the antibody is selected from the group consisting of a murine antibody, a chimeric antibody, a humanized antibody, and a fully human antibody.

45. The protein according to any one of claims 32-44, wherein the protein is a monomer or a multimer, preferably a dimer, a trimer, or a tetramer.

46. The protein according to any one of claims 32-45, wherein Pc comprises a polypeptide fragment at a N-terminus and/or a C-terminus, and the fragment comprises one or more tyrosine residues;
preferably, the fragment is linked to the N-terminus and/or the C-terminus of the protein, either directly or by a linker;
more preferably, the linker is a GS or polyG linker;
most preferably, the N-terminus of the protein comprises YGGGG or YGGGGS, and/or the C-terminus comprises GGGGY or SGGGGY.

47. A method for preparing a conjugate, comprising:
a) providing a protein, wherein the protein comprises an antibody constant region or a fragment thereof, and the antibody constant region or the fragment thereof comprises an engineered tyrosine residue,
b) contacting the protein of a) with tyrosinase to convert the phenol moiety of the engineered tyrosine residue into an o-quinone moiety, and
c) subjecting the o-quinone moiety to a [4+2] cycloaddition reaction with an alkene or alkyne compound, wherein the alkene or alkyne compound comprises a cycloalkenyl, heterocycloalkenyl, cycloalkynyl, or heterocycloalkynyl moiety,
wherein preferably, the protein in a) is the protein according to any one of claims 32-46.

48. The method according to claim 47, wherein the tyrosinase is monomeric tyrosinase, and the monomeric tyrosinase is preferably derived from *Bacillus megaterium* or *Verrucomicrobium spinosum.*

49. The method according to claim 47 or 48, wherein the protein does not require removal/reduction of glycosylation modification before reacting with tyrosinase.

50. The method according to any one of claims 47-49, wherein the alkene or alkyne compound comprises a cycloalkenyl, heterocycloalkenyl, cycloalkynyl, or heterocycloalkynyl moiety, and optionally comprises a payload (D).

51. The method according to claim 50, wherein D is a therapeutic agent or a diagnostic agent;
preferably, D is a radioactive agent, a cytotoxic agent, a nucleic acid, a polypeptide, a fluorophore, a fluorescent dye, a radionuclide, an enzyme, an antibiotic, a chelating agent, a lipid, or an antibody or an antigen-binding fragment thereof;
more preferably, D is exatecan or a derivative thereof or eribulin or a derivative thereof.

52. The method according to any one of claims 47-51, wherein the o-quinone moiety in step b) undergoes a [4+2] cycloaddition reaction with the alkene or alkyne compound to form a structure represented by Za or Zb: 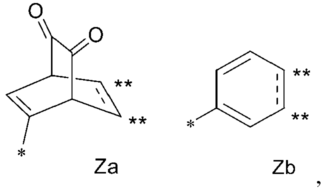
wherein * represents an end linked to the protein;
** represents an end linked to a linker;
---- is a single bond or a double bond.

53. An isotopically substituted form of the conjugate according to any one of claims 1-32, wherein preferably, the isotopically substituted form is a deuterated form.

54. A polynucleotide, wherein the polynucleotide encodes the protein according to any one of claims 33-46.

55. A vector, comprising or expressing the polynucleotide according to claim 54.

56. A pharmaceutical composition, comprising:
a therapeutically effective amount of the conjugate according to any one of claims 1-32, and
a pharmaceutically acceptable carrier, diluent, or excipient.

57. Use of the protein according to any one of claims 33-46 for the manufacture of a conjugate.

58. The conjugate according to claims 1-32 and the isotopically substituted form of the conjugate according to claim 53, wherein the conjugate and the isotopically substituted form include antibody-drug conjugates, polypeptide-drug conjugates, and antibody-peptide conjugates.
